(19) Europäisches Patentamt
European Patent Office
Office européen des brevets

(11) EP 4 242 221 A1

(12) EUROPEAN PATENT APPLICATION

(43) Date of publication:
**13.09.2023 Bulletin 2023/37**

(21) Application number: **22461521.1**

(22) Date of filing: **09.03.2022**

(51) International Patent Classification (IPC):
***C07K 14/00*** *(2006.01)*

(52) Cooperative Patent Classification (CPC):
**C07K 14/001;** C07K 2319/00; G01N 2440/36

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **UNIWERSYTET JAGIELLONSKI**
**31-007 Kraków (PL)**

(72) Inventors:
- **Glatt, Sebastian**
**30-231 Kraków (PL)**
- **Ethiraju Ravichandran, Keerthiraju**
**30-384 Kraków (PL)**

(74) Representative: **Witek, Rafal**
**WTS Patent Attorneys**
**Witek, Sniezko & Partners**
**ul. Weigla 12**
**53-114 Wroclaw (PL)**

# (54) METHOD FOR THIOLATION AND COVALENT CONJUGATION OF TARGET PROTEIN

(57) The present invention relates to methods for transferring a sulfur moiety from a sulfur carrier protein to a cysteine residue of a target protein and corresponding protein conjugation methods, as well as methods of protecting proteins against cysteine oxidation. Particularly, the invention refers to the thiolation of non-canonical ubiquitin-like protein (UBL) targets, in particular Urm1 targets. Corresponding uses of sulfur donor proteins, in particular UBL protein, such as Urm1 are envisaged as well.

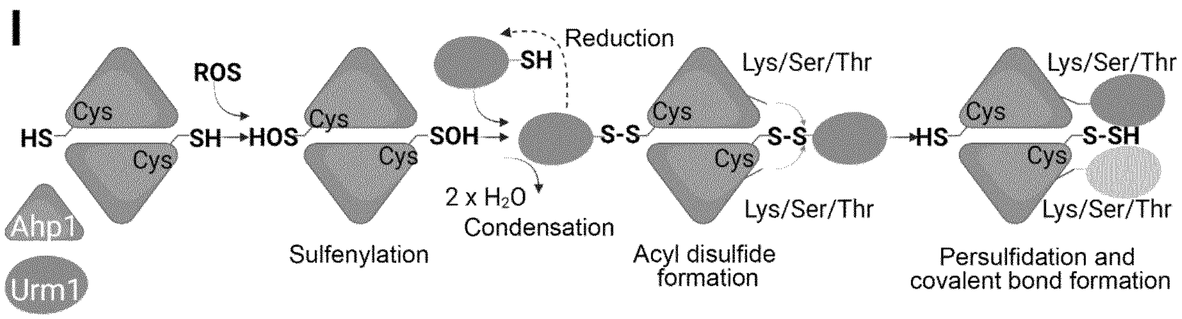


Fig. 4 I



Processed by Luminess, 75001 PARIS (FR)

## Description

## FIELD OF THE INVENTION

The present invention relates to methods for transferring a sulfur moiety from a sulfur carrier protein to a cysteine residue of a target protein and corresponding protein conjugation methods, as well as methods of protecting proteins against cysteine oxidation. Particularly, the invention refers to the thiolation of non-canonical ubiquitin-like protein (UBL) targets, in particular Urm1 targets. Corresponding uses of sulfur donor proteins, in particular UBL protein, such as Urm1 are envisaged as well.

## BACKGROUND OF THE INVENTION

Ubiquitin related modifier 1 (Urm1) is an evolutionarily conserved member of the ubiquitin (Ub) family that adopts the β-grasp fold characteristic of all eukaryotic ubiquitin-like proteins (UBLs) (e.g. ubiquitin, SUMO, NEDD8, ATG12, UFM) (Bedford et al., 2011). Urm1 acts as a sulfur carrier protein SCP and is an essential member of the modification cascade that thiolates wobble uridine ($s^2U_{34}$) in eukaryotic tRNA anticodons via the Ncs2-Ncs6 sulfur-transferase complex (Leidel et al., 2009; Nakai et al., 2008; Noma et al., 2009; Schlieker et al., 2008). The attachment of a sulfur atom at the C2 position of the tRNA anticodon base optimizes ribosomal dynamics during translational elongation (Nedialkova and Leidel, 2015; Ranjan and Rodnina, 2016) and affects cellular responses to nutrient starvation (Bruch et al., 2020; Gupta et al., 2018; Laxman et al., 2013). The disruption of tRNA anticodon modifications is associated with dramatic proteome perturbations and the onset of severe human diseases, including cancer and neurodegenerative diseases (Close et al., 2018; Hawer et al., 2018; Nedialkova and Leidel, 2015; Rezgui et al., 2013; Schaffrath and Leidel, 2017). In addition to its role as SCP in tRNA thiolation, early reports also showed that upon oxidative stress, Urm1 covalently attaches to target proteins in different organisms (Furukawa et al., 2000; Goehring et al., 2003; Schlieker et al., 2008; Khoshnood et al., 2016; Jüdes et al., 2015; Van Der Veen et al., 2011; Wang et al., 2019).

The conjugation reaction of an UBL to its target protein starts with adenylation of its C-terminus by ATP-dependent E1 ubiquitin-activating enzymes. Next, the activated C-terminus of the UBL is relayed via active site cysteines of an E1-, E2- and E3-enzyme cascade (Passmore and Barford, 2004). Finally, the UBL is attached to a specific target protein via a covalent iso-peptide bond formed between a highly conserved diglycine motif at its C-terminus and a lysine side chain of the target protein (Cappadocia and Lima, 2018; Hochstrasser, 2000; McDowell and Philpott, 2013; Stewart et al., 2016). Similarly, the initial activation steps for SCPs also start with adenylation of the C-terminus by specialized E1-like proteins. Although SCPs typically do not form thioesters, recent work revealed the necessity of a thioester intermediate for the activation of Urm1 by its genuine E1 enzyme, Uba4 (Pabis et al., 2020; Termathe and Leidel, 2018). Following the formation of a thioester between Uba4 and Urm1, the rhodanese domain of Uba4 transfers a persulfide group to the activated C-terminus of Urm1. The resulting thiocarboxylated C-terminus (COSH) is present in all prokaryotic and archaeal SCPs (Kessler, 2006). However, amongst eukaryotic UBLs only Urm1 is known to carry this unique terminal modification (Urm1-SH). Hence, Urm1 and Uba4 are located at a crucial evolutionary branchpoint between prokaryotic SCPs and eukaryotic UBLs since they combine molecular features otherwise exclusively present in either SCPs or UBLs (Jüdes et al., 2015; Pedrioli et al., 2008; Xu et al., 2006).

As the role of Urm1-SH for tRNA thiolation is well established and no E2 and E3 enzymes for Urm1 have been identified so far, the conjugation of Urm1 to proteins (also referred to as protein "urmylation") has been questioned. Nonetheless, several studies employed mass spectrometry to identify proteins targeted for Urm1 conjugation under specific environmental conditions (Schlieker et al., 2008; Van Der Veen et al., 2011; Khoshnood et al., 2017). *In vivo,* the most robust conjugation of Urm1 was observed for Ahpl (Goehring et al., 2003; Van Der Veen et al., 2011; Jüdes et al., 2015, 2016; Brachmann et al., 2020), a peroxiredoxin that acts as an antioxidant enzyme in scavenging reactive oxygen species (Lee et al., 1999). However, the biochemical details and the E2 enzymes and E3 ligases that are required for the conjugation reaction to Ahpl or any of the other targets remain undefined. Furthermore, as the unique COSH moiety at the C-terminus of Urm1 is absent from all other eukaryotic UBLs, its role and fate after the conjugation reaction appear elusive. Finally, the necessity of oxidative stress and redox-active cysteines for the conjugation of Ahpl (Brachmann et al., 2020) raised the question whether the conjugation to other proteins would depend on similar target motifs and reaction conditions.

## OBJECTIVES AND SUMMARY OF THE INVENTION

The inventors provide novel methods for protein thiolation, covalent conjugation of proteins as well as methods for protection of cysteine residues against oxidation.

The inventors showed for the first time that the Urm1 pathway represents a distinct cysteine persulfidation pathway which does not require E2 enzymes or E3 ligases. Further, they showed that the transfer of sulfur from the C-terminus of Urm1 onto cysteine residues in the Urm1 target proteins is a mechanistic consequence of the urmylation reaction, which further comprises the conjugation of Urm1 to the Urm1 target protein. By synthetically designing thiocarboxylated model proteins, they could show that the sulfur moiety can be transferred also from proteins other than Urm1 that comprises a sulfur moiety at their C-terminus. In addition, with regard to the target protein they showed that the sulfur attachment site requires a peroxidatic cysteine. Thereby the inventors establish a universal platform for protein tagging as well as persulfidation of cysteine residues in proteins protecting them against oxidation.

Thus, the inventors provide a method which allows

- the transfer of a sulfur moiety from a sulfur carrier protein to a cysteine residue of a target protein, and
- the conjugation of the sulfur carrier protein to the target protein.

Accordingly, a first aspect of the invention refers to a method for transferring a sulfur moiety from a sulfur carrier protein to a cysteine residue of a target protein, comprising the steps:

a) providing

- a sulfur carrier protein comprising a thiocarboxylated C-terminus,
- a target protein comprising at least one oxidizable cysteine residue,

b) contacting the sulfur carrier protein with the target protein under conditions allowing the transfer of the sulfur moiety from the sulfur carrier protein to the target protein to obtain a thiolated target protein.

Accordingly, a further aspect of the invention relates to a method for protein conjugation comprising the steps:

a) providing

- a sulfur carrier protein comprising a thiocarboxylated C-terminus,
- a target protein comprising at least one oxidizable cysteine residue,

b) contacting the sulfur carrier protein with the target protein under conditions allowing the conjugation of the sulfur carrier protein and the target protein to obtain a conjugated target protein.

The described methods do not involve E2 ubiquitin-conjugating enzymes and/or E3 ligases.

Typically, the conditions allowing the conditions allowing the transfer of the sulfur moiety from the sulfur carrier protein and/or conjugation of the sulfur carrier protein and the target protein are mild oxidative stress conditions.

In specific embodiment, the sulfur carrier protein is a non-canonical UBL, such as Urm1.

Typically, the oxidizable cysteine of the target protein is in proximity to a lysine, threonine and/or serine in the target protein.

In some embodiments, the target proteins are Urm1 target proteins, such as HsPRDX5, ScTdh3, HsGAPDH, ScSes1, HsSARS1, ScPyk1, HsPKM2.

In some embodiments, the methods may comprise further steps, such as changing the conditions to stop the transfer of the sulfur moiety from the sulfur carrier protein and the conjugation of the sulfur carrier protein and the target protein.

Accordingly, further aspects relate to uses of a sulfur carrier protein comprising a thiocarboxylated C-terminus for transferring a sulfur moiety to a target protein comprising at least one oxidizable cysteine residue and/or for transferring a sulfur moiety to a target protein comprising at least one oxidizable cysteine residue.

The application also refers to proteins obtained by the method of the invention.

Moreover, the application also refers to an isolated covalently conjugated protein comprising a sulfur carrier protein covalently bound to a target protein, wherein the target protein comprises a sulfenylated cysteine. Preferably the sulfur carrier protein is covalently bound a lysine, serine or threonine residue of the target protein.

**FIGURES**

Figure 1 presents Urm1-SH forms an iso-peptide bond *in vitro* with Ahpl in response to thiol-reactive species:

**A.** Scheme of different methodologies to generate Urm1-SH *in vitro.* Sulfur from cysteine is passed from Nfs1 to Uba4 directly or via Tum1. Uba4 transfers sulfur to Urm1 by catalyzing sequential adenylation and thiocarboxylation reactions. The intein based inducible cleavage system can produce carboxylated Urm1 (OH) or thiocarboxylated Urm1 (SH) by using DTT or ammonium sulfide $(NH_4)_2S$, respectively. Intein produced SH is in orange and Uba4 produced SH* is in blue.

**B.** ESI-MS analyses of Urm1-OH and Urm1-SH produced via Uba4 (blue and asterisk) or the intein system (orange). The expected and assessed masses of the samples are listed. Inset: APM-gel analyses of the same Urm1 samples. The slow retardation of the Urm1 samples corresponds to Urm1-SH. SDS-PAGE gel below for the same proteins using a sample buffer containing DTT. APM: ([N-Acryloyl-amino] phenyl) mercuric chloride. SDS-PAGE gels were stained with Coomassie.

**C.** *In vivo* conjugation analyses in the presence (left) or absence of NEM (N-Ethylmaleimide) (right) with protein extracts obtained from the indicated yeast strains expressing *HA-URM1.* Unconjugated Urm1 and Urm1-conjugates were detected by anti-HA (top panels) Western blots. Anti-Ahpl blots (middle) detect unconjugated Ahpl. Anti-Cdc19 (bottom panels) served as loading control.

**D.** Analysis of covalent adduct formation between *Sc*Ahp1 or *Sc*Ahp1$_{C31S}$ or *Sc*Ahp1$_{C31S\ C62S}$ mutant and carboxylated (OH) or thiocarboxylated *Sc*Urm1 (SH) or Uba4 produced thiocarboxylated Urm1 (SH*) in the presence of TBH (tert-Butyl hydroperoxide). Confirmation of the isopeptide bond was carried by addition of DTT, TCEP or HA respectively. Unconjugated Ahpl and Urm1 as well as their formed conjugate (Ahpl-Urm1) are indicated on the right. DTT: 1,4-Dithiothreitol; TCEP: Tris(2-carboxyethyl) phosphine; HA: Hydroxylamine.

Figure 2 presents structure of the urmylated-Ahp1 complex:

**A.** Size exclusion chromatography profiles of *Ct*Ahp1$_{C30S}$ (yellow), *Ct*Urm1$_{C55S}$ (cyan) and the *Ct*Ahp1$_{C30S}$-*Ct*Urm1$_{C55S}$ complex (pink).

**B.** Crystal structures of dimeric *Ct*Ahp1 and *Ct*Ahp1$_{C30S}$ in cartoon representation. Insets: Close-up of the active sites highlighting redox active cysteines (Cys60 and Cys/Ser30 in red).

**C.** Front (up) and top (down) view of the *Ct*Ahp1$_{C30S}$-*Ct*Urm1$_{C55S}$ complex structure in cartoon representation. All three entities in the asymmetric unit are superimposed on Ahp1 and the various Urm1 molecules are labeled. The position of Lys63 (violet) is highlighted and labeled. Insets: Close-up of the active sites highlighting the position of the redox active cysteines (Cys60 and Ser30 in red).

**D.** Comparison of the three observed states of Urm1 conjugation in relation to the linkage site of Ahp1. All 6 Ahp-Urm1 entities in the asymmetric unit are superimposed on Ahp1. All relevant residues are labeled and highlighted in the ball and stick representation. The C-terminal residues of the attached Urm1 are shown in cartoon representation and the remaining parts as transparent surface. Cysteines are highlighted (Cys60 and Ser30 in red).

Figure 3 presents urmylation is an E2/E3-independent, and primarily lysine-directed protein conjugation:

**A.** Comparison of the individual Ahp1-Urm1 units in the asymmetric unit. Close up of the formed peptide bond with all nearby residues labeled and highlighted in a balls-and-sticks representation (Cys60 in red). The last strand of the attached Urm1 protein is shown in cartoon representation and the refined 2Fo-Fc density (blue) is shown around Ahp1$_{Lys63}$ and Urm1$_{G111}$ at 1$\sigma$.

**B.** Top: MS/MS spectrum of the K63-$\varepsilon$-GGH containing peptide from *Ct*Ahp1$_{C30S}$ and K32-$\varepsilon$-GGH containing peptide from *Sc*Ahp1$_{C31S}$. A representative annotated fragmentation spectrum is shown with the b- and y-ions marked in red and blue, respectively. The precursor ions are labelled in green. The peptide sequence is shown at the top with the collision-induced fragmentation pattern. m/z: mass to charge ratio. Bottom: Schematic summary of the detected conjugated peptides by mass spectrometry for *Ct*Ahp1$_{C30S}$, *Ct*Ahp1$_{C30S\ K63A}$ and *Sc*Ahp1$_{C31S}$. The thickness of the line represents the number of replicates where the respective site was detected, and the height represents the associated maximum Mascot ion score (n>≥3). The urmylation was identified as HisGlyGly tag on lysines, serines and threonines while persulfidation was searched as a mass shift: +32 Da (persulfidation) or +89 Da (persulfidation + carbamidomethylation) on cysteines.

**C.** Analysis of conjugation between *Ct*Ahp1$_{C30S}$ variants in combination with thiocarboxylated *Ct*Urm1$_{C55S}$ in the presence of TBH. Unconjugated Ahpl and Urm1 as well as the formed conjugates (Ahp1-Urm1) are indicated on the right.

**D.** Analysis of conjugation between *Sc*Ahp1$_{C31S}$ variants in combination with thiocarboxylated *Sc*Urm1 in the presence of TBH. Unconjugated Ahpl and Urm1 as well as the formed conjugates (Ahp1-Urm1) are indicated on the right. *Sc*Ahp1$_{4KR}$: *Sc*Ahp1$_{C31S\ K47/48R\ K124R\ K156R}$; *Sc*Ahp 1$_{5KR}$: *Sc*Ahp1$_{C31S\ K32R\ K47/48R\ K124R\ K156R}$.

**E.** TBH cytotoxicity assay *in vivo* of the *Sc*Ahp1 lysine mutants in yeast. Growth of *yap1Δahp1Δ* cells carrying empty vector (ev), wild type peroxiredoxin gene (*AHP1*) or indicated cysteine or lysine substitutions was monitored without or with 1 mM TBH. *Sc*Ahp1$_{4KR}$: *Sc*Ahp1$_{C31SK47/48RK124RK156R}$; *Sc*Ahp1$_{5KR}$: *Sc*Ahp1$_{C31SK32RK47/48RK124RK156R}$.

**F.** *In vivo* conjugation analyses of *Sc*Ahp1 lysine mutants with protein extracts obtained from the indicated yeast strains expressing *HA-URM1*. Unconjugated Urm1 and Urm1-Ahpl conjugates were detected by anti-HA (top panels) Western blots. Anti-Ahpl blots (middle) detect unmodified Ahpl. Anti-Cdc19 (bottom panels) served as loading control. *Sc*Ahp1$_{4KR}$: *Sc*Ahp1$_{C31S\ K47/48R\ K124R\ K156R}$; *Sc*Ahp1$_{5KR}$: *Sc*Ahp1$_{C31S\ K32R\ K47/48R}$ K124R K156R.

**G.** Comparison of the crystal structures of the oxidized dimeric forms of Ahpl from *Ct*Ahp1$_{C30S}$ (left) and *Sc*Ahp1 (right; PDB ID 4DSR). All lysine residues (green) are shown in the ball and sticks representation. Conjugated lysine residues detected by mass spectrometry (in B) are labelled and highlighted in black. Other lysine residues are labeled in grey and peroxidatic cysteines (Cys60 in *Ct*Ahp1 and Cys62 in ScAhp1) and their surroundings are labeled in red.

Figure 4 presents Urm1 conjugation directly mediates cysteine persulfidation *in vitro:*

**A.** Analysis of conjugation between Urm1-SH with *Sc*Ahp1$_{C31S}$ (left) and *Ct*Ahp1$_{C30S}$ (right) using lysine-less variants (*Sc*Ahp1$_{C31S^{-}KtoR}$ and *Ct*Ahp1$_{C30S^{-}KtoR}$). *Sc*Urm1 or *Ct*Urm$_{C55S}$ were used. The control reactions with Urm1-OH and without TBH are indicated.

**B.** Structural close-up of the Ahp1-Urm1 conjugation site with all nearby residues labeled and highlighted in balls-and-sticks representation (Cys60 in red). The last strand of the attached Urm1 protein (green) is shown in the cartoon. The distance between the peptide bond that forms between Lys63 (violet)/Gly1 11 (green) and Cys60 (red) is indicated.

**C.** Analysis of conjugation between thiocarboxylated *Ct*Urm1$_{C55S}$ and *Ct*Ahp1$_{C30S}$ variants, combining substitutions of serine and threonine residues in the active site and lysine-less variants in the presence of TBH.

**D.** MS/MS spectrum of the S-ε-GGH/T-ε-GGH containing peptide from *Ct*Ahp 1 $_{C30S/KtoR}$ (top), and *Sc*Ahp1$_{C31S/KtoR}$ (bottom) A representative annotated fragmentation spectrum is shown with the b- and y-ions marked in red and blue, respectively. The peptide sequence is displayed at the top with the collision-induced fragmentation pattern.

**E.** Radioisotope assay for $^{35}$S-labelled Urm1. Analysis of conjugation and sulfur transfer between Sc $^{35}$S-Urm1 with *Sc*Ahp1 variants (left) and *Ct* $^{35}$S-Urm1$_{C55S}$ with *Ct*Ahp1 variants in presence (top) or absence of TBH (bottom). The samples were separated by non-reducing SDS-PAGE and visualized by Coomassie staining. Subsequently, dried gels were exposed overnight to storage phosphor screens.

**F.** (top) MS/MS spectrum of the persulfidated Cys60 and K63-ε-GGH containing peptide from *Ct*Ahp1$_{C30S}$. (bottom) MS/MS spectrum of the persulfidated Cys62 peptide from *Sc*Ahp1$_{C31S}$. Representative annotated fragmentation spectra are shown with the b- and y-ions marked in red and blue, respectively. The peptide sequence is displayed at the top with the collision-induced fragmentation pattern. mass shift: +32 Da (persulfidation) or +89 Da (persulfidation + carbamidomethylation).

**G.** Structural close-up of the peroxidatic cysteine (Cys60) and Lys63 (violet) in *Ct*Ahp1$_{C30S}$ pre-conjugation (left) and post-conjugation reaction (right). panels) and (right panels).

**H.** Same view as in G of the refined 2Fo-Fc maps in the *Ct*Ahp1$_{C30S}$-*Ct*Urm1$_{C55S}$ complex around Cys60 (red) at 2 σ (left) and the anomalous difference Fourier map at 2.5 σ (right). The respective resolution ranges of the maps in G and H are indicated.

**I.** Proposed mechanism of urmylation. The peroxidatic cysteine of Ahp1 gets oxidized and sulfenylated. Urm1-SH recognizes the sulfenylated cysteine and condenses to form an acyl disulfide intermediate. The formation of this intermediate can be reverted by reduction. A lysine/serine/threonine residue in proximal distance from the cysteine of the target protein mediates a nucleophilic attack on Urm1 to resolve the acyl disulfide. This results in a persulfidated cysteine and the covalent conjugation of the C-terminus of Urm1 with the respective lysine/serine/threonine acceptor residue.

Figure 5 presents cellular Urm1 targets are persulfidated upon conjugation *in vitro:*

**A.** Scatter plot of normalized SILAC H/L ratios (NEM-treated versus untreated) for 547 quantified proteins, plotted against the sum of the respective peptide intensities. Proteins are color-coded according to their respective p-values (red <0.001, orange 0.001-0.01, yellow 0.01-0.05 and blue >0.05).

**B.** Analysis of conjugation between thiocarboxylated *Sc*Urm1 or *Hs*Urm1 and various urmylation target proteins (*Sc*Ahp1$_{C31S}$, *Hs*PRDX5, *Sc*Tdh3, *Hs*GAPDH, *Sc*Ses1, *Hs*SARS1, *Sc*Pyk1, *Hs*PKM2) in the presence of diamide. Unconjugated target proteins and Urm1 as well as their conjugates are indicated on the right.

**C.** Schematic summary of the detected conjugated peptides by mass spectrometry for the various targets in B. The thickness of the line represents the number of replicates where the respective site was detected, and the height represents the associated maximum Mascot ion score (n>≥3). The urmylation was identified as HisGlyGly tag on lysines, serines and threonines, while persulfidation was searched as a mass shift: +32 Da (persulfidation) or +89 Da (persulfidation + carbamidomethylation) on cysteines.

**D.** Analysis of conjugation between thiocarboxylated *Sc*Urm1 or *Hs*Urm1 and various urmylation target proteins (*Sc*Ahp1$_{C31S}$, *Hs*PRDX5, *Sc*Tdh3, *Hs*GAPDH, *Sc*Ses1, *Hs*SARS1, *Sc*Pyk1, *Hs*PKM2) and their cysteine mutants in the presence of diamide. Unconjugated target proteins and Urm1 as well as their conjugates are indicated on the right.

**E.** Radioisotope assay for $^{35}$S-labelled Urm1. Analysis of conjugation and sulfur transfer between *Sc* $^{35}$S-Urm1 with *Sc*Tdh3, *Sc*Ses1 and *Sc*Pyk1 in the presence or absence of diamide. The samples were separated by non-reducing SDS-PAGE and visualized by Coomassie staining. The dry gels were exposed overnight to storage phosphor screens. * Uba4 background

Figure 6 presents thiocarboxylated Ubiquitin and SUMO conjugate to Ahpl *in vitro:*

**A.** Analysis of covalent adduct formation by carboxylated (OH) or thiocarboxylated (SH) *Sc*Ubiquitin or *Sc*SUMO on *Sc*Ahp1$_{C31S}$ or ScTdh3 in the presence of diamide. Thiocarboxylated *Sc*Urm1 was used as a control. Unconjugated Ubls and target proteins as well as their conjugates are indicated on the right.

B. Schematic summary of the conjugated peptides that were detected by mass spectrometry for ubiquitin or SUMO conjugation on Ahpl. The thickness of the line represents the number of replicates where the respective site was detected, and the height represents the associated ion score (n>3). MS/MS spectrum of the ubiquitin (K-ε-GG/ S-ε-GG/ T-ε-GG) or SUMO (K-ε-GGIQ/ S-ε-GGIQ/ T-ε-GGIQ) conjugated peptide on *Sc*Ahp$1_{C31S}$ and the persulfidated cysteine spectrum by ubiquitin or SUMO. A representative annotated fragmentation spectrum is shown with b- and y-ions marked in red and blue, respectively. The peptide sequence is shown at the top along with the collision-induced fragmentation pattern. The ubiquitin or SUMO conjugation site was identified by detection of the GG and GGIQ remnant motif, respectively. m/z: mass to charge ratio. Persulfidation was searched as a mass shift: +32 Da (persulfidation) or +89 Da (persulfidation + carbamidomethylation) on cysteines.

**C.** Analysis of covalent adduct formation between *Sc*Ahp$1_{C31S}$ and various thiocarboxylated C-terminal variants of *Sc*Urm1 in the presence of TBH. Unconjugated Urm1, Ahpl and the conjugates are indicated on the right.

Figure 7 presents trans-sulfuration and Uba4-Urm1 are independent pathways for protein thiolation:

**A.** TBH cytotoxicity assay *in vivo*. Basic growth and the synthetic effect in wild type, *ahp1Δ, urm1Δ, tum1Δ, ncs6Δ, cys3Δ, cys4Δ, cys3Δ urm1Δ,* and *cys4Δ urm1Δ* strains was monitored without or with 1 mM TBH.

**B.** $H_2S$ production using Biggy agar plates- Wild type, *ahp1Δ, urm1Δ, tum1Δ, ncs6Δ, cys3Δ, cys4Δ, cys3Δ urm1Δ,* and *cys4Δ urm1Δ* strains were grown on Biggy medium, and $H_2S$ production was recorded as indicated in the material and methods section.

**C.** *In vivo* Ahpl conjugation analyses with protein extracts obtained from the indicated yeast strains expressing *HA-URM1.* Unconjugated Urm1 and Urm1-Ahp1 conjugates were detected by anti-HA (top panels) Western blots. Anti-Ahpl blots (middle) detect unmodified Ahpl. Anti-Cdc19 (bottom panels) served as loading control.

D. Northern-blot analysis for thiolation levels of $\mathrm{tRNA}_{\mathrm{UUC}}^{\mathrm{Glu}}$ in the genetic background of the indicated yeast strains. Total tRNA was resolved on denaturing PAGE supplemented with APM to retard the migration of thiolated $\mathrm{tRNA}_{\mathrm{UUC}}^{\mathrm{Glu}}$ .APM: ([N-Acryloyl-amino] phenyl) mercuric chloride.

**E.** Scheme of sulfur source for thiolation. Sulfur released as $H_2S$ from transsulfuration enzymes Cys4 and Cys3 is used for protein persulfidation, scavenging free radicals metabolic signaling/stimulation. The sulfur from the Uba4-Urm 1 pathway is directed to tRNA thiolation and to persulfidation of specific cysteine of target proteins.

Figure 8 presents radioisotope labelling for the in vivo urmylation targets:

**A.** Analysis of *in vitro* conjugation of active site mutants. *Ct*Ahp1 variants *Ct*Ahp$1_{C30S}$, *Ct*Ahp$1_{C30S/C60S}$, *Ct*Ahp$1_{C30S/R151A}$ and thiocarboxylated Urm1 variants *Ct*Urm$1_{C55S\text{-}H109A}$, *Ct*Arm$1_{C55S\text{-}L108AM109A}$ in the presence of TBH. The samples were separated by SDS-PAGE and visualized by Coomassie stain. Unconjugated target proteins as well as the formed conjugates are indicated on the right.

**B.** Deconvoluted mass spectra of (Top) *Sc*Urm1-SH and (Bottom) *Ct*Urm1 csss -SH produced via IscS and *Sc*Uba4/*Ct*Uba4 using L-cysteine as a sulfur source. The expected and determined masses of the samples are indicated.

C. Radioisotope labelling. Production of $^{35}$S-labelled Urm1 by desulfurase ISCS and Uba4 using $^{35}$S L-cysteine. Pyridoxal-5'-phosphate (PLP) and ATP were added as mentioned.

**D.** Radioisotope assay for $^{35}$S-labelled Urm1. Analysis of conjugation and sulfur transfer from *Sc*$^{35}$S-Urm1 onto *Sc*Ahp1, *Sc*Ahp$1_{C31S}$, *Sc*Ahp$1_{C62S}$, *Sc*Ahp1C$_{31SC62S}$ and *Sc*Ahp1C$_{31S^{-}KtoR}$ in presence of TBH. The samples were separated by non-reducing SDS-PAGE and visualized by Coomassie staining. The dried gels were exposed overnight to storage phosphor screens.

**E.** Radioisotope assay for $^{35}$S-labelled Urm1. Analysis of conjugation and sulfur transfer from *Sc*$^{31}$S-Urm1 onto *Sc*Ahp1, *Sc*Ahp$1_{C31S}$, *Sc*Ahp$1_{C62S}$, *Sc*Ahp$1_{C31SC62S}$ and *Sc*Ahp$1_{C31S^{-}KtoR}$ in absence of TBH. The samples were separated by non-reducing SDS-PAGE and visualized by Coomassie staining. Subsequently, the dried gels were exposed overnight to storage phosphor screens.

**F.**Radioisotope assay for $^{35}$S-labelled Urm1. Analysis of conjugation and sulfur transfer from *Ct*$^{35}$S-Urm$1_{C55S}$ onto *Ct*Ahp1, *Ct*Ahp$1_{C30S}$, *Ct*Ahp$1_{C60S}$, *Ct*Ahp$1_{C30SC60S}$ and *Ct*Ahp$1_{C30S\text{-}KtoR}$ in presence of TBH. The samples were separated by non- reducing SDS-PAGE and visualized by Coomassie staining and then the dried gels were exposed overnight to storage phosphor screens.

**G.** Radioisotope assay for $^{35}$S-labelled Urm1. Analysis of conjugation and sulfur transfer from *Ct*$^{35}$S-Urm$1_{C55S}$ onto *Ct*Ahp1, *Ct*Ahp$1_{C30S}$, *Ct*Ahp$1_{C60S}$, *Ct*Ahp$1_{C30SC60S}$ and *Ct*Ahp 1 $_{C30S^{-}KtoR}$ in absence of TBH. The samples were separated by non- reducing SDS-PAGE and visualized by Coomassie staining. The dried gels were exposed overnight to storage phosphor screens.

Figure 9 presents specificity of Urm1 conjugation in various *vivo* targets:

**A.** Analysis of covalent adduct formation by carboxylated *Sc*Urm1 or *Hs*Urm1 on various urmylation targets (*Sc*Ahp1$_{C31S}$, *Hs*PRDX5, *Sc*Tdh3, *Hs*GAPDH, *Sc*Ses1, *Hs*SARS1, *Sc*Pyk1, *Hs*PKM2) in the presence of diamide.

**B.** Analysis of covalent adduct formation by thiocarboxylated *Sc*Urm1 or *Hs*Urm1 on various urmylation targets (*Hs*PRDX5, ScTdh3, *Hs*GAPDH, *Sc*Ses1, *Hs*SARS1, *Sc*Pyk1, *Hs*PKM2) and cysteine mutants thereof in the presence of diamide.

**C.** MS/MS spectrum of the *Sc*Urm1 or *Hs*Urm1 conjugated peptide K-ε-GGH on *Hs*PRDX5, *Sc*Tdh3, *Hs*GAPDH, *Sc*Ses1, *Hs*SARS1, *Sc*Pyk1, *Hs*PKM2. A representative annotated fragmentation spectrum is shown with the b- and y-ions marked in red and blue, respectively. The peptide sequence is shown at the top with the collision-induced fragmentation pattern. The Urm1 conjugation site was identified by detection of the HGG motif generated by chymotrypsin digestion. m/z: mass to charge ratio. The urmylation was identified as HGG tag on lysines, while persulfidation was searched as a mass shift: +32 Da (persulfidation) or +89 Da (persulfidation + carbamidomethylation) on cysteines.

D. Comparison of the crystal structures of Urm1 targets. *Sc*Ahp1 reduced (PDB ID: 4DSR) alongside *Hs*PRDX5 (PDB ID: 1HD2). *Sc*Tdh3 (PDB ID: 3PYM) alongside HsGAPDH (PDB ID: 6VND). *Sc*Pyk1 (PDB ID: 1A3W) alongside *Hs*PKM2 (PDB ID: 6V74). *Sc*Ses1 (homology model built using 4RQE) alongside *Hs*SARS1 (PDB ID: 4RQE). Cartoon representation of crystal structures. The lysines are shown as grey color sticks and the Urm1 conjugated lysines are highlighted by blue color sticks. The cysteines are shown as orange color sticks and the persulfidated cysteines are highlighted as red color sticks.

Figure 10 presents GFP-Urm 1 for tracing persulfidation *in vivo :*

**A.** Analysis of in vitro conjugation between *Sc*Ahp1$_{C31S}$ or *Sc*Pyk1 and various thiocarboxylated variants of GFP in the presence of TBH. Unconjugated target proteins as well as the formed conjugates are indicated on the right

**B.** MS/MS Spectra of GFP conjugated to *Sc*Ahp1$_{C31S}$ via lysine. A representative annotated fragmentation spectrum is shown with the b- and y-ions marked in red and blue, respectively. The peptide sequence is shown at the top with the collision-induced fragmentation pattern. The GFP conjugation site was identified by detection of conjugated lysine residues generated by chymotryptic digestion.

**C.** ATP hydrolysis by *Sc*Uba4 WT in the presence of *Sc*Urm1 variants, ScUbiquitin, *Sc*Sumo and GFP variants. Data are representative of three independent experiments (n>3). The error bars represent the calculated standard error of the mean (SEM).

**D.** (Left) Crystal structure of Uba4 in complex with Urm1 (PDB ID 6YUC). The C-terminus of Urm1 highlighted in red is conjugated to the catalytic Uba4$_{C202K}$ that is a mimetic of the thioester bond usually formed. (Right) Structural close-up view of Uba4 activation loop with Urm1 C-terminus highlighted in red with Uba4 the crossover loop.

**E.** Analysis of *in vitro* conjugation of *Sc*Ahp1$_{C31S}$, ScTdh3 and *Sc*Ses1 by carboxylated GFP-Urm1 or Uba4-thiocarboxylated GFP-Urm1 in presence or absence of diamide. Unconjugated proteins as well as the formed conjugates are indicated on the right.

## DETAILED DESCRIPTION OF THE INVENTION

Before the invention is described in detail with respect to some of its preferred embodiments, the following general definitions are provided.

The present invention as illustratively described in the following may be suitably practiced in the absence of any element or elements, limitation or limitations, not specifically disclosed herein.

The present invention will be described with respect to particular embodiments and with reference to certain figures, but the invention is not limited thereto but only by the claims.

Where the term "comprising" is used in the present description and claims, it does not exclude other elements. For the purposes of the present invention, the term "consisting of" is considered to be a preferred embodiment of the term "comprising of'. If hereinafter a group is defined to comprise at least a certain number of embodiments, this is also to be understood to disclose a group which preferably consists only of these embodiments.

For the purposes of the present invention, the term "obtained" is considered to be a preferred embodiment of the term "obtainable". If hereinafter e.g. an antibody is defined to be obtainable from a specific source, this is also to be understood to disclose an antibody which is obtained from this source.

Where an indefinite or definite article is used when referring to a singular noun, e.g. "a", "an" or "the", this includes a plural of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of ±10%, and preferably of ±5%.

Technical terms are used by their common sense or meaning to the person skilled in the art. If a specific meaning is conveyed to certain terms, definitions of terms will be given in the following in the context of which the terms are used.

A first aspect of the invention refers to a method for transferring a sulfur moiety from a sulfur carrier protein to a cysteine residue of a target protein, comprising the steps:

a) providing

- a sulfur carrier protein comprising a thiocarboxylated C-terminus,
- a target protein comprising at least one oxidizable cysteine residue,

b) contacting the sulfur carrier protein with the target protein under conditions allowing the transfer of the sulfur moiety from the sulfur carrier protein to the target protein to obtain a thiolated target protein.

In particular, in step b) the sulfur is transferred from the sulfur carrier protein to the oxidizable cysteine residue of the target protein to obtain a sulfenylated cysteine on the target protein.

Typically, in step b) the sulfur carrier protein is conjugated to the target protein.

The term "sulfur carrier protein comprising a thiocarboxylated C-terminus" refers to any protein that comprises a C-terminal thiocarboxyl group and thereby carries a sulfur atom that can be transferred to a target protein.

The target protein is capable of receiving a sulfur atom at least one oxidizable cysteine residues by the method of the invention under conditions allowing the transfer of the sulfur moiety from the sulfur carrier protein, such as mild oxidative stress conditions.

The term "oxidizable cysteine residue" can be used interchangeable with "peroxidatic cysteine" and "redox-active cysteine". Typically, the oxidizable cysteine residue is on the surface of the protein and its side chain does not face the inside/core of the folded protein.

Typically, the conditions allowing the transfer of the sulfur moiety from the sulfur carrier protein are mild oxidative stress conditions. Mild oxidative stress conditions may be generated the presence of oxidizing reagents such as tert-butyl hydroperoxide (TBH), hydrogen peroxide (HP), peroxynitrite or diamide or combinations thereof in step b).

The method may be an in vitro method, an ex vivo or an in vivo method. Preferably, the method is carried out in vitro. In vitro refers to reactions in the test tube or in cell culture. Preferably in vitro refers to reactions in the test tube, e.g. to reactions comprising isolated proteins, preferably isolated purified proteins.

The term "conjugated" as used herein refers to the generation of a covalent bond, e.g. the generation of a covalent bond between the sulfur carrier protein and the target protein.

The term "thiolated target protein" refers to a target protein to which by the method of the invention a sulfur atom was transferred to at least one of its oxidizable cysteine residues.

The method may further comprise at least one of the steps

c) changing the conditions to stop the transfer of the sulfur moiety from the sulfur carrier protein to the target protein.

d) isolating the thiolated target protein.

In some embodiments, the sulfur carrier protein is a non-canonical ubiquitin-like protein (UBL), such as Ubiquitin related modifier 1 (Urm1). In specific embodiments, the sulfur carrier protein comprises amino acid sequence which is set out in SEQ ID NO: 1 (MAAPLSVEVEFGGGAELLFDGIKKHRVTLPGQEEPWDIRNLLIWIKKNLLKERPELFI QGDSVRPGILVLINDADWELLGELDYQLQDQDSVLFISTLHGG) or a sequence which has at least 80 % identity thereto.

The terms "at least 80% identical", "which has at least 80 % identity" and "having an amino acid sequence which is at least 80% identical" can be used interchangeably and include that the amino acid sequence is at least 80%, 81%, 82%, 83%, 84%, 85%, 86%, 87%, 88%, 89%, 90%, 91%, 92%, 93%, 94%, 95%, 96%, 97%, 98%, or 99% identical to the amino acid sequence set out.

The determination of percent identity between multiple sequences is preferably accomplished using the AlignX application of the Vector NTI AdvanceTM 10 program (Invitrogen Corporation, Carlsbad CA, USA). This program uses a modified Clustal W algorithm (Thompson et al., 1994. Nucl Acids Res. 22: pp. 4673-4680; Invitrogen Corporation; Vector NTI AdvanceTM 10 DNA and protein sequence analysis software. User's Manual, 2004, pp.389-662). The determination of percent identity is performed with the standard parameters of the AlignX application.

In some embodiments, the method does not involve E2 ubiquitin-conjugating enzyme and/or E3 ligase.

In some embodiments, the oxidizable cysteine is in proximity to a lysine, threonine and/or serine in the target protein. Preferably, the oxidizable cysteine is in proximity to a lysine. In some embodiments, thus this means that the oxidizable cysteine is in distance of 15 Å to a lysine, threonine and/or serine in the target protein, preferably that the oxidizable cysteine is in distance of 15 Å to a lysine.

In some embodiments, the target protein is a target of non-canonical UBL protein, preferably of Urm 1. Target proteins of non-canonical UBL proteins, preferably of Urm1 may be identified by mass spectrometry and validated by detecting a mass shift using SDS-PAGE and/or western blotting. In exemplary embodiments, the target proteins are Urm1 target proteins selected from the group consisting of *Hs*PRDX5, *Sc*Tdh3, *Hs*GAPDH, *Sc*Ses1, *Hs*SARS1, *Sc*Pyk1 and *Hs*PKM2.

In some embodiments, the invention refers to a method for transferring a sulfur moiety from a sulfur carrier protein to a cysteine residue of a target protein and conjugating the sulfur carrier protein and the target protein, comprising the steps:

a) providing

- a sulfur carrier protein comprising a thiocarboxylated C-terminus,
- a target protein comprising at least one oxidizable cysteine residue,

b) contacting the sulfur carrier protein with the target protein under conditions allowing the transfer of the sulfur moiety from the sulfur carrier protein to the target protein and the conjugation the sulfur carrier protein with the target protein to obtain a thiolated conjugated target protein.

In more specific embodiments, the invention refers to a method for transferring a sulfur moiety from Urm1 to a cysteine residue of a target protein of Urm1 and conjugating the Urm1 and the Urm1 target protein, comprising the steps:

a) providing

- Urm1 comprising a thiocarboxylated C-terminus,
- a Urm1 target protein comprising at least one oxidizable cysteine residue,

b) contacting Urm1 with the target protein under conditions allowing the transfer of the sulfur moiety from Urm1 to the Urm1 target protein and the conjugation Urm1 with the Urm1 target protein to obtain a thiolated Urm1 target protein which is conjugated to Urm1.

Reactions in which Urm1 is use and conjugated to the target protein are also termed "urmylation".

By the transfer of sulfur to the oxidizable cysteine residue, the oxidizable cysteine residues is protected against oxidation. Accordingly, the invention also provides methods of protecting a protein comprising a least oxidizable cysteine against oxidation of said cysteine.

The invention also relates to a novel platform and according to methods for protein conjugation. By the method of the invention, the sulfur carrier protein is covalently bound to the target protein. The inventors found that not only Urm1 is able to covalently bind to a Urm1 target protein, but that any other protein comprising a thiocarboxylated C-terminus can be covalently bound to an oxidizable cysteine, preferably in proximity to lysine, threonine and/or serine. This allows to conjugate any such target protein comprising an oxidizable cysteine to a desirable molecule, tag or marker that comprises a thiocarboxylated C-terminus. The skilled person is aware of techniques to modify the C-terminus that it contains a thiocarboxylated C-terminus.

Accordingly, also encompassed is a method for protein conjugation comprising the steps:

a) providing

- a sulfur carrier protein comprising a thiocarboxylated C-terminus,
- a target protein comprising at least one oxidizable cysteine residue,

b) contacting the sulfur carrier protein with the target protein under conditions allowing the conjugation of the sulfur carrier protein and the target protein to obtain a conjugated target protein.

Thus, the sulfur carrier protein can be any desirable molecule (comprising a thiocarboxylated C-terminus) to be covalently conjugated to the target protein. In one embodiment, the sulfur carrier protein is a detectable marker.

The detectable marker may be any conventional tag such as myc-tag, FLAG-tag, T7-tag, HA (hemagglutinin)-tag, His-tag, S-tag, TST-tag, Halo-tag, GST-tag, or myc, T7, GST or fluorescent marker, such as a fluorescent protein, such as GFP or GFP derived proteins. In one embodiment, the sulfur carrier protein is GFP with a thiocarboxylated C-terminus.

Typically, the covalent linkage occurs at a lysine, serine or threonine, preferably a lysine.

In some embodiments, in step b) the target protein and the sulfur carrier protein are mixed, preferably in equimolar amounts, in reaction buffer comprising 20 mM Tris with pH 7.5 and 200 mM NaCl.

The skilled person knows which conditions to choose in order to change the conditions to stop the transfer of the sulfur moiety from the sulfur carrier protein to the target protein. In some embodiments step c) comprises adding Laemmli sample buffer containing DTT.

Further, the skilled person is aware of methods for isolating the target protein in step b). For Example, in some embodiments step d) comprises a chromatography, preferably gel electrophoresis under denaturing conditions.

Accordingly, the application also refers to an isolated covalently conjugated protein comprising a sulfur carrier protein covalently bound to a target protein, wherein the target protein comprises a sulfenylated cysteine. Preferably the sulfur carrier protein is covalently bound a lysine, serine or threonine residue of the target protein.

Another aspect refers to a kit comprising a sulfur carrier protein comprising a thiocarboxylated C-terminus and an oxidizing reagent.

**EXAMPLES**

Research leading to subject invention started with studying Urm1 conjugation to Ahpl *in vitro* using purified components. We show that purified Urm1-SH can be efficiently attached to Ahpl and other target proteins *in vitro* using mild oxidative stress conditions in the absence of E2 enzymes or E3 ligases. We demonstrate that the conjugation depends on the thiocarboxylated C-terminus of Urm1, a redox-active cysteine in the target protein and that the covalent linkages can occur on a variety of lysine residues. Moreover, we used our mechanistic insights to engineer artificially thiocarboxylated proteins and recapitulate the conjugation reaction by using a variety of model proteins, including other UBLs and GFP. Our findings imply several fundamental consequences for the understanding of the molecular mechanisms that enable SPC, such as Urm1 to protect enzymes during oxidative stress and maintain their importance for redox homeostasis and during aging.

**Example 1. *In vitro* Urm1 conjugation of target protein**

Ubiquitin related modifier 1 (Urm1) is an evolutionarily conserved member of the ubiquitin family that adopts the β-grasp fold characteristic of all eukaryotic ubiquitin-like proteins (UBLs) (e.g. ubiquitin, SUMO, NEDD8, ATG12, UFM) (Bedford et al., 2011). Urm1 acts as a sulfur carrier protein (SCP) and is an essential member of the modification cascade that thiolates the C2 position of wobble uridine ($s^2U_{34}$) in eukaryotic tRNA anticodons via the Ncs2-Ncs6 sulfur-transferase complex (Leidel et al., 2009; Nakai et al., 2008; Noma et al., 2009; Schlieker et al., 2008). The attachment of a sulfur atom to the wobble base in the tRNA anticodon optimizes ribosomal dynamics during translational elongation (Nedialkova and Leidel, 2015; Ranjan and Rodnina, 2016) and promotes cellular responses to nutrient starvation (Bruch et al., 2020; Gupta et al., 2019; Laxman et al., 2013). The disruption of tRNA anticodon modifications can result in dramatic proteome perturbations and the onset of severe human diseases, including cancer and neurodegenerative pathologies (Close et al., 2018; Hawer et al., 2018; Nedialkova and Leidel, 2015; Rezgui et al., 2013; Schaffrath and Leidel, 2017). In addition to its role as SCP in tRNA thiolation, early reports showed that Urm1 covalently attaches to target proteins in conditions of oxidative stress in different organisms, reminiscent of other UBLs (Furukawa et al., 2000; Goehring et al., 2003; Jüdes et al., 2015; Khoshnood et al., 2017; Schlieker et al., 2008; Van der Veen et al., 2011; Wang et al., 2019).

The conjugation reaction of an UBL to its target protein starts with adenylation of its C-terminus by ATP-dependent E1 ubiquitin-activating enzymes. Next, the activated C-terminus of the UBL is relayed via active site cysteines of an E1-, E2- and E3-enzyme cascade (Passmore and Barford, 2004). Finally, the UBL is attached to a specific target protein via a covalent iso-peptide bond formed between a highly conserved diglycine motif at its C-terminus and a lysine side chain of the target protein (Cappadocia and Lima, 2018; Hochstrasser, 2000; McDowell and Philpott, 2013; Stewart et al., 2016). Similarly, the initial activation steps for SCPs start with adenylation of the C-terminus by specialized E1-like proteins. Although SCPs typically do not form thioesters, our recent work revealed the necessity of a thioester intermediate for the activation of Urm1 by its dedicated E1 enzyme, Uba4 (Pabis et al., 2020; Termathe and Leidel, 2018). Following the formation of a thioester between Uba4 and Urm1, the rhodanese domain of Uba4 transfers a persulfide group to the activated C-terminus of Urm1. The resulting thiocarboxylated C-terminus (COSH) is present in all prokaryotic and archaeal SCPs (Kessler, 2006) and is required for the thiolation of nucleic acids as well as in the biosynthesis of Molybdenum cofactor (Moco) and thiamine (Leimkühler, 2017; Shigi, 2018). However, amongst eukaryotic UBLs only Urm1 is known to carry this unique terminal modification (Urm1-SH). Hence, Urm1 and Uba4 are located at a crucial evolutionary branchpoint between prokaryotic SCPs and eukaryotic UBLs since they combine molecular features otherwise exclusively present in either SCPs or UBLs (Jüdes et al., 2015; Pedrioli et al., 2008; Xu et al., 2006).

As the role of Urm1-SH for tRNA thiolation is well established and no E2 or E3 enzymes for Urm1 have been identified so far, the potential conjugation of Urm1 to proteins (also referred to as protein "urmylation"), analogous to other UBLs, has been questioned. Nonetheless, several mass spectrometry studies have identified proteins conjugated with Urm1 under specific environmental conditions (Khoshnood et al., 2017; Schlieker et al., 2008; Van der Veen et al., 2011). *In vivo,* the most robust evidence for conjugation of Urm1 was observed for yeast Ahpl (Brachmann et al., 2020; Goehring et al., 2003; Jüdes et al., 2015; Van der Veen et al., 2011), a peroxiredoxin that acts as an antioxidant enzyme in scavenging reactive oxygen species (Lee et al., 1999). However, the biochemical details including which E2 and E3 enzymes are required for the conjugation reaction to Ahp1 or other targets remain undefined. Furthermore, as the unique COSH moiety at the C-terminus of Urm1 is absent from all other eukaryotic UBLs, its role and fate after the conjugation reaction remain elusive. Finally, the necessity of oxidative stress and redox-active cysteines for the conjugation of Ahp1 (Brachmann et al., 2020) raised the question whether the conjugation to other proteins would depend on similar target motifs and reaction conditions.

We sought to close these gaps by determining the mechanism of Urm1 conjugation to target proteins. We show that purified Urm1-SH can be efficiently attached to Ahpl and other target proteins *in vitro* using mild oxidative stress conditions in the absence of E2 enzymes or E3 ligases. We demonstrate that the conjugation depends on the thiocarboxylated C-terminus of Urm1 and a redox-active cysteine in the target protein, and that these covalent linkages can occur on a variety of lysine, serine and threonine residues. Our high-resolution crystal structures of Ahpl before and after the conjugation reaction confirm our biochemical findings and provide in-depth insights into the molecular mechanism of Urm 1 attachment.

Strikingly, we observed that Urm 1-SH transfers its sulfur moiety to the urmylated target protein, resulting in directed persulfidation of specific redox-active cysteines. Persulfidation of cysteines (also called protein S-sulfhydration) has been directly linked to aging and may be regulated by intracellular $H_2S$ levels (Zivanovic et al., 2019). However, the pathway responsible for targeted cysteine persulfidation has remained elusive. Oxidation of specific cysteines triggers the process of Urm1 conjugation, providing a molecular rationale that enables cells to protect vulnerable cysteine residues from reactive oxygen species (ROS) by a highly selective mechanism. Finally, we used our mechanistic insights to engineer artificially thiocarboxylated proteins and recapitulate the conjugation reaction on a variety of model proteins, including other UBLs and GFP, providing a platform to covalently modify proteins of interest with proteinaceous tags through this selective reaction. Our findings demonstrate how Urm1 protect proteins during oxidative stress, and reveal a critical evolutionary link between sulfur transfer and covalent modification by ubiquitin-family proteins.

## Results

### Urm1-SH directly conjugates to Ahpl *in vitro*

To study the function of Urm1 and understand the details of its protein conjugation reaction, we produced large quantities of purified Urm1-SH using two methods. *In vivo,* the cysteine desulfurase Nfs1 and the sulfur-transferase Tum 1 relay sulfur to the rhodanese domain of Uba4, which transfers an activated sulfur group to the C-terminus of Urm 1 (Figure 1A) (Noma et al., 2009). The use of thiosulfate as a sulfur source for Uba4 makes it possible to bypass Nfs1 and Tum1, thereby catalyzing the direct thiocarboxylation of Urm1 *in vitro* (Termathe and Leidel, 2018). We used this approach to produce Urm1-SH from purified *Saccharomyces cerevisiae* Urm1 and Uba4 proteins (*Sc*Urm1 and *Sc*Uba4). As an alternative strategy, we generated Urm1-SH or Urm1-OH from *Homo sapiens* and *Chaetomium thermophilum*, a thermophilic fungus harboring highly stable proteins (Bock et al., 2014), using a chitin-based inducible cleavage (intein) system (Kinsland et al., 1998). We verified all purified proteins by mass spectrometry and independently confirmed the presence of a sulfur atom in Urm1-SH by its increased gel retardation in PAGE supplemented with ((N-acryloylamino)phenyl)mercuric chloride (APM) (Figure 1B) (Igloi, 1988).

*In vivo,* Urm1 conjugation requires the presence of oxidative stressors and the catalytic cysteine (Cys62) in *Sc*Ahp1 (Brachmann et al., 2020; Van der Veen et al., 2011). Surprisingly, we observed that mutating the resolving cysteine in Ahpl (C31S) not only permitted the attachment of Urm1 *in vivo,* but even promoted the conjugation reaction in absence of N-Ethylmaleimide (NEM), a compound that irreversibly alkylates cysteines and is generally required to detect Urm1-Ahp1 conjugates in cell lysates (Figure 1C). Hence, *Sc*Ahp1$_{C31S}$ represented a very promising tool to recapitulate the conjugation reaction *in vitro.* First, we tested purified ScUrm1-OH and *Sc*Urm1-SH in combination with various purified variants of *Sc*Ahp1 (*Sc*Ahp1$_{C31S}$, *Sc*Ahp1$_{C31R}$, *Sc*Ahp1$_{C62S}$ and *Sc*Ahp1$_{C31S\ C62S}$; Figure 1C). We were not able to detect Urm1 conjugation to the Ahpl substrate by incubating wild-type *Sc*Ahp1 with *Sc*Urm1-OH or *Sc*Urm1-SH in the presence of the oxidizing agent *tert-butyl* hydroperoxide (TBH), previously shown to stimulate the reaction *in vivo* (Brachmann et al., 2020; Van der Veen et al., 2011). However, *Sc*Ahp1$_{C31S}$ and *Sc*Ahp1$_{C31R}$ formed specific conjugates with Urm1-SH in the presence of TBH, resulting in an increase in the molecular weight of Ahp1 roughly by the mass of one Urm1 molecule (Figure 1D). Of note, the conjugates formed with Urm1-SH prepared by both thiocarboxylation protocols, but not with Urm1-OH or in the absence of TBH (Figure 1D). To further characterize the conjugates, we treated the samples after the reaction with either tris(2-carboxyethyl)phosphine (TCEP) to resolve disulfide bonds, hydroxylamine (HA) to resolve thioester bonds, or DTT to resolve both (Termathe and Leidel, 2018). None of these reagents disrupted the conjugates, suggesting that a covalent iso-peptide bond is formed between Urm1-SH and *Sc*Ahp1$_{C31S}$ (Figure 1D). In agreement with previous *in vivo* data (Brachmann et al., 2020), the mutation of the peroxidatic cysteine (Cys62) in Ahpl (*Sc*Ahp1$_{C31S\ C62S}$) completely abolishes Urm1 attachment *in vitro* (Figure 1D). As different oxidative stressors can induce Urm1 conjugation *in vivo* (Jüdes et al., 2015; Van der Veen et al., 2011), we tested whether "urmylation" can similarly be triggered by different oxidative compounds *in vitro.* Indeed, conjugate formation likewise occurred in the presence of hydrogen peroxide (HP), peroxynitrite and diamide. On the other hand, methylglyoxal, di-tert-butyl disulfide and NEM did not promote the conjugation reaction, but also did not influence the stability of Urm1 conjugation if added after the reaction. None of the reagents compromised *Sc*Ahp1 integrity, and only the alkylating agents NEM and peroxynitrite negatively affected the stability of the thiocarboxyl group at the C-terminus of Urm1. This observation suggests that NEM does not promote the urmylation of Ahpl *in vivo* but rather facilitates its detection by blocking potential inhibitory factor(s) or deconjugating enzyme(s). In addition, we used *Ct*Urm1 and variants of *Ct*Ahp1 to independently confirm the necessity of the C-terminal thiocarboxyl group, oxidative stress conditions, and redox-active cysteines for the reaction to occur. In summary, our results demonstrate that Urm1, like all other canonical eukaryotic UBLs, can form a covalent bond with a target protein. However, Urm1 conjugation does not depend on the canonical cascade of E2 ubiquitin-conjugating enzymes and/or E3 ligases. Instead, the conjugation reaction requires a thiocarboxylated C-terminus of Urm1 and a peroxidatic cysteine in the target protein, which in the case of Ahp1 appears to be promoted by site-specific substitution of the resolving cysteine.

**Structure of urmylated Ahp1 complex**

To gain detailed molecular insights into the mechanisms of Urm1 conjugation, we purified and determined the structures of urmylated and unmodified *Ct*Ahp1 by macromolecular crystallography. The purified *Ct*Ahp1$_{C30S}$-Urm1$_{C55S}$ complex showed a clear shift in size in comparison to the individually purified Ahpl and Urm1 proteins (Figure 2A). We independently crystallized *Ct*Ahp1 and *Ct*Ahp1$_{C30S}$, which is equivalent to *Sc*Ahp1$_{C31S}$, and collected complete datasets at 1.75 Å and 1.85 Å resolution, respectively (Table 1). We solved both structures by molecular replacement using *Sc*Ahp1 structure (PDB ID 4DSR) and refined the corresponding atomic models to R/R$_{free}$ values of 18.8%/20.2% and 15.7%/18.3%, respectively (Table 1, PDB ID 7Q68 and 7Q69). The structures confirm that *Ct*Ahp1, like *Sc*Ahp1, forms a homodimer, which is maintained by conserved hydrophobic residues (e.g. Phe56) at the center of the dimer interface (Figure 2B) (Trivelli et al., 2003). Wild-type *Ct*Ahp1 contains a disulfide bond between Cys30 and Cys60, which indicates that in the absence of thioredoxins the dimer becomes trapped in the (post)oxidized state after release of $H_2O$. *Ct*Ahp1$_{C30S}$ cannot form the disulfide bond between the two redox-active cysteines, resulting in a structural rearrangement of the entire loop region harboring the peroxidatic cysteine (Cys60). As the mutated residue (Ser30) remains in an almost identical position as in the wildtype protein, *Ct*Ahp1$_{C30S}$ structurally mimics the reduced form that can accumulate oxidized/sulfenylated Cys60 (Figure 2B). Due to the presence of reducing agent in the crystallization condition, there were no indications for the oxidation of the thiol group of Cys60.

Next, we obtained crystals of the *Ct*Ahp1$_{C30S}$-Urm1$_{C55S}$ complex, which after several rounds of optimization, diffracted to an overall resolution of 2.5 Å (Table 1). We solved the structure by molecular replacement using the high-resolution model of *Ct*Ahp1$_{C30S}$ in combination with the model of *Ct*Urm1 taken from the Uba4$_{C202K}$7-Urm 1 complex (PDB ID 6YUC). The final model was refined to R/R$_{free}$ values of 23.4%/27.2% obeying perfect stereochemistry (Table 1, PDB ID 7Q5N). The structure shows an almost unchanged homodimer of *Ct*Ahp 1$_{C30S}$, where the Lys63 residue from each Ahp1 molecule forms a covalent isopeptide bond between the lysine sidechain and the C-terminus of Urm1 (Figure 2C). The overall dimeric structure of Ahp1 and the position of its peroxidatic cysteines remain almost identical in the presence or absence of Urm1 (Figure 2C). The asymmetric unit contains three individual *Ct*Ahp1$_{C30S}$-Urm1$_{C55S}$ dimers, which are all linked via the same peptide bond between Ahp1$_{Lys63}$ and Urm1$_{Gly111}$. The conformations of the three urmylated Ahpl homodimers are superimposable, but the relative position of the respective Urm1 molecules, the quality of the density map, and the associated B-factors differ between the individual copies (Figure 2C). These variations arise most likely due to different packing environments in the crystal lattice and might result from the fact that Ahp 1 and the attached Urm1 do not form an extensive interaction surface. However, there are no indications that a hexameric form of *Ct*Ahp1$_{C30S}$-Urm1$_{C55S}$ or a dimerization of Urm1 between neighboring complexes occurs in solution or has any functional significance *in vivo.* Of note, the Urm1-Urm1 packing interfaces are almost identical, and changes in relative positioning are not caused by structural changes of the individual Ahp1 and Urm1 proteins but are strictly related to variations in the flexible C-terminal region of Urm 1. Strikingly, we found three distinct types of Urm 1 positioning relative to the attachment site around Ahp1$_{Lys63}$ (Figure 2D). These differences are not only visible at the domain level, but also the specific conformation of interacting residues in Urm1 and Ahpl varies between the observed attachment sites. The structure of *Sc*Ahp1 bound to its recycling factor thioredoxin 2 (Trx2) has been determined in different reaction states (Lian et al., 2012, 2020), showing that Trx2 directly interacts with Cys30 of Ahpl to resolve the disulfide bond formed between the redox-active cysteines. In contrast, our urmylated Ahp1 structure places Urm1 close to the peroxidatic Cys62, indicating that certain conformations of Urm1 attachment are compatible with the binding of Trx2 while others are sterically impossible. Our crystallographic data confirm the formation of a covalent iso-peptide bond between Ahpl and Urm1. Furthermore, we identify Lys63 as the major attachment site in *Ct*Ahp1 and reveal the structural variability of the conjugated complex, which is linked close to the peroxidatic cysteine.

**Urm1 can conjugate to various sites in Ahp1**

Although the crystal structure of *Ct*Ahp1$_{C30S}$-Urm1$_{C55S}$ displays an exclusive linkage with unambiguous density between *Ct*Urm1$_{Gly111}$ and *Ct*Ahp1$_{Lys63}$ (Figure 3A), we previously found that Urm1 can be conjugated to different lysine residues of *Sc*Ahp1 *in vivo* (Brachmann et al., 2020). Hence, we analyzed the products of the *in vitro* conjugation reactions by mass spectrometry to independently confirm the presence of HGG-K linked peptides after digestion with chymotrypsin (Figure 3B). Since ubiquitin is known to form poly-ubiquitin chains on target proteins, we asked whether we could detect this kind of polymerization for Urm1 conjugates. In our dataset, the discrete molecular weights of the conjugates indicated that internal conjugation events did not occur between Urm1 molecules under these conditions. In contrast to the exclusive Lys63-mediated linkage in our crystals, we identified additional lysine residues of *Ct*Ahp1 (Lys44, Lys63, Lys99, Lys141, Lys156 and Lys171) conjugated to the C-terminus of *Ct*Urm1. Furthermore, we used a *Ct*Ahp$1_{C30S\ K63R}$ mutant, lacking the most commonly conjugated Lys63, to promote the occurrence of rare conjugation events and detected urmylation of Lys71, which is in close proximity to Cys60. Similarly, in *Sc*Ahp1, which harbors more lysine residues than *Ct*Ahp1 (Figure 3B), we detected various additional attachment sites by mass spectrometry (Lys32, Lys47/Lys48, Lys79, Lys81, Lys107, Lys124 and Lys156). Next, we mutated various lysine residues in *Ct*Ahp1 and *Sc*Ahp1 individually and performed *in vitro* conjugation reactions using these purified Ahpl variants. Our results clearly demonstrated that even after simultaneously mutating the major attachment sites in *Ct*Ahp1 (*Ct*Ahp$1_{K44R\ K63R\ K99R}$), residual conjugates were still detectable (Figure 3C). In *Sc*Ahp1, only the simultaneous mutation of several lysine residues (*Sc*Ahp$_{1K32R,\ K47R,\ K48R,\ K124R,\ K1S6R}$) slightly reduced urmylation levels (Figure 3D). Last, we tested the effects of these lysine mutants *in vivo* in S. *cerevisiae*, and did not observe any phenotypic consequences in the presence of TBH (Figure 3E) nor the complete inhibition of conjugation (Figure 3F).

Of note, the linked lysine residues in *Ct*Ahp 1 and *Sc*Ahp1 are not evolutionarily conserved, and their spatial distribution does not reveal a specific distribution pattern (Figure 3G). However, our complementary mass spectrometry and mutational analyses show that the attachment of Urm 1 to lysine residues in Ahp 1 exclusively depends on the presence of a peroxidatic cysteine in the target protein. Our results in *Sc*Ahp1 emphasize that in the absence of a particular lysine residue a different residue will be used as target with similar efficiency, indicating low specificity for the particular residue, motif, or structural context at which urmylation can occur.

**Urm1 conjugates to other residues in the absence of lysines**

As non-canonical linkages to serine, threonine or cysteine residues have also been described for other UBLs (McDowell and Philpott, 2013), we analyzed our mass spectrometry data for any such possible Urm1 linkage on Ahpl (*Sc*Ahp$1_{C31S}$ and *Ct*Ahp$1_{C30S}$). Strikingly, we found Urm1 being conjugated to several serine and threonine residues in *Sc*Ahp$1_{C31S}$ and few in *Ct*Ahp$1_{C30S}$ (Figure 3B). To test whether lysine residues are essential for catalyzing the conjugation reaction, we simultaneously replaced all lysine residues in *Sc*Ahp$1_{C31S}$ and *Ct*Ahp$1_{C30S}$ with arginine residues (*Sc*Ahp$1_{C31S\text{-}KtoR}$ and *Ct*Ahp$1_{C30S\text{-}KtoR}$). Strikingly, we still observed the formation of Ahp1-Urm1 conjugates with these lysine-less Ahpl variants in the presence of Urm1-SH and mild oxidative stress *in vitro* (Figure 4A). We mutated semi-conserved serine and threonine residues in the spatial proximity of the peroxidatic cysteine and other nearby residues in *Ct*Ahp1 (Arg151) or *Ct*Urm1 (Leu108, His109) (Figure 4B). Whereas none of the mutations affected the conjugation efficiency in the presence of lysine residues, the simultaneous depletion of all possible serine and threonine attachment sites that are in proximity to the peroxidatic cysteines in *Sc*Ahp$1_{C31S^-KtoR}$ and *Ct*Ahp$1_{C30S^-KtoR}$ strongly reduced the formation of Ahp1-Urm1 conjugates *in vitro* (Figure 4C and Figure 8A). Therefore, these serine and threonine residues can act as alternative attachment sites, but play no significant role during the canonical reaction when lysines are present. Since the lysine-less variants still showed traces of conjugates, we used mass spectrometry to unambiguously test for other low-frequency attachment sites. This approach allowed us to confirm non-canonical ester linkages between *Sc*Urm1 and Ser59 and Thr61 sites on *Sc*Ahp1 and between CtUrm1 and Thr57 and Thr59 on *Ct*Ahp1 (Figure 4D). In summary, our detailed mutational analyses indicate that Urm1-SH preferably conjugates to lysine residues, but is also able to attach to other amino acid side chains as has been shown for other UBLs.

**Urm1-SH transfers sulfur to the peroxidatic cysteine of Ahpl**

To the best of our knowledge, Urm1 is the only eukaryotic UBL that carries a C-terminal thiocarboxyl group, which we showed is essential for the conjugation reaction. Intriguingly, this unique sulfur atom was undetectable at the site of linkage in our mass spectrometry analyses above. Hence, we were curious about the fate of the attached sulfur moiety during and after peptide-bond formation. To address this question, we generated Urm1-SH carrying radioactive sulfur ($^{35}S$) at its C-terminus (see Methods and Figure 8B) for *in vitro* "urmylation" reactions (Figure 8C). We incubated $^{35}S$-labelled thiocarboxylated *Sc*Urm1 and *Ct*Urm$1_{C55S}$ with various variants of *Sc*Ahp1 and *Ct*Ahp1 in the presence or absence of TBH (Figure 4E and Figure 8D-G). Strikingly, we detected a very efficient transfer of $^{35}S$ from Urm1-$^{35}SH$ to *Sc*Ahp1 and *Ct*Ahp1 variants (ScAhp$1_{C31S}$, *Sc*Ahp$1_{C31S\text{-}KtoR}$, *Ct*Ahp$1_{C30S}$ and *Ct*Ahp$1_{C30S\text{-}KtoR}$). The mutation of the peroxidatic cysteine not only diminished the Urm1 conjugation reaction but also blocked the sulfur transfer between Urm1-SH and Ahp1 (ScAhp$1_{C62S}$, *Ct*Ahp$1_{C60S}$ and *Ct*Ahp $1_{C30S\ C60S}$). In the case of *Sc*AhpI$_{C31S\ C62S}$, we still observed a weak level of sulfur transfer, which is most likely mediated by an additional cysteine residue in *Sc*Ahp1 (Cys120), which is not present in *Ct*Ahp1. Interestingly, the $^{35}S$ transfer to Ahp1 cannot only occur on the Urm1-conjugated molecule of the Ahp1 homodimer, but also to the other unconjugated chain of the homodimer.

Next, we searched our mass spectrometry datasets of the complexes for possible forms of peptide S-sulfhydration. Strikingly, we identified persulfidation of the catalytically active peroxidatic cysteine in both *Sc*Ahp1 and *Ct*Ahp1 (Figure 4F), indicating that Urm1-SH transfers its sulfur atom to the sulfenylated cysteine on its target protein. The archaeal TfuA protein, which is also C-terminally thiocarboxylated, was recently shown to replace oxygen with sulfur in the peptide backbone of its target protein (Liu et al., 2021). We reanalyzed our crystallographic data to identify the precise spatial position of the post-translational modification. A prominent density next to the sulfur atom of the cysteine side chain was identifiable in the crystal structure of the *Ct*Ahp$1_{C30S}$-Urm$1_{C55S}$ complex, which was absent from individual Ahpl structures alone (Figure 4G). To confirm the position and identity of the additional sulfur moiety, we collected diffraction data for the *Ct*Ahp $1_{C30S}$-Urm $1_{C55S}$ crystals at suitable wavelengths and calculated anomalous difference Fourier maps (Figure 4H). Foremost, we did not observe an anomalous signal at the position of the backbone oxygen atom, excluding the possibility of a sulfur substitution reaction, like TfuA. Furthermore, we obtained a structure at 1.1 Å resolution of the non-reacted *Ct*Ahp$1_{C30S}$, which we repurified after the conjugation reaction with Urm1-SH (Table 1, PDB ID 7Q6A). Although, this Ahpl sample underwent an identical treatment, we did not observe any extra densities around Cys60 without Urm1 conjugation (post-reaction; Figure 4G). Our complementary biochemical, mass spectrometry, and crystallographic analyses show that the detected persulfide group is absent in the starting material and that the post-translational thio-modification of the cysteine side chain in Ahpl occurred during the conjugation reaction with Urm1-SH.

We propose a very simple reaction mechanism that directly couples urmylation with the observed sulfur transfer to the target protein (Figure 41). In detail, the exposure of a redox active cysteine in Ahpl to oxidative stress leads to the sulfenylation of its side chain (-SOH). Urm1-SH recognizes the sulfenylated cysteine and after condensation forms a transient acyl disulfide intermediate. The reduction of the transient acyl disulfide intermediate can lead to the recycling of Urm1-SH. Otherwise, a lysine, serine or threonine residue in proximal distance from the linked Cys-Cys pair triggers a nucleophilic attack on the acyl disulfide. This reaction scheme simultaneously produces a persulfidated cysteine and a covalent conjugation site between the C-terminus of Urm1 and respective lysine, serine or threonine residue in the target protein (Figure 41).

Our results show that the Urm1-conjugation reaction is mechanistically and directly coupled to the process of cysteine persulfidation. Foremost, our results demonstrate that Urm1 can specifically transfer the sulfur atom of its thiocarboxyl group to redox-active cysteine residues in Ahp1, if the target is exposed to oxidative conditions.

**Cellular Urm1 targets are persulfidated *in vitro***

It is important to emphasize that Ahp1, an antioxidant enzyme, represents an unusual target of Urm 1 under oxidative stress conditions. For instance, even under optimized experimental conditions, we detected only weak conjugation activity of wildtype Ahpl *in vitro* and we had to use *Sc*Ahp$1_{C31S}$ or *Ct*Ahp$1_{C30S}$ to detect substantial conjugation activity. To identify and characterize additional Urm1 targets *in vitro,* we used a list of 547 candidate proteins that had been identified in yeast by LC-MS/MS after Urm1 pull-down. 21 of the hits were significantly enriched upon NEM treatment, recapitulating the behavior of Ahpl (Figure 5A). We decided to focus on a shortlist of potential Urm1 targets from yeast and humans based on available purification protocols. Hence, we used Tdh3, Ses1 and Pyk1 (Jurica et al., 1998; Liu et al., 2012; Weygand-Durasevic et al., 1987) as well as their human homologues (PRDX5 (human homologue of Ahpl), GAPDH (human homologue of Tdh3), SARS1 (human homologue of Ses1) and PKM2 (human homologue of Pyk1). Strikingly, we were able to detect Urm1 conjugates for the entire set of yeast and human targets *in vitro* using either *Sc*Urm1-SH or *Hs*Urm1-SH, respectively (Figure 5B). Formation of these conjugates required the addition of an oxidizing agent (Figure 5B) and only occurred in the presence of Urm1-SH, but not Urm1-OH (Figure 9A). Unlike Ahp1, these targets showed robust Urm1 conjugation without introducing additional mutations or removing potential resolving cysteines (Figure 9B). To confirm the formation of covalent peptide bonds, we performed exhaustive mass spectrometry analyses to identify numerous HGG-K, HGG-T and HGG-S linked peptides and map the individually conjugated residues (Figure 5C and Figure 9C/D). As the conjugation of Urm1 to Ahpl requires at least one peroxidatic cysteine, we tested whether known catalytic cysteines are necessary for the conjugation reaction to the additional target proteins. We observed a complete loss of Urm1 conjugation to *Hs*PRDX5, ScTdh3 and *Hs*GAPDH when a single peroxidatic cysteine was mutated (Figure 5D). The mutation of individual cysteine residues in the other targets only partially reduced the Urm1 conjugation efficiency, indicating that several cysteines can support the Urm1 conjugation reaction in these targets (Figure 9B). Furthermore, we used Urm1-$^{35}$SH for *in vitro* conjugation of yeast targets and observed the direct transfer of sulfur during Urm1 conjugation onto *Sc*Tdh3, *Sc*Ses1 and *Sc*Pyk1 in the presence of diamide (Figure 5E). Furthermore, we used mass spectrometry to verify that the sulfur atom from Urm1-SH was transferred, leading to persulfidation of individual cysteine residues in *Hs*GAPDH, ScTdh3 and *Hs*PRDX5 (Figure 5C). Consistent with the observation that mutation of individual cysteines did not abolish Urm 1 conjugation, we found that indeed more than one peroxidatic cysteine was persulfidated in *Sc*Ses1, *Hs*SARS1, *Sc*Pyk1 and *Hs*PKM2 (Figure 5C). Our data thus uncover the previously unknown source of the sulfur donor for targeted persulfidation of eukaryotic proteins.

**Any thiocarboxylated C-terminus can conjugate to proteins**

We established that Urm1 can be covalently conjugated to various target proteins *in vitro* without the need for specific E2 enzymes and E3 ligases. The intein system that we used to generate Urm1-SH above enables us to produce virtually any protein with a thiocarboxylated C-terminus. Therefore, we tested whether other UBLs that harbor the C-terminal GlyGly-motif can also be conjugated to the identified Urm1 target proteins in response to oxidative stress. Using purified Ubiquitin-SH or SUMO-SH (Figure 6A), we were able to attach these synthetically thiocarboxylated UBLs to *Sc*Ahp1 and ScTdh3 under the same experimental conditions that worked for Urm1-SH (Figure 6A). As expected, ubiquitin-OH and SUMO-OH that lack the thiocarboxyl group did not form conjugates with the target proteins. We used mass spectrometry to map all conjugation sites. Interestingly, the pattern of conjugation sites differs between the different thiocarboxylated UBLs (Figure 6B). Nonetheless, we confirmed the identity of the peptides linked to the C-termini (Figure 6B) and found that the sulfur is similarly deposited as a persulfide on the same oxidizable cysteines in the target proteins (Figure 6B).

Next, we tested whether the highly conserved C-terminal GG-motif of UBLs is required for the conjugation reaction. We replaced the terminal Gly99 of *Sc*Urm1 with all other canonical amino acids and produced the thiocarboxylated versions via the intein system. Strikingly, the presence of nearly any amino acid at the C-terminus permitted the conjugation reaction, as long as the protein remained thiocarboxylated (Figure 6C). *Sc*Urm1$_{G99C}$ was not thiocarboxylated by the intein system, since it undergoes autocleavage before the final cleavage and elution step could be induced with ammonium sulfide. We also observed reduced conjugation efficiencies for *Sc*Urm1$_{G99E}$ and *Sc*Urm1$_{G99H}$ for reasons that remain unclear. When removing both C-terminal glycine residues (*Sc*Urm1$_{H\Delta GG}$), histidine (His97) is located at the C-terminus, which also led to strongly reduced conjugation efficiency. However, when histidine is replaced by alanine (*Sc*Urm1$_{H97A\Delta GG}$), or when both glycine were mutated to alanine or serine (*Sc*Urm1$_{G98A,G99A}$, *Sc*Urm1$_{G98S,G99S}$), Urm1 did conjugate even without the characteristic GG-motif. In summary, our data show that Urm1 variants carrying any thiocarboxylated amino acid at their C-termini can undergo a covalent attachment to defined target proteins *in vitro.* Next, we tested whether the attachment requires the typical β-grasp fold of UBLs or whether an unrelated thiocarboxylated model protein could be attached to Urm1 targets *in vitro.* We generated thiocarboxylated versions of GFP (GFP-SH) and a fusion protein between GFP and *Sc*Urm1 (GFP-Urm1-SH). Both artificially thiocarboxylated proteins can be conjugated to *Sc*Ahp1$_{C31S}$ or *Sc*Pyk1 (Figure 10A). As GFP-SH contains a C-terminal lysine residue, we verified the formation of a conjugated XK-K peptide (Figure 10B) by mass spectrometry.

The results of our *in vitro* studies suggest that the highly conserved C-terminus of Urm1 is functionally not required for conjugation. However, it is important to highlight that by using the intein system to produce thiocarboxylated proteins, we can bypass the naturally occurring reaction catalyzed by the ATP-dependent E1 activating enzyme Uba4. Our recent work showed that the C-terminus of Urm1 is positioned deep inside the active site of Uba4 and that it most likely remains buried throughout the entire process of thiocarboxylation (Figure 10D) (Pabis et al., 2020). We performed an ATP hydrolysis assay to test whether different Urm1 variants and model proteins are able to activate Uba4 *in vitro* (Figure 10C). Strikingly, only Urm 1 and the GFP-Urm 1 fusion protein induced the ATPase activity of Uba4. Any variation in the C-terminus of Urm1 (*Sc*Urm1$_{HG\Delta C}$, *Sc*Urm1$_{H\Delta GG}$, *Sc*Urm1$_{HG99W}$, *Sc*Urm1$_{HAA}$, *Sc*Urm1$_{Hss}$) failed to activate Uba4 (Figure 10C). Furthermore, the use of ubiquitin, SUMO, GFP or a GFP variant carrying the six most C-terminal residues of Urm1 (GFP-U6) did not induce a detectable Uba4 response (Figure 10C). Finally, we performed Uba4-mediated thiocarboxylation of GFP-Urm1 *in vitro* and subsequently confirmed its direct conjugation to *Sc*Ahp1, ScTdh3 and *Sc*Ses 1 (Figure 10E).

In summary, our findings highlight the broad reactivity of various thiocarboxylated proteins with Urm1 target proteins *in vitro* under mild oxidative stress conditions. Even if all other UBLs are highly similar and carry the same C-terminal GlyGly-motif, the high specificity and selectivity between Urm 1 and Uba4 strongly suggest that Uba4 exclusively thiocarboxylates Urm1 *in vivo,* thereby ensuring that Urm1 is unique in its ability to covalently attach to target proteins independent of E2 or E3 enzymes and simultaneously catalyze persulfidation of its target.

**Sulfur transfer by the Uba4-Urml pathway to proteins is not linked to the $H_2S$ trans-sulfuration pathway**

Hitherto, the gaseous $H_2S$ molecule was thought to constitute the major cellular response signal to initiate the protection of cysteines by persulfidation upon oxidative stress (Kimura and Kimura, 2004; Mustafa et al., 2009; Zivanovic et al., 2019). Cys3, Cys4 and Tum1 are key components of the trans-sulfuration pathway in yeast and were shown to regulate intracellular $H_2S$ levels. After obtaining experimental evidence for the existence of Urm1-SH mediated persulfidation of cysteine residues in target proteins, we asked whether the previously known pathway is directly or indirectly linked to Urm 1 and its role in tRNA and protein thiolation. We generated a panel of yeast strains carrying individual deletions of trans-sulfuration pathway components or urmylation pathway components (*ahp1Δ, urm1Δ, tum1Δ, ncs6Δ, cys3Δ* and *cys4Δ*) or double deletions of a trans-sulfuration pathway component and *URM1* (*cys3Δurm1Δ* and *cys4Δurm1Δ*). We found that only the deletion of *AHP1* leads to a measurable growth defect in response to the presence of TBH (Figure 7A). The deletion of *URM1* did not affect this response, confirming that Urm1 conjugation is not essential for the function of Ahpl (Brachmann et al., 2020; Van der Veen et al., 2011). We also observed a modest growth defect for *cys3Δ* and *cys4Δ* mutants, in contrast to deletions of *URM1* or known components of the tRNA thiolation pathway, which grew normally. To test whether the trans-sulfuration pathway is linked to Urm1 activity and whether the Uba4/Urm1 pathway regulates $H_2S$ levels independently, we analyzed the generation of $H_2S$ in the different deletion strains. As expected, the *cys3Δ* and *cys4Δ* strains displayed dramatically altered $H_2S$ levels. However, the deletion of *URM1, TUM1* or *NCS6* had no detectable impact on intracellular $H_2S$ levels (Figure 7B). The *cys3Δurm1Δ* and *cys4Δurm1Δ* double mutants display phenotypes similar to *cys3Δ* and *cys4Δ* strains alone. Our data thus indicate that Urm1 does not regulate $H_2S$ directly or via Cys4/Cys3. Next, we analyzed *in vivo* conjugation rates of Urm1 to Ahpl and tRNA thiolation levels in the various deletion strains. Importantly, loss of Tum 1, but not Ncs6, strongly reduces Urm1 conjugation to Ahpl *in vivo* (Figure 7C). Therefore, Urm1 conjugation and protein persulfidation do not require tRNA thiolation. Consistent with previous studies, the elimination of components of the tRNA thiolation cascade, like Urm1 or Ncs6, completely abolishes the formation of $mcm^5s^2U_{34}$ in tRNAs from these strains (Figure 7D). In contrast, inactivation of Cys3 or Cys4 leads to a reduction of tRNA modification levels, while the *AHP1* knockout has no effect. The reduction in tRNA thiolation levels could be affected by the availability of intracellular sulfur sources that are regulated by Cys4 and Cys3 in the cysteine biosynthesis pathway. Nonetheless, the inactivation of Cys3 or Cys4 does not affect the Urm1 conjugation levels of Ahp1, indicating that Urm1-SH is formed at similar levels. Foremost, we show that persulfidation of cysteines by Urm1 is independent of the previously established trans-sulfuration pathway. Finally, our results indicate that protein conjugation and cysteine persulfidation by Urm1 are also independent of its role in tRNA thiolation. Altogether, we reveal an unexpected function of Urm1, which appears to act as a universal SCP for the regulation of amino acid persulfidation and tRNA base thiolation.

**Buffers**

**Buffers used for thiocarboxylated proteins:**

**Lysis Buffer:** 30 mM Tris-HCl pH 7.5, 300 mM NaCl, 30 mM imidazole, 0.15 % TX-100, 10 mM $MgSO_4$, 10 mg/ml DNase, 1 mg/ml lysozyme, 10% glycerol and a cocktail of protease inhibitors

**Elution Buffer:** 30 mM Tris-HCl pH 7.5, 300 mM NaCl, 500 mM imidazole and 10 % glycerol

**Dialysis Buffer:** 30 mM Tris-HCl pH 7.5 and 500 mM NaCl

**Cleavage buffer:** 30 mM Tris-HCl pH 8.5, 500 mM NaCl and 35 mM ammonium sulfide or 50 mM DTT

**Protein Storage Buffer:** 20 mM Tris-HCl pH 7.5, 200 mM NaCl

**Buffers used for all other proteins:**

**Lysis Buffer:** 20 mM Tris-HCl pH 7.5, 200 mM NaCl, 10 mM imidazole, 0.15% TX-100, 10 mM $MgCl_2$, 1 mM β mercaptoethanol, 10 mg/ml DNase, 1 mg/ml lysozyme, 10% glycerol and a cocktail of protease inhibitors

**Elution Buffer:** 30 mM Tris-HCl pH 7.5, 300 mM NaCl, 500 mM imidazole 1 mM β mercaptoethanol, and 10 % glycerol

**Protein Storage Buffer:** 20 mM Tris-HCl pH 7.5, 200 mM NaCl and 1 mM DTT

***In vitro* Urmylation reaction buffer:**

20 mM Tris-HCl pH 7.5, 200 mM NaCl

**Thiocarboxylation reaction buffer:**

20 mM HEPES pH 8.5, 150 mM NaCl and 2 mM $MgCl_2$

**De-Sulfuration reaction buffer:**

20 mM HEPES pH 8.5, 150 mM NaCl, 2 mM $MgCl_2$, and 80 μM pyridoxal phosphate

**Crystallization buffers:**

***Ct*Ahp1, *Ct*Ahp1$_{C30S}$**

2 M Ammonium Sulfate and 0.1 M Sodium cacodylate pH 6.5

For cryoprotection: 2 M Ammonium Sulfate and 0.1 M Sodium cacodylate pH 6.5 and 50 % glycerol ***Ct*Ahp1$_{C30S}$ -*Ct*Urm1$_{C55S}$ Complex:**

0.05 M Zinc Acetate Dihydrate, 0.1 M Sodium Cacodylate pH 6.5, 4 % w/v polyethylene glycol 8000 and 30 % w/v ethylene glycol

For cryoprotection: 0.05 M Zinc Acetate Dihydrate, 0.1 M Sodium Cacodylate pH 6.5, 4 % w/v polyethylene glycol 8000 and 50 % w/v ethylene glycol

**Protein Expression and Purification**

Wild-type, mutants, and truncated proteins were expressed in BL21(DE3) pRARE in LB or TB media at 37 °C for 6 h and overnight induction with 0.5 M IPTG at 20 °C. Bacterial pellets were resuspended in lysis buffer (20 mM Tris-HCl pH 7.5, 200 mM NaCl, 10 mM imidazole, 0.15% TX-100, 10 mM $MgCl_2$, 1 mM β-mercaptoethanol, 10 mg/ml DNase, 1 mg/ml lysozyme, 10% glycerol and a cocktail of protease inhibitors) and lysed to homogeneity using a high pressure homogenizer Emulsiflex C3 (Avestin). The proteins were purified with Ni-NTA agarose (Qiagen) under standard conditions. Tags were cleaved with TEV protease and removed with a second Ni-NTA purification step. Subsequently, the proteins were purified by size-exclusion chromatography (SEC) on HiLoad 26/600 Superdex 200 and HiLoad 16/600 Superdex 75 prep grade columns (GE Healthcare) using AKTA™ start. Purified proteins were stored at -80 °C in storage buffer (20 mM Tris-HCl pH 7.5, 200 mM NaCl and 1 mM DTT).

**Thiocarboxylated protein expression and purifications**

To obtain thiocarboxylated *Hs*Urm1, *Sc*Urm1 *Ct*Urm1, *Sc*Ubiquitin, *Sc*Sumo and GFP, the sequences of the respective proteins were N-terminally Intein-CBD-$His_6$ fused and overexpressed in E. *coli* and purified according to (Kinsland et al., 1998; Termathe and Leidel, 2018) with modifications. In brief, the bacterial pellet was resuspended in lysis buffer without reducing agent (30 mM Tris-HCl pH 7.5, 300 mM NaCl, 30 mM imidazole, 0.15 % TX-100, 10 mM $MgSO_4$, 10 mg/ml DNase, 1 mg/ml lysozyme, 10% glycerol and a cocktail of protease inhibitors) and lysed to homogeneity. The lysate was passed through a Ni-NTA column, and, following washes, the fusion protein was eluted with elution buffer (30 mM Tris-HCl pH 7.5, 300 mM NaCl, 500 mM imidazole and 10 % glycerol). The eluates were dialyzed overnight to chitin-column buffer (30 mM Tris-HCl pH 7.5 and 500 mM NaCl) and applied on a chitin column. The column was washed with chitin-column buffer and the cleavage of the tag was induced through incubation with cleavage buffer (30 mM Tris-HCl pH 8.5, 500 mM NaCl and 35 mM ammonium sulfide or 50 mM DTT) for 16 h at 4 °C. This approach enabled us to purify proteins with C-termini that were carboxylated (-OH) by using DTT or thiocarboxylated (-SH) by using ammonium sulfide and without additional residues at the N-terminus (Kinsland et al., 1998). The eluted proteins were further purified by size-exclusion chromatography on a HiLoad 16/600 Superdex 75 column on AKTA™ start system and stored at -80 °C in storage buffer (20 mM Tris pH 7.5 and 200 mM NaCl) for further use. The presence of thiocarboxylated C-terminus was confirmed by running the protein on a *polyacrylamide gel containing ([N-acryloylamino] phenyl)mercuric chloride (APM).* The Laemmli sample buffer without any reducing agent was used. For protein visualization, the gels were stained with Coomassie *Brilliant Blue.* Of note, we removed a non-conserved surface cysteine (Cys55) in *Ct*Urm1 to circumvent super-shifting in the APM gels. Positioning a cysteine at the C-terminus of Urm1 (*Ct*$Urm1_{G111C}$) also led to a retardation of the purified protein in the APM gel.

**Thiocarboxylation of Urm1 by Uba4**

Uba4-mediated thiocarboxylation of Urm1 was performed as described (Termathe and Leidel, 2018). Briefly, 20 μM of Urm1 was mixed with 10 μM of Uba4 in the thiocarboxylation buffer (20 mM HEPES pH 8.5, 150 mM NaCl and 2 mM $MgCl_2$), supplemented with 5 mM ATP, 5 mM TCEP and 180 μM of sodium thiosulfate ($Na_2S_2O_3$) was added to the reaction mix and incubated for 1 h at 30 °C. For Urm1-SH, samples were desalted using PD SpinTrapTM G-25 (Cytiva) columns and either loaded on SDS-PAGE gels supplemented with 20 μM of APM to visualize the shift of the thiol group or buffer exchanged into 40 mM ammonium acetate and analysed by ESI-MS. Thiocarboxylation of Urm1 was scaled up and Urm1-SH was purified by using Superdex 200 Increase 10/300 GL (Cytiva) on ÄKTA™ start system. The purified Urm1-SH was snap frozen and stored at -80 °C in storage buffer (20 mM Tris pH 7.5 and 200 mM NaCl) for further use.

**Mass determination of thiocarboxylated proteins by mass spectrometry**

Carboxylated (-OH) and thiocarboxylated (-SH) protein samples were analyzed using micrOTOF-Q II mass spectrometer (Bruker Daltonics, Germany) equipped with an electrospray ionization source. The instrument was calibrated prior to measurements with ESI-L Low Concentration Tuning Mix (Agilent Technologies). Samples were desalted on Amicon Ultra-0.5 3K (Millipore) using 0.05 %-0.1 % formic acid (FA) as a washing solution. Samples at a protein concentration of about 0.2 mg/ml in formic acid (from 0.0125 % up to 1 %) and acetonitrile (from 25 % to 50 %) were directly infused to mass spectrometer with a syringe pump at a flow rate of 6 μl/min. Mass spectrometer was operated in positive mode with a spray voltage of 4500 V and dry gas temperature of 180 °C. MS scans were acquired over a mass range of m/z 500-3000. The MS spectra were processed with Maximum Entropy Deconvolution algorithm in Data Analysis 4.1 software (Bruker Daltonics, Germany).

***In vitro* Urm1 conjugation / urmylation assay**

20 μM of Ahpl and 10 μM of carboxylated or thiocarboxylated Urm1 were mixed in reaction buffer (20 mM Tris pH 7.5 and 200 mM NaCl). 0.5 mM *tert*-Butyl hydroperoxide (TBH) was included and excluded as indicated. The reaction mix was incubated for 30 min at 37 °C for *Chaetomium thermophilum, and* 30 °C for *Saccharomyces cerevisiae* and *Homo sapiens* proteins. For the confirmation of the isopeptide bond TCEP, DTT and hydroxylamine were added at a final concentration of 5 mM after the initial 30-minute reaction and further incubated for 5 min at 37 °C or 30 °C, respectively. The reactions were stopped by adding Laemmli sample buffer containing DTT and incubated for 5 min at 95 °C. Subsequently the samples were loaded on Bolt™ 4-12 % Bis-Tris Plus Gels (Thermo Fisher Scientific). For protein visualization, the gels were stained with Coomassie *Brilliant Blue*.

***In vitro* Urm1 conjugation under different oxidative agents**

20 μM of Ahp1 and 10 μM of thiocarboxylated Urm1 were mixed in reaction buffer (20 mM Tris pH 7.5 and 200 mM NaCl). Oxidative agents such as *tert*-Butyl hydroperoxide (TBH), Hydrogen peroxide (HP), Diamide, Peroxynitrite, Methylglyoxal, and DTB-Disulfide at final concentration of 0.5 mM were included and excluded as indicated. The reaction mix was incubated for 30 min at 37 °C for *C. thermophilum, and* 30 °C for *S. cerevisiae.* The reactions were stopped by adding Laemmli sample buffer containing DTT and incubated for 5 min at 95 °C. Subsequently the samples were loaded on Bolt™ 4-12 % Bis-Tris Plus Gels (Thermo Fisher Scientific). For protein visualization, the gels were stained with Coomassie *Brilliant Blue.*

**Stability of Ahpl and thiocarboxylated Urm1 to oxidative agents**

10 μM of Ahpl or 10 μM of thiocarboxylated Urm1 were added in reaction buffer (20 mM Tris pH 7.5 and 200 mM NaCl) and oxidative agents such as TBH, HP, Diamide, Peroxynitrite, Methylglyoxal, and DTB-Disulfide at a final concentration of 0.5 mM were included or excluded as indicated. The reaction mix was incubated for 30 min at 30 °C. The reactions were stopped by adding Laemmli sample buffer containing DTT for Ahp1 and Laemmli buffer without reducing agents for Urm1-SH and incubated for 5 min at 95 °C. Subsequently, the samples of Ahpl were loaded on Bolt™ 4-12 % Bis-Tris Plus Gels (Thermo Fisher Scientific). Samples of Urm1-SH were loaded on SDS-Page gels supplemented with 20 μM of APM. For protein visualization, the gels were stained with Coomassie *Brilliant Blue.*

**Urm1-Ahp1 complex formation and purification for Crystallization**

Large scale urmylation of *Ct*Ahp1$_{C30S}$ and *Sc*Ahp1$_{C31S}$ was carried for crystallization purpose. 250 μM of *Ct*Ahp1$_{C30S}$and 125 μM of *Ct*Urm1$_{C55S}$-SH were mixed in 4 ml reaction buffer (20 mM Tris pH 7.5 and 200 mM NaCl) and TBH was added for the final concentration of 0.5 mM. The reaction was carried out for 30 min at 37 °C. The reaction mixtures were applied to an ion exchange column HiTrap Q FF GL column. The fractions were collected, and samples were run on SDS-Page to locate the fractions containing the *Ct*Ahp1$_{C30S}$-*Ct*Urm1$_{C55S}$ complex. The fractions containing the complex were pooled and concentrated for further purification. The concentrated sample was loaded in to HiLoad 16/600 Superdex 75 pg GL column on ÄKTA™ pure system that was equilibrated with in 20 mM Tris pH 7.5, 200 mM NaCl and 1 mM TCEP. The integrated peak areas corresponding to the complex *Ct*Ahp1$_{C30S}$-*Ct*Urm1$_{C55S}$ and individual proteins *Ct*Ahp1$_{C30S}$ and *Ct*Urm1$_{C55S}$-SH were calculated using the UNICORN 7.0 software. Subsequently, the samples from the fractions were loaded on Bolt™ 4-12 % Bis-Tris Plus Gels (Thermo Fisher Scientific). For protein visualization, the gels were stained with Coomassie *Brilliant Blue. The fractions containing the complex 30 kDa in size were collected and up concentrated to* 25 mg/ml *and* proceeded with crystallization or *snap frozen and stored at -80* °C for further use. *For urmylation of ScAhp1 $_{C31S}$and sample preparation of crystallization for the ScAhp1 $_{C31S}$-ScUrml complex, similar procedures as CtAhp1$_{C30S}$ were used.*

**Crystallization, data collection, structure determination and refinement**

For crystallization, *Ct*Ahp1 and *Ct*Ahp1$_{C30S}$ were concentrated to 25 mg ml$^{-1}$ in 20 mM Tris pH 7.5, 200 mM NaCl and 1 mM DTT. Crystals of the protein were grown at 21 °C by vapor diffusion in sitting drops composed of equal volumes (1 μl each) of protein solution and crystallization buffer (2 M Ammonium Sulfate and 0.1 M Sodium cacodylate pH 6.5). Crystals collected from reservoirs containing 2 M Ammonium Sulfate and 0.1 M Sodium cacodylate pH 6.5 were cryoprotected by serial transfer into cryoprotecting buffer (2 M Ammonium Sulfate and 0.1 M Sodium cacodylate pH 6.5 and 50 % glycerol). X-ray diffraction data for *Ct*Ahp1 at 1.75 Å resolution were recorded at ESRF beamline in Grenoble, France. X-ray diffraction data for *Ct*Ahp1$_{C30S}$ at 1.85 Å resolution were recorded at BESSY in Berlin, Germany. Crystallization of *Ct*Ahp1$_{C30S}$ post-urmylation reaction was carried by pooling the unconjugated protein from the urmylation reaction and further loading in to the HiLoad 16/600 Superdex 75 pg GL column on ÄKTA™Pure system. The fractions of *Ct*Ahp1$_{C30S}$ were pooled and concentrated to 25 mg ml$^{-1}$ 20 mM Tris pH 7.5, 200 mM NaCl and 1 mM TCEP. Crystals of the protein were grown at 21 °C by vapor diffusion in sitting drops composed of equal volumes (1 μl each) of protein solution and crystallization buffer (2 M Ammonium Sulfate and 0.1 M Sodium cacodylate pH 6.0). Crystals collected from reservoirs containing 2 M Ammonium Sulfate and 0.1 M Sodium cacodylate pH 6.0 were cryoprotected by serial transfer into cryoprotecting buffer (2 M Ammonium Sulfate and 0.1 M Sodium cacodylate pH 6.0 and 30 % glycerol). X-ray diffraction data for *Ct*Ahp1$_{C30S}$ at 1.10 Å resolution were recorded at BESSY in Berlin, Germany. Crystals of *Ct*Ahp1$_{C30S}$-*Ct*Urm1$_{C55S}$ complex were grown at 21 °C using hanging drop vapor diffusion technique. In each drop, 2 μl of protein sample was mixed with 2 μl of reservoir solution (0.05 M Zinc Acetate Dihydrate, 0.1 M Sodium Cacodylate pH 6.5, 4 % w/v polyethylene glycol 8000 and 30 % w/v ethylene glycol). The crystals appeared after 48 h and grew to maximal size in two weeks. The crystals were cryoprotected by serial transfer into cryoprotecting buffer (0.05 M Zinc Acetate Dihydrate, 0.1 M Sodium Cacodylate pH 6.5, 4 % w/v polyethylene glycol 8000 and 50 % w/v ethylene glycol) and snap frozen in liquid nitrogen. X-ray diffraction data at 2.40 Å resolution were collected at BESSY in Berlin, Germany. For data collection details see Table 1. The structures of *Ct*Ahp1, *Ct*Ahp1$_{C30S}$ and *Ct*Ahp1$_{C30S}$-*Ct*Urm1$_{C55S}$ complex were determined by molecular replacement using Phaser (McCoy et al., 2007) with *S. cerevisiae* Ahpl (4DSR) and Urm1 (2QJL) , respectively. Structures were refined using Phenix (Adams et al., 2010) and Refmac5 in CCP4 (Winn et al., 2011) programs. The comprehensive validation was done by MolProbity (Davis et al., 2007). Structural visualization was done with PyMOL (http://pymol.sourceforge.net/). For structure refinement statistics see Table 1. Anomalous density collection for Zn peak and below Zn peak data was collected for the *Ct*Ahp1$_{C30S}$-*Ct*Urm1$_{C55S}$ complex at BESSY in Berlin, Germany.

### *In vitro* Urm1 conjugation of *in vivo* targets

20 μM of target proteins such as *Hs*PRDX5, *Sc*Tdh3, *Hs*GAPDH, *Sc*Ses1, *Hs*SARS1, *Sc*Pyk1, *Hs*PKM2 and 20 μM of carboxylated or thiocarboxylated Urm 1 were mixed in reaction buffer (20 mM Tris-HCl pH 7.5 and 200 mM NaCl). Diamide at final concentration of 0.5 mM was included or excluded as indicated. The reaction mix was incubated for 30 min at 30 °C. The reactions were stopped by adding Laemmli sample buffer containing DTT and incubated for 5 min at 95 °C. Subsequently the samples were loaded on Bolt™ 4-12 % Bis-Tris Plus Gels (Thermo Fisher Scientific). For protein visualization, the gels were stained with Coomassie *Brilliant Blue*.

### Protein identification from gel bands - sample preparation and LC-MS/MS measurement

Samples were prepared and measured as described (Pabis et al., 2020) with minor changes. The flow rate during peptide separation on analytical column was 250 nl/min. Moreover, different proteolytic enzymes were used depending on what modification was to be detected. Chymotrypsin was used to find urmylation and GFP conjugation, while trypsin to identify ubiquitination. To detect sumoylation, trypsin, chymotrypsin and V8 protease were employed.

### Protein identification from solution - sample preparation and LC-MS/MS measurement

Protein samples were prepared in 20 mM Tris, 200 mM NaCl. Sample amount corresponding to about 10 μg of the protein of interest was used for digestion. Final volume of digestion solution was 60 μl. Urea was added to the concentration of 0.5 M. Digestion solution was filled up to 60 μl with 50 mM ammonium bicarbonate. Chymotrypsin was used in the enzyme to protein ratio of 1:20. Digestion solution was supplemented with $CaCl_2$ to the concentration of about 10 mM. Samples were incubated at 25 °C overnight. The next day, digestion was stopped by adding trifluoroacetic acid to a concentration of 0.5 %. Approximately 1% of digested samples were injected for LC-MS/MS analysis, which was performed the same as for samples from gel bands.

### LC-MS/MS data analysis for protein identification from gel bands and solution

The LC-MS/MS data were processed using the Proteome Discoverer platform (v. 1.4; Thermo Scientific) and searched using an in-house MASCOT server (v.2.5.1; Matrix Science, London, UK) against cRAP database (https://www.thegpm.org/crap/) supplemented with the sequences of the proteins of interest. The following modifications were included in search parameters depending on the sample: urmylation (HisGlyGly tag after chymotrypsin, Δ mass = 251.101839); ubiquitination (GlyGly tag after trypsin, Δ mass = 114.042927); sumoylation (GlyGlyIleGlnGlu tag after trypsin, Δ mass = 503.246552; GlyGlyIleGln tag after chymotrypsin or V8 protease, Δ mass = 374.203959); GFP conjugation (Lys tag after chymotrypsin; Δ mass = 128.094963); cysteine carbamidomethylation (Δ mass = 57.021464); cysteine persulfidation (Δ mass = 31.972071); carbamidomethylated cysteine persulfidation (Δ mass = 88.993534); methionine oxidation (Δ mass = 15.994915).

### Thiocarboxylation of Urm1 using L-cysteine as sulfur source

To produce free sulfide from L-Cys, 10 μM of *E. coli* desulfurase IscS was incubated at 25 °C overnight with 1 mM L-Cys and 20 μM PLP co-factor in a desulfurase buffer (20 mM Hepes pH 8.5, 150 mM NaCl, 2 mM $MgCl_2$). To examine the fate of sulfur in the yeast system, 10 μM of ScUba4 and 40 μM of *Sc*Urm1 were added, with 2.5 mM ATP and 1 mM TCEP, and incubated for 1h at 30 °C. Analogously, for *Ct*., 10 μM of *Ct*Uba4 and 40 μM of *Ct*Urm1$_{C55S}$ were added with 2.5 mM ATP and 1mM TCEP, and incubated for 1 h at 37 °C. Following incubation, the samples were desalted using PD SpinTrapTM G-25 (Cytiva) columns, buffer exchanged into 40 mM ammonium acetate and analyzed by ESI-MS as previously described.

**Radioactive sulfur transfer assay**

To generate thiocarboxylated Urm1 labeled with $^{35}S$, we purchased $^{35}S$-containing L-cysteine (Perkin Elmer, NEG022T001MC) and used it as a sulfur source that simulates one of the plausible sulfur sources *in vivo*. Approximately 0.1 mCi of $^{35}S$ L-cysteine was desulfurated using a 20 μM recombinant IscS desulfurase from *E. coli* for 1 hour at the room temperature in a desulfuration buffer containing 20 mM HEPES pH 8.5, 150 mM NaCl, 2 mM $MgCl_2$, and 80 μM pyridoxal phosphate (PLP) necessary for IscS activity. Next, 10 μM Uba4 and 70 μM Urm1 proteins together with 2 mM ATP and 1 mM TCEP were added to the tube containing desulfurated cysteine. The Urm1 thiocarboxylation reaction was executed for 1 hour at 30 °C. To ensure that most of the $^{35}S$ atoms were transferred to Urm1, 180 μM of sodium thiosulfate was added to the reaction mix and incubated for 15 minutes. Samples were desalted using Amersham WB MiniTrap kit (Cytiva) following the manufacturer's protocol to get rid of free cysteine, $^{35}S$, and reducing agent. Fresh $^{35}S$-thiocarboxylated Urm1 was used for urmylation reactions in combination with 20 μM corresponding target proteins in presence of 0.5 mM oxidizing agents (TBH or Diamide). The urmylation reaction was performed at 30 °C for 1 h after which samples were denatured at 95 °C in sample buffer without any reducing agents. Proteins were separated using SDS-PAGE, gels were stained with Coomassie and dried for 2 h in the Gel Dryer Model 583 (BioRad). Dry gels were exposed overnight to storage phosphor screens (Cytiva) and the accumulated signal was visualized using the Personal Molecular Imager system (BioRad). All assays shown herein were repeated at least three times.

**Malachite green ATPase assay**

To examine the activatory potential of different UBLs on ATPase activity of Uba4, we used a commercially available Malachite Green Kit (Sigma-Aldrich). Assembled reactions contained 40 μM Uba4 and 60 μM of the UBL of interest, resuspended in 100 mM MES pH 6.0, 100 mM NaCl, 2 mM $MgCl_2$ and 160 μM ATP. Addition of 5 mU/reaction of inorganic pyrophosphatase (Thermo Fischer Scientific) allowed to convert all inorganic pyrophosphate crated by Uba4 to phosphate molecules that can be quantified using Malachite Green. The reaction took place for 90 min at 37 °C. The reaction product was diluted 20x in water and developed according to the manufacturer's instructions. Absorbance was measured at 620 nm using SpectraMax 190 Microplate Reader (Molecular Devices). Data were acquired in three independent experiments with two technical replicates each.

**Thiocarboxylation of GFP-ScUrml**

ScUba4-mediated thiocarboxylation of GFP-*Sc*Urm1 was done as described for *Sc*Urm1. Briefly, 20 μM of GFP-*Sc*Urm1 was mixed with 10 μM of ScUba4 in the thiocarboxylation buffer (20 mM Hepes pH 8.5, 150 mM NaCl, 2 mM $MgCl_2$), supplemented with 5 mM ATP and 5 mM TCEP and incubated for 1 h at 30 °C. The GFP-ScUrm1-SH sample was loaded on Superdex 200 Increase 10/300 GL (Cytiva) to isolate GFP-*Sc*Urm1-SH.

**Conjugation reaction of GFP-ScUrm1-SH and target proteins**

Conjugation reactions between 20 μM of GFP-*Sc*Urm1-SH and 20 μM of target proteins *Sc*Ahp1, *Sc*Pyk1 and *Sc*Ses1 were done in the presence of 0.5 mM diamide for 30 min at 30 °C. Carboxylated GFP-*Sc*Urm1-OH was used as negative control. The protein samples were denatured at 95 °C in the presence of Laemmli sample buffer and analysed on Bolt™ 4-12 % Bis-Tris Plus Gels (Thermo Fisher Scientific). For protein visualization, the gels were stained with Coomassie *Brilliant Blue.*

**Generation of yeast strains and plasmid constructions**

*S. cerevisiae* strains used and generated in this study (Table S4) were grown on complete (YPD) or synthetic (SC) media (Sherman, 1991) at 30°C unless otherwise indicated. 80 μg/ml L-cysteine hydrochloride was supplemented for growth of auxotrophic strains when required. Yeast gene deletions were generated by PCR using the pUG plasmid system (Gueldener et al., 2002) and gene specific oligonucleotides (Table S5). Correct gene replacements were confirmed by PCR using primer pairs located outside of the target loci (Table S5). For the construction of yeast expression vectors (Gietz and Akio, 1988) carrying Ahpl lysine to arginine mutants, we used the FastCloning technique (Li et al., 2011). In brief, the mutated *AHP1* gene sequence was amplified from the corresponding pETM-30 vector (Table S3) using primer pairs (Table S5) adding overlapping ends homologous to the linearized pAJ31 target plasmid. All mutations were verified by Sanger-based DNA sequencing. The yeast expression plasmid pHA-URM1 (Table S6) was used as a template to N-terminally insert a polyhistidine affinity tag (8xHis) by PCR-based site-directed mutagenesis (Wang and Malcolm, 1999) with the appropriate oligonucleotide primers (Table S6) resulting in pLK20 (Table S6) that was used for *in vivo* urmylation assays. Transformation of yeast cells with PCR products or plasmids (Table S6) was performed as previously published (Gietz and Woods, 2002).

### *In vivo* urmylation

*In vivo* urmylation studies were performed as previously described (Jüdes et al., 2015). In brief, yeast cells grown to an $OD_{600nm}$ of 1.0 were harvested and lysed mechanically with glass beads in a buffer (10 mM K-HEPES pH 7.0, 10 mM KCl, 1.5 mM MgCl2, 0.5 mM PMSF and 2 mM benzamidine) containing complete protease inhibitors (Roche) and 2.5 mM N-ethylmaleimide (NEM). Protein concentrations were determined according to Bradford assay (Bradford, 1976) and lysates were mixed with SDS sample buffer (62.5 mM Tris-HCl pH 6.8, 2% SDS, 10% glycerol, 0.002% bromophenol blue and 5% β-mercaptoethanol) according to Laemmli (Laemmli, 1970). For Western blot analyses, proteins were transferred to PVDF membranes and incubated with primary anti-HA antibodies (F7, Santa Cruz Biotechnology or 2-2.214, Invitrogen). Unconjugated Ahpl was detected using anti-Ahpl serum (Iwai et al., 2010) kindly provided by Dr Kuge (Tohoku Pharmaceutical University, Japan). Equal protein loading was verified with anti-Cdc19 antibodies donated by Dr Thorner (University of California-Berkeley, USA). Detection of target proteins involved horseradish peroxidase-conjugated secondary goat anti-mouse or anti-rabbit IgGs (Jackson ImmunoResearch) and the WesternBright ECL Spray (Advansta Inc.) according to manufacturers' instructions. For visualization of target proteins, the Odyssey® Fc Imaging System (LI-COR, Inc.) was used.

### Phenotypical analyses and TBH toxicity assay

Overnight yeast cultures were diluted to an $OD_{600nm}$ of 1.0. Ten-fold serial dilutions were prepared ranging from $10^{-1}$ to $10^{-3}$ and transferred to YPD medium using a pin-tool. For toxicity assays, the medium was supplemented with the organic peroxide TBH as indicated. Plates were incubated at 30°C unless otherwise stated and monitored after 36-48 h.

### RNA isolation and Northern blot analysis

Total tRNA was extracted from yeast as previously described (Krutyholowa et al., 2019), using NucleoZOL reagent according to the manufacturers' instructions. The pelleted tRNA was washed once with 75% ethanol and stored in 100% ethanol for subsequent northern blot analysis. 0.4 μg of total tRNA was separated by electrophoresis on 12 % denaturing polyacrylamide gels (7 M Urea, 0.5x TBE buffer), visualized by SYBR Gold (Invitrogen) and transferred to a nylon membrane (Immobilon-Ny+) at 400 mA for 45 min using a Trans-Blot® SD Semi-Dry Transfer Cell (Bio-Rad). To analyze the tRNA thiolation levels, the gels were supplemented with ([N-Acryloyl-amino]phenyl)mercuric chloride (APM) at a final concentration of 60 μg/ml (APM stock solution: 3 μg/μl in formamide). For APM+ gels the transfer was performed for 1 h and the transfer buffer was supplemented with 10 mM DTT to improve the transfer of thiolated tRNA. Membranes were hybridized at 42 °C to a 32P-5'-end-labelled DNA probe 5'-tggctccgatacggggagtcgaac-3', which is complementary to a 3' part of $tRNA^{Glu}_{UUC}$. Labelling of the probe with [γ-32P]-ATP, hybridization and subsequent steps of Northern blotting procedure were performed as described in (Leidel et al., 2009). The quantitative analysis was performed using a GelAnalyzer system. Thiolation levels (%) were calculated for 3 biological replicates. Aliquots from spotting experiment were thawed on ice and total RNA was extracted by using hot phenol/chloroform extraction. 1 μg of total RNA was resolved on an 8 % PAGE containing 0.5 x TBE, 7 M Urea and 50 μg/ml APM (Igloi, 1988). Northern blot analysis was performed as described previously by using the probe against $tRNA^{Glu}_{UUC}$ (5'- tggctccgatacggggagtcgaac -3') (Leidel et al., 2009).

### Protein isolation and Western blot analysis

Aliquots (3 $OD_{600}$ units) from spotting experiments were thawed on ice and total proteins were extracted as previously described (von der Haar, 2007). Total protein extracts were resolved by SDS-PAGE and transferred by semi-dry blotting onto a PVDF-membrane. Membranes were probed using a monoclonal anti-HA antibody (Covance MMS-101R).

### Qualitative identification of $H_2S$ production

The yeast strain used in this experiment was BY4742. The wild-type strain together with the single knockout strains *ahp1Δ, urm1Δ, tum1Δ, ncs6Δ, cys3Δ, cys4Δ* and the double knockout deletion strains *cys3Δurm1Δ* and *cys4Δurm1Δ* were maintained and grown on yeast extract-peptone-dextrose medium (YPD). For the Bismuth Glucose Glycine Yeast agar (BiGGY) screening, strains were grown overnight in YPD medium and diluted to an OD600nm= 0.8 and OD600nm= 0.4. Four microliters of each strain were spotted on BiGGY agar plates and incubated for 72 hours at 27 °C. The production of $H_2S$ was evaluated using a color scale dependent on the production of sulfide, the more precipitation of bismuth sulfide, the darker the colony. Taking this into consideration, the scale comprises the following colors: white, cream, light brown, and dark brown. (Cirigliano et al., 2016; Mezzetti et al., 2014)

## Claims

1. Method for transferring a sulfur moiety from a sulfur carrier protein to a cysteine residue of a target protein, comprising the steps:

a) providing

- a sulfur carrier protein comprising a thiocarboxylated C-terminus,
- a target protein comprising at least one oxidizable cysteine residue,

b) contacting the sulfur carrier protein with the target protein under conditions allowing the transfer of the sulfur moiety from the sulfur carrier protein to the target protein to obtain a thiolated target protein.

**2.** Method according to claim 1, wherein the method is an *in vitro* method or an *in vivo* method, preferably an *in vitro* method.

**3.** Method according to claim 1 or 2, wherein the conditions allowing the transfer of the sulfur moiety from the sulfur carrier protein are mild oxidative stress conditions.

**4.** Method according to any one of the preceding claims, wherein in step b) the sulfur carrier protein and the target protein are covalently conjugated.

**5.** Method according to any one of the preceding claims, wherein the method further comprises the step
c) changing the conditions to stop the transfer of the sulfur moiety from the sulfur carrier protein to the target protein.

**6.** Method according to claim 5, wherein the method further comprises the step d) isolating the thiolated target protein.

**7.** Method according to any one of the preceding claims, wherein the sulfur carrier protein is a non-canonical ubiquitin-like protein (UBL).

**8.** Method according to any one of the preceding claims, wherein the sulfur carrier protein is Urm1.

**9.** Method according to any one of the preceding claims, wherein the method does not involve E2 ubiquitin-conjugating enzyme and/or E3 ligase.

**10.** Method according to any one of the preceding claims, wherein the oxidizable cysteine residue is on the surface of the protein.

**11.** Method according to any one of the preceding claims, wherein the oxidizable cysteine residue is in proximity to a lysine, threonine and/or serine in the target protein.

**12.** Method according to any one of the preceding claims, wherein the oxidizable cysteine is in distance of 15 Å to a lysine, threonine and/or serine in the target protein.

**13.** Method according to any one of the preceding claims, wherein the target protein is a target of Urm1.

**14.** Method according to any one of the preceding claims, wherein said target protein is selected from the group consisting of *Hs*PRDX5, ScTdh3, *Hs*GAPDH, *Sc*Ses1, *Hs*SARS1, *Sc*Pyk1 and *Hs*PKM2.

**15.** Method of protecting a protein comprising a least oxidizable cysteine against oxidation of said cysteine comprising the steps as defined in claims 1 to 14.

**16.** Method for protein conjugation comprising the steps:

a) providing

- a sulfur carrier protein comprising a thiocarboxylated C-terminus
- a target protein comprising at least one oxidizable cysteine residue

b) contacting the sulfur carrier protein with the target protein under conditions allowing the conjugation of the sulfur carrier protein and the target protein to obtain a conjugated target protein.

**17.** Method according to any one of the preceding claims, wherein in step b) the sulfur moiety from the sulfur carrier protein is transferred to the target protein to obtain a thiolated target protein.

**18.** Method according to claim 17, wherein the method further comprises the step c) changing the conditions to stop the conjugation of the sulfur carrier protein and the target protein.

**19.** Method according to claim 17 or 18, wherein the method further comprises the step d) isolating the conjugated target protein.

**20.** Method according to any one of the preceding claims, wherein the sulfur carrier protein is a detectable marker.

**21.** Method according to claim 20, wherein the detectable marker is a fluorescent protein.

**22.** Method according to claim 21, wherein the fluorescent protein is GFP.

**23.** Method according to any one of the proceeding claims, wherein in step b) target protein and sulfur carrier protein are mixed, preferably in equimolar amounts, in reaction buffer comprising 20 mM Tris with pH 7.5 and 200 mM NaCl.

**24.** Method according to any one of claims 5 to 15 and 18 to 23, wherein step c) comprises adding Laemmli sample buffer containing DTT.

**25.** Method according to any one of claims 6 to 15 and 19 to 24, wherein step d) comprises a chromatography, preferably gel electrophoresis under denaturing conditions.

**26.** Method according to any one of the preceding claims, wherein step b)

- providing a sulfur carrier protein comprising a thiocarboxylated C-terminus comprises thiocarboxylation of the sulfur carrier protein.

**27.** Use of a sulfur carrier protein comprising a thiocarboxylated C-terminus for conjugating the sulfur carrier protein to a target protein comprising at least one oxidizable cysteine residue.

**28.** Use of a sulfur carrier protein comprising a thiocarboxylated C-terminus for transferring a sulfur moiety to a target protein comprising at least one oxidizable cysteine residue.

**29.** Fusion protein comprising a sulfur carrier protein covalently bound to a target protein, wherein the target protein comprises a sulfenylated cysteine in proximity to the covalent bond conjugating the sulfur carrier protein and the target protein.

**30.** Kit comprising

- a sulfur carrier protein comprising a thiocarboxylated C-terminus
- oxidizing reagent.

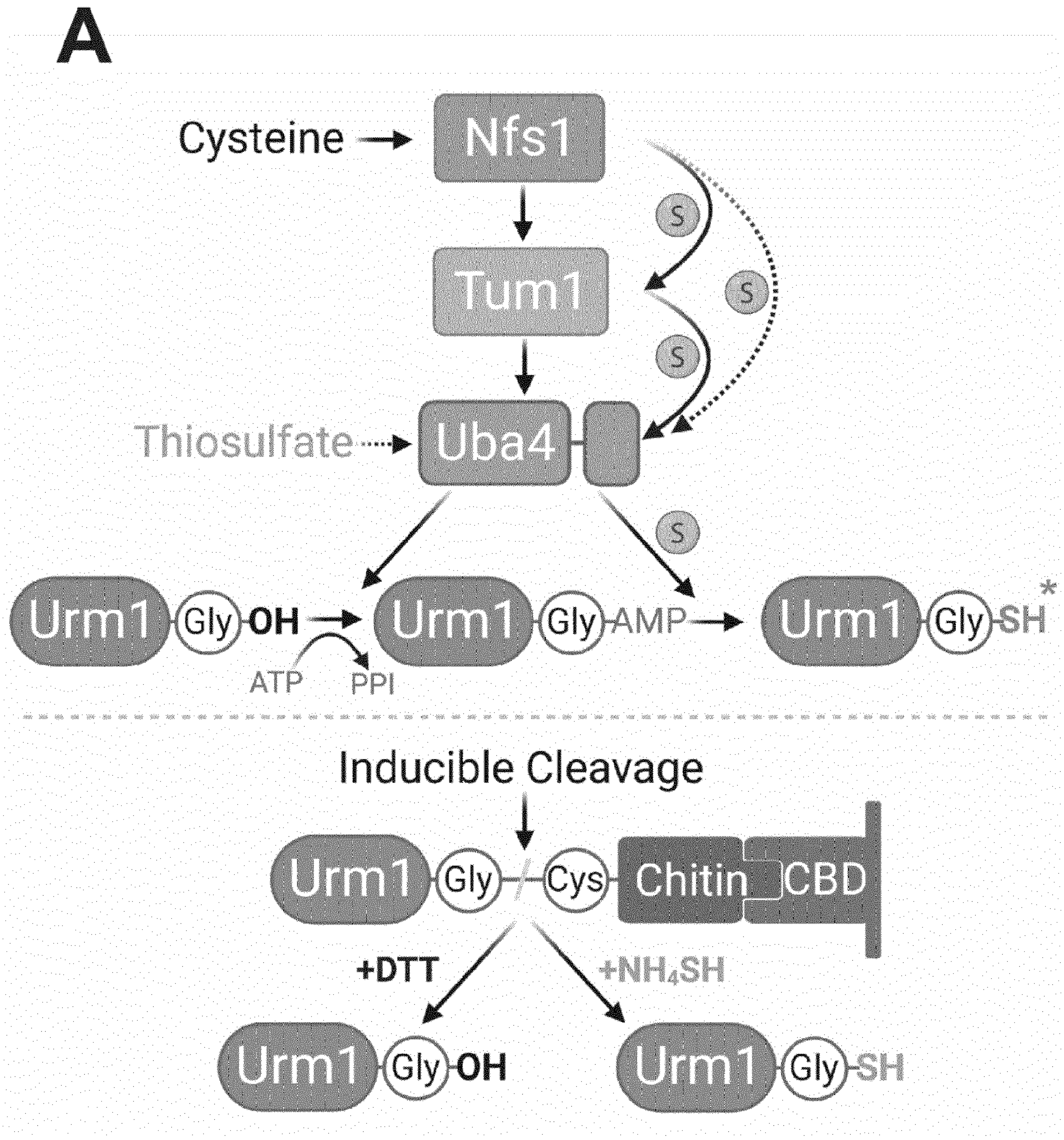
A
Cysteine
Nfs1
Tum1
Thiosulfate
Uba4
S
S
S
S
Urm1
Gly
OH
ATP
PPI
Urm1
Gly
AMP
Urm1
Gly
SH
*
Inducible Cleavage
Urm1
Gly
Cys
Chitin
CBD
+DTT
+NH4SH
Urm1
Gly
OH
Urm1
Gly
SH

Fig. 1 A

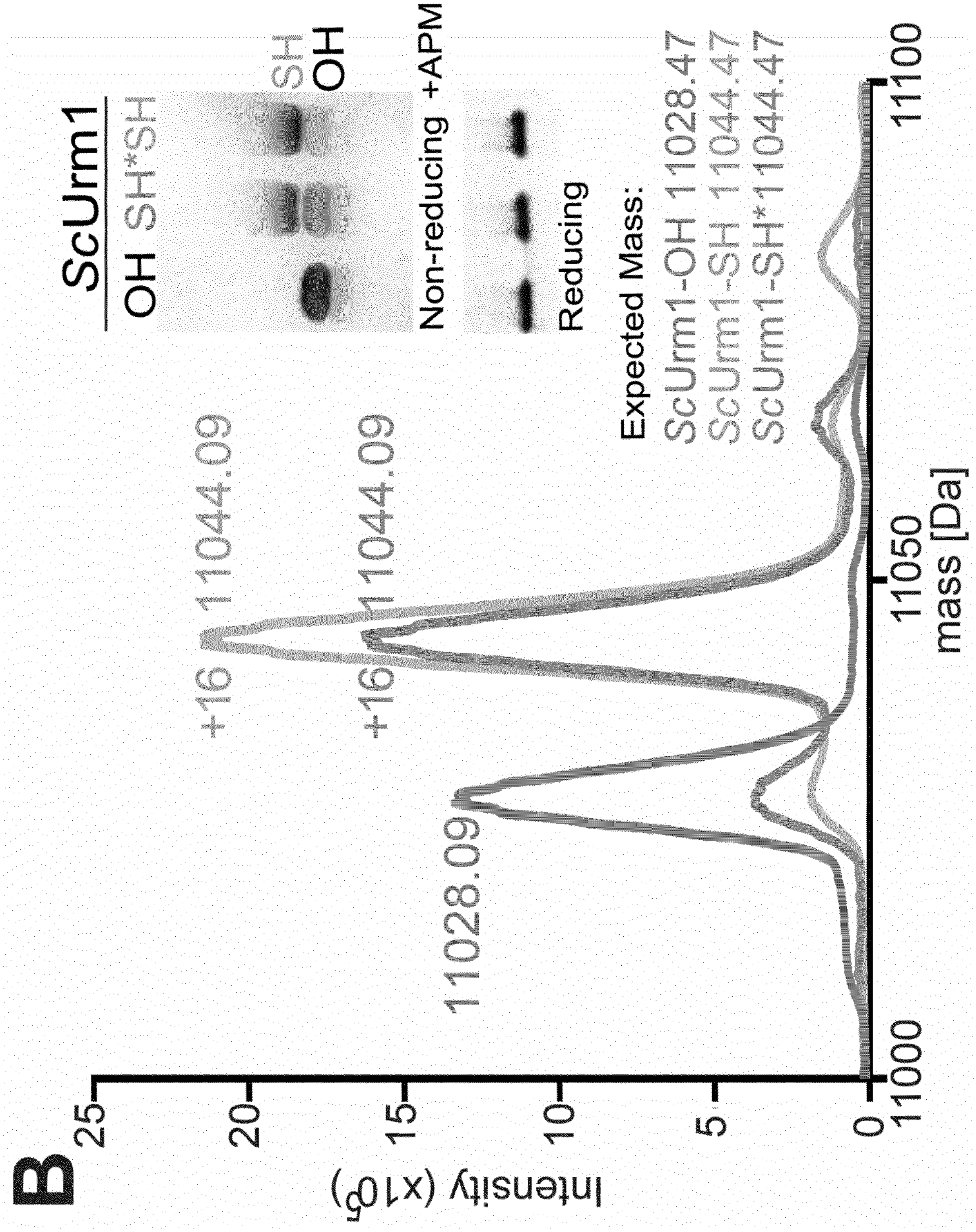
B
ScUrm1
OH SH SH*SH
SH
OH
Non-reducing +APM
Reducing
Expected Mass:
ScUrm1-OH 11028.47
ScUrm1-SH 11044.47
ScUrm1-SH*11044.47
11044.09
+16
11044.09
+16
11028.09
Intensity (x10^5)
25
20
15
10
5
0
11000
11050
11100
mass [Da]

Fig. 1 B

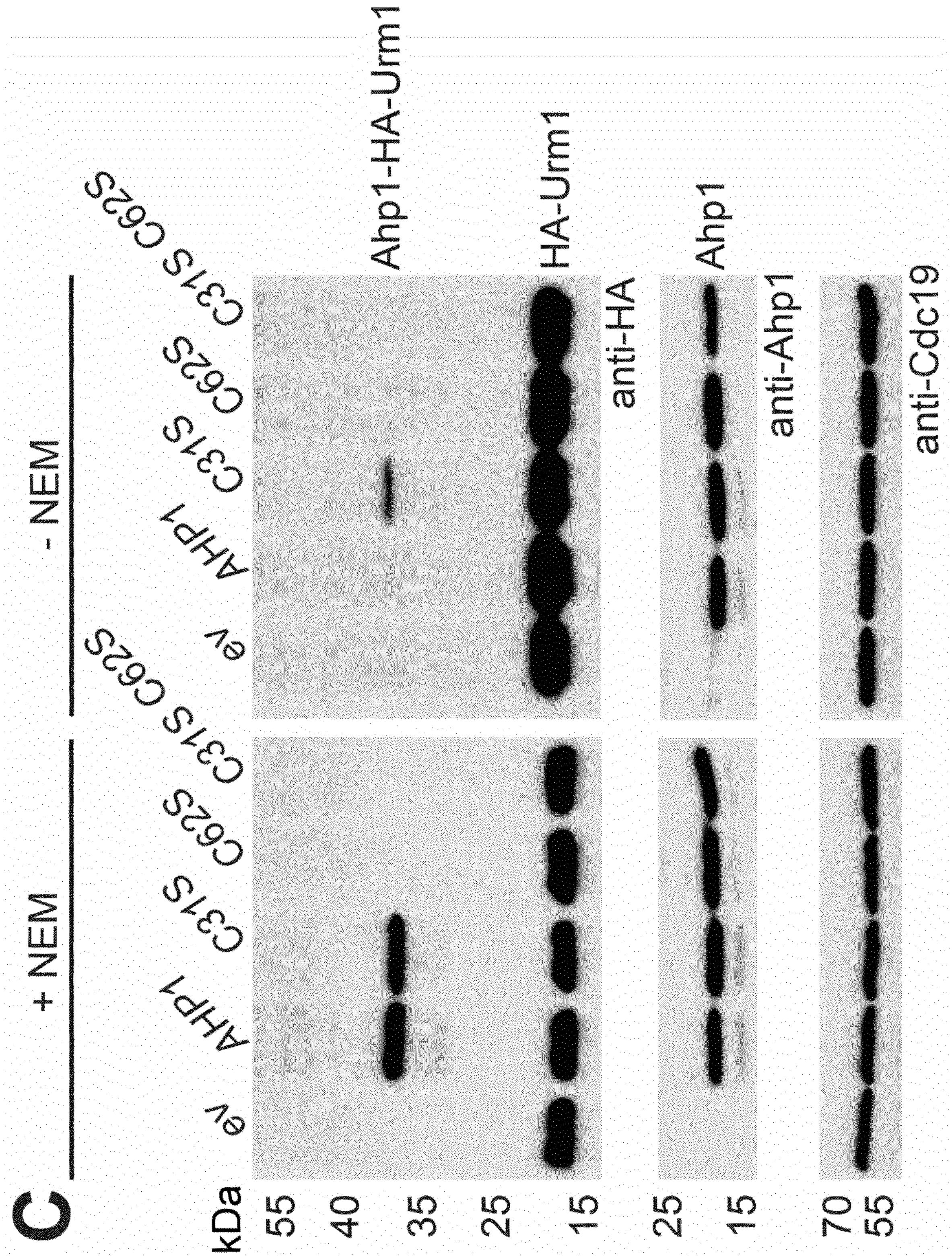
C
+ NEM
- NEM
ev
AHP1
C31S
C62S
C31S C62S
kDa
55
40
35
25
15
Ahp1-HA-Urm1
HA-Urm1
anti-HA
Ahp1
anti-Ahp1
25
15
anti-Cdc19
70
55

**Fig. 1 C**

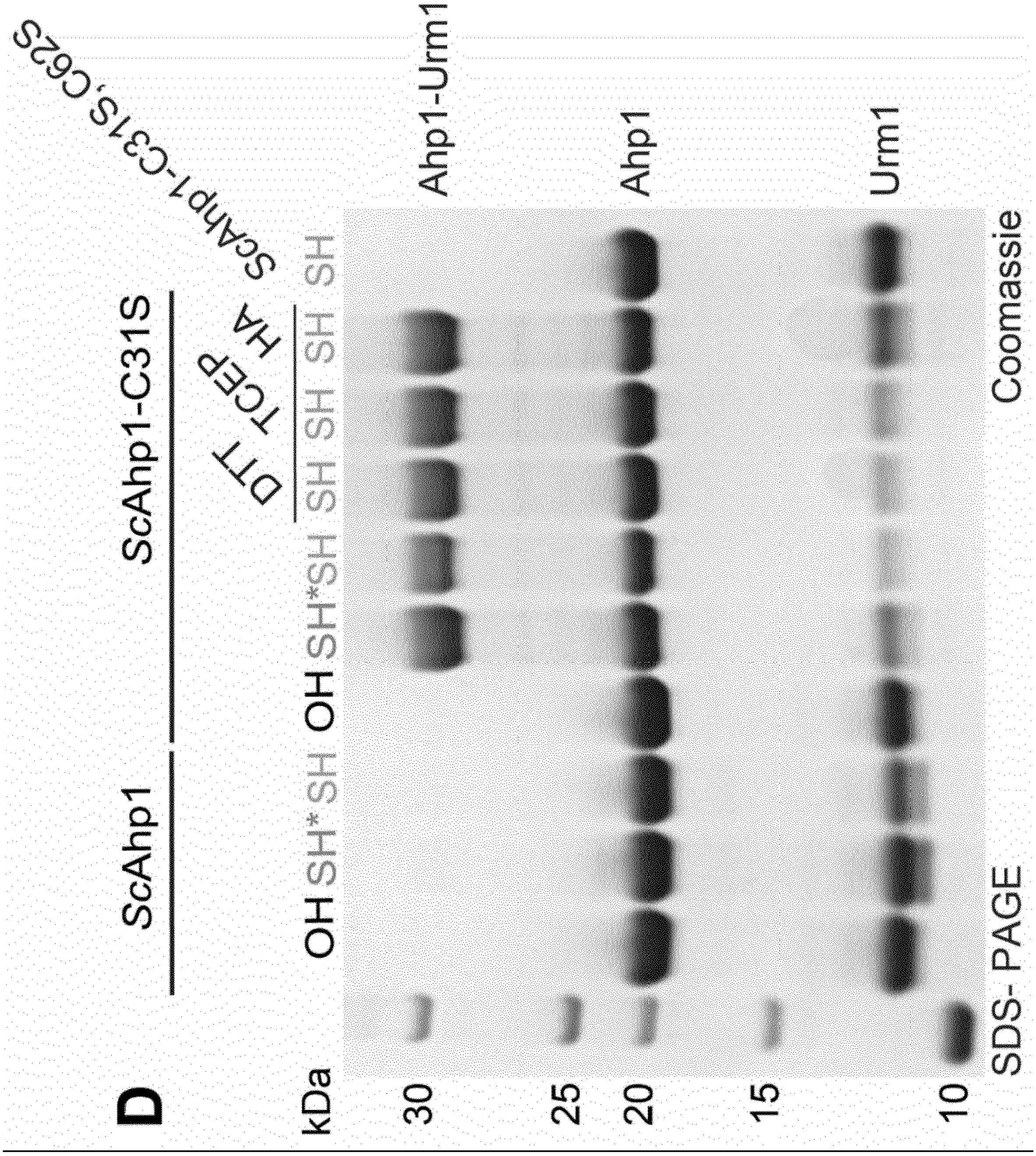
D
kDa
30
25
20
15
10
ScAhp1
ScAhp1-C31S
DTT
TCEP
HA
ScAhp1-C31S,C62S
OH SH* SH OH SH* SH SH SH SH SH
Ahp1-Urm1
Ahp1
Urm1
SDS- PAGE
Coomassie

Fig. 1 D

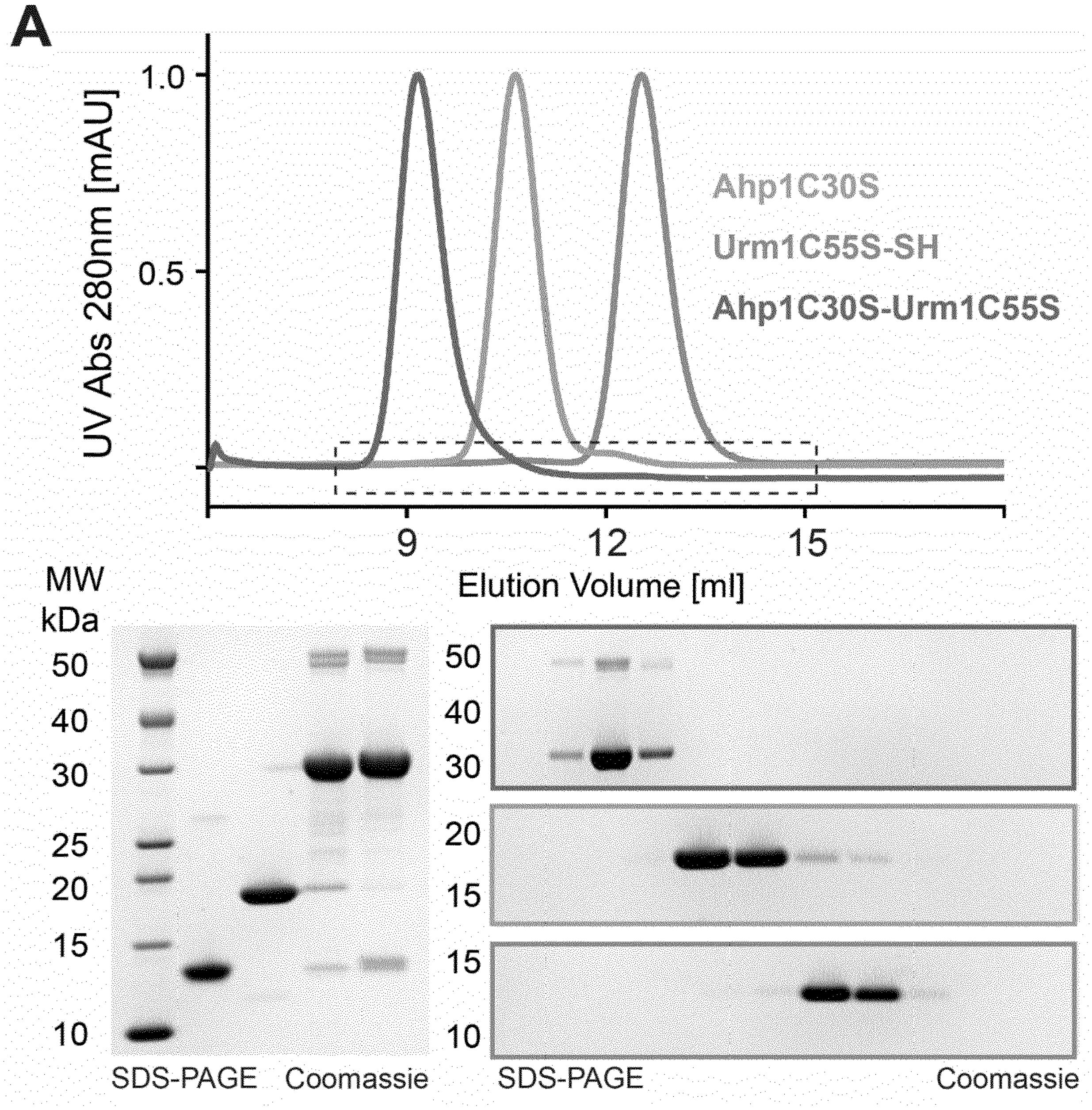
A
1.0
0.5
UV Abs 280nm [mAU]
Ahp1C30S
Urm1C55S-SH
Ahp1C30S-Urm1C55S
9
12
15
Elution Volume [ml]
MW
kDa
50
40
30
25
20
15
10
SDS-PAGE
Coomassie
50
40
30
20
15
15
10
SDS-PAGE
Coomassie

Fig. 2 A

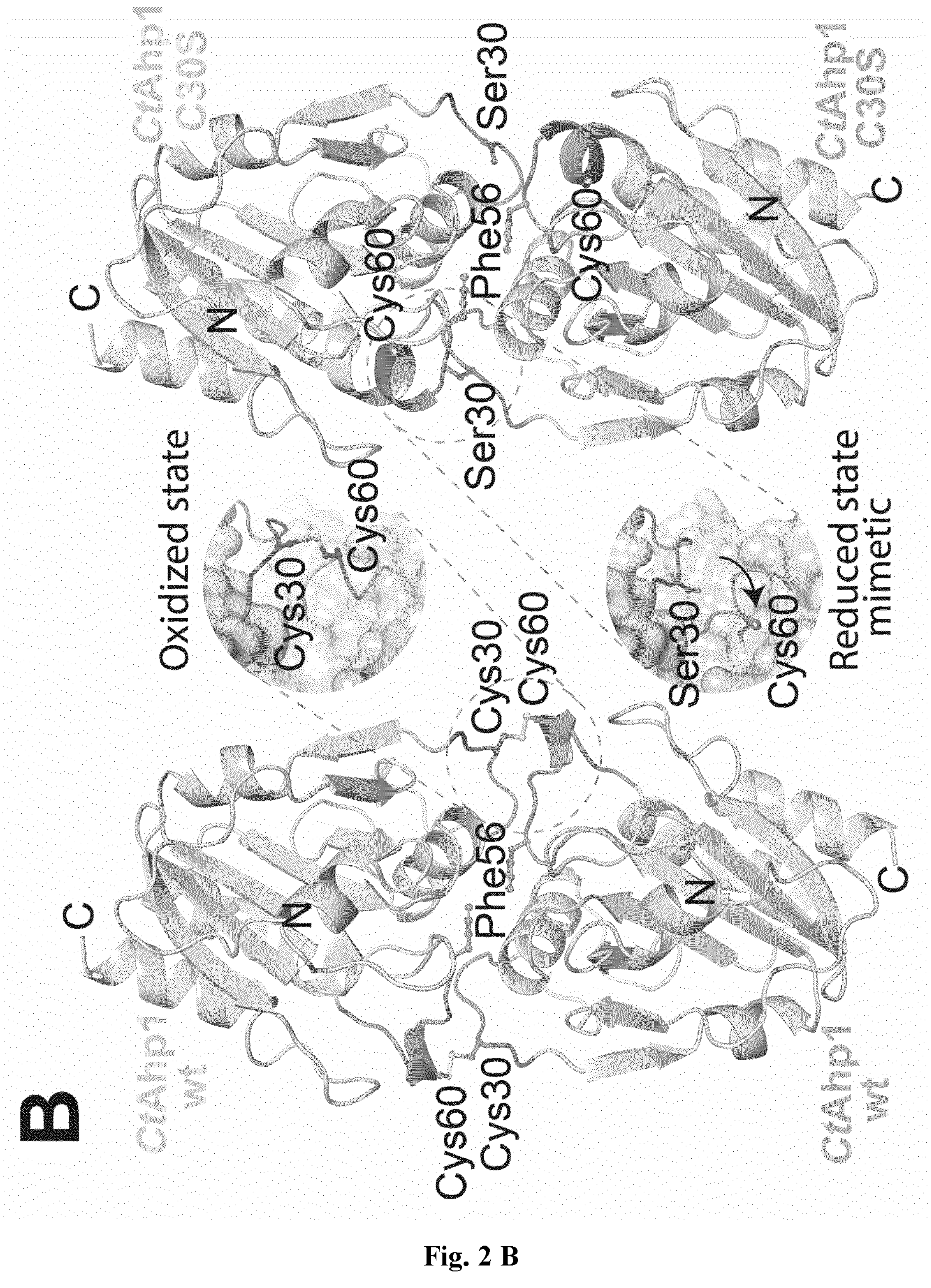
B
CtAhp1 wt
CtAhp1 C30S
Oxidized state
Reduced state mimetic
Cys30
Cys60
Ser30
Phe56
N
C

**Fig. 2 B**

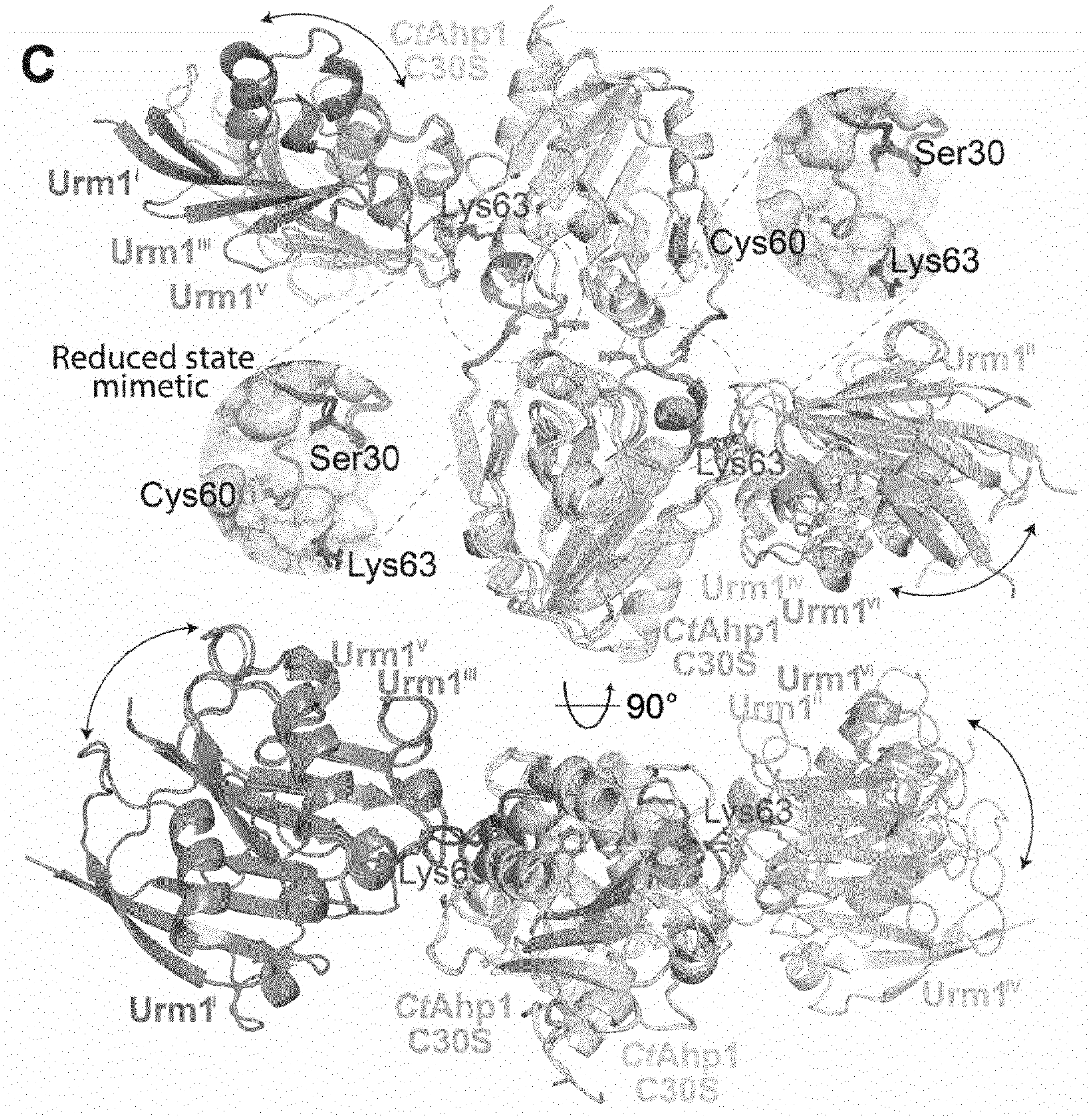
C
CtAhp1 C30S
Urm1I
Urm1III
Urm1V
Lys63
Cys60
Ser30
Lys63
Urm1II
Reduced state mimetic
Ser30
Cys60
Lys63
Lys63
Urm1IV
Urm1VI
CtAhp1 C30S
Urm1V
Urm1III
90°
Urm1VI
Urm1II
Lys63
Lys63
Urm1I
CtAhp1 C30S
CtAhp1 C30S
Urm1IV

Fig. 2 C

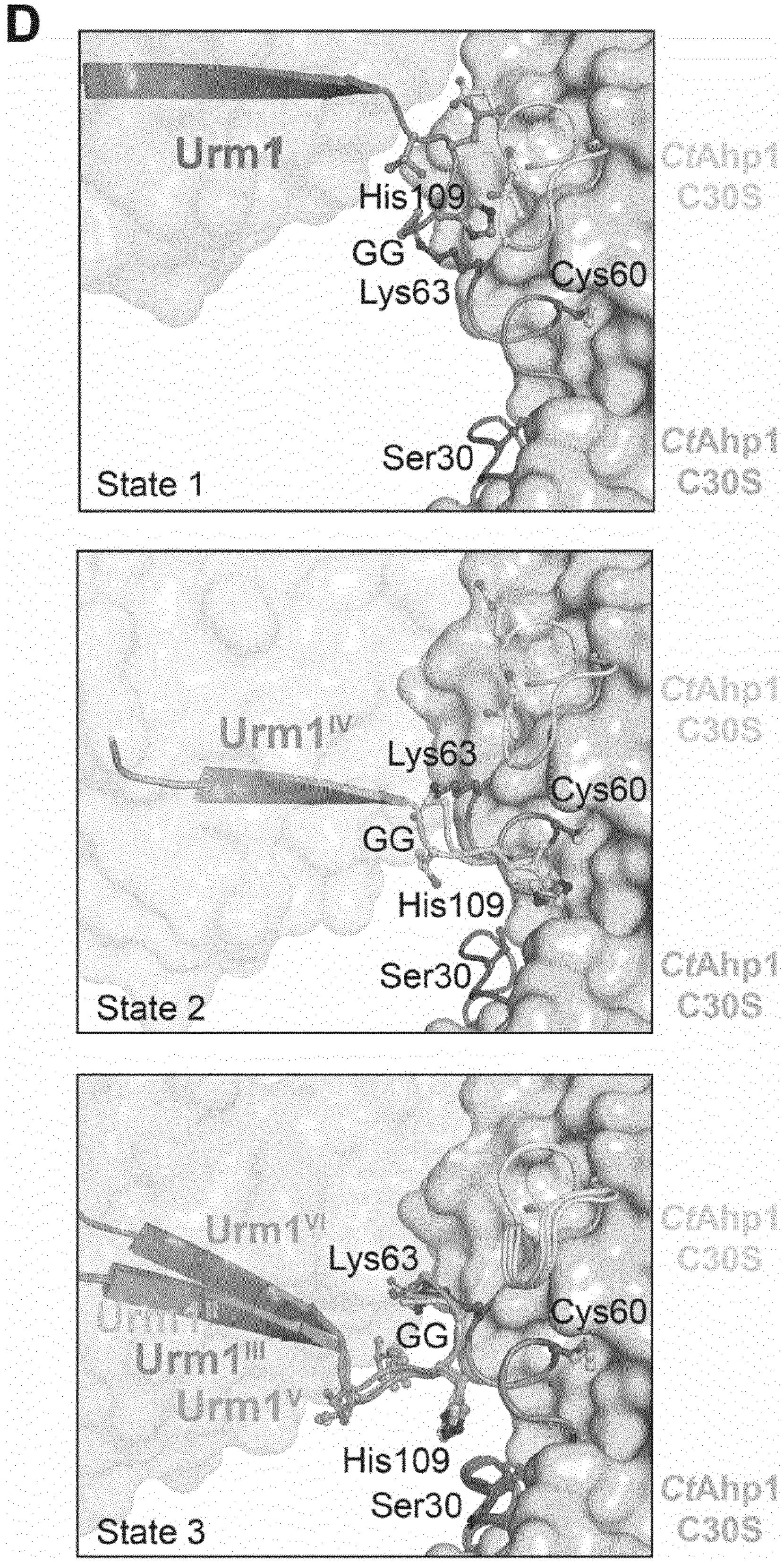
D
Urm1
His109
GG
Lys63
Cys60
Ser30
State 1
CtAhp1
C30S
CtAhp1
C30S
Urm1IV
Lys63
Cys60
GG
His109
Ser30
State 2
CtAhp1
C30S
CtAhp1
C30S
Urm1VI
Lys63
Urm1II
Urm1III
Urm1V
GG
Cys60
His109
Ser30
State 3
CtAhp1
C30S
CtAhp1
C30S

Fig. 2 D

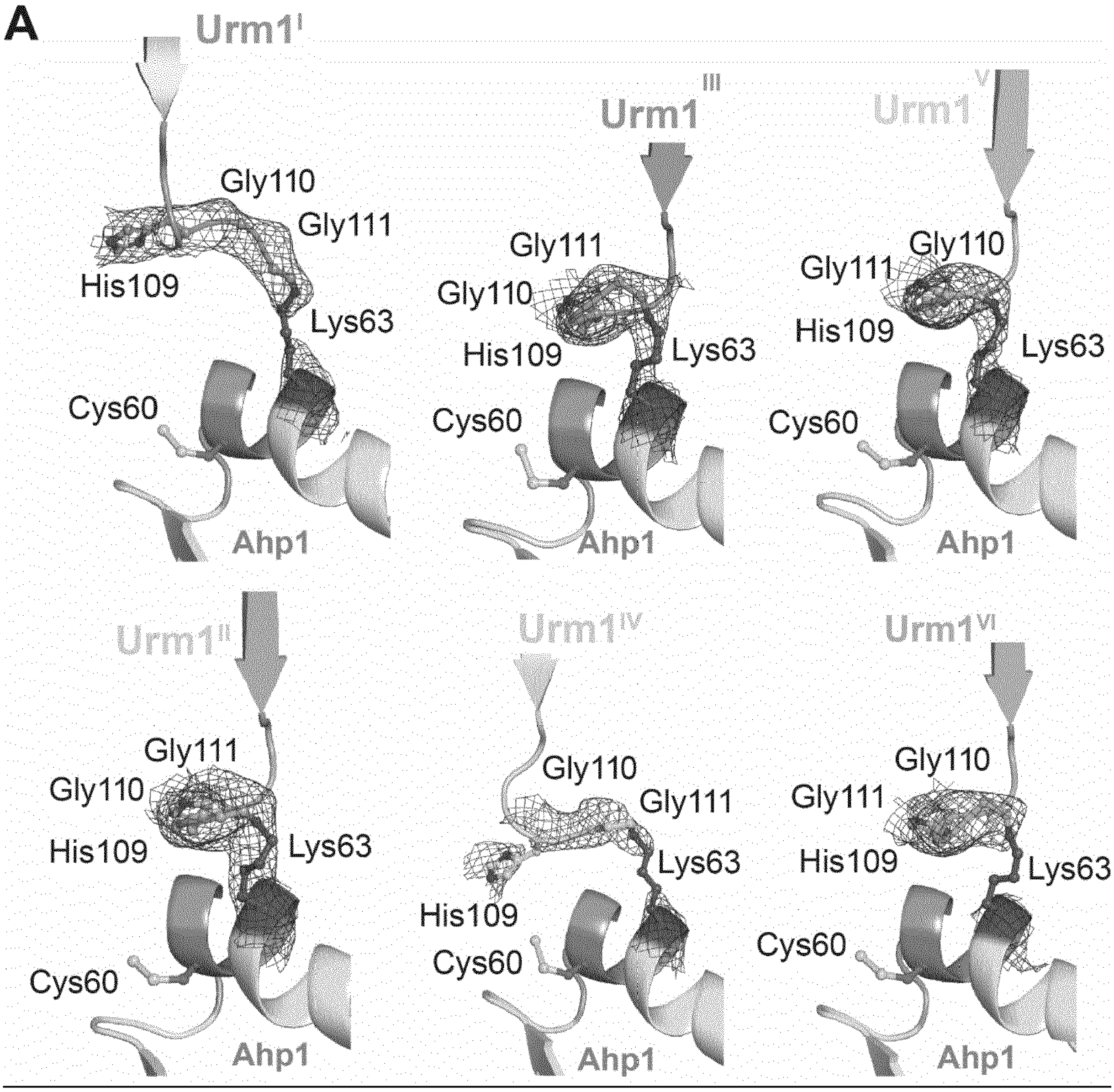
A
Urm1I
Urm1III
Urm1V
Gly110
Gly111
His109
Lys63
Cys60
Ahp1
Urm1II
Urm1IV
Urm1VI

Fig. 3 A

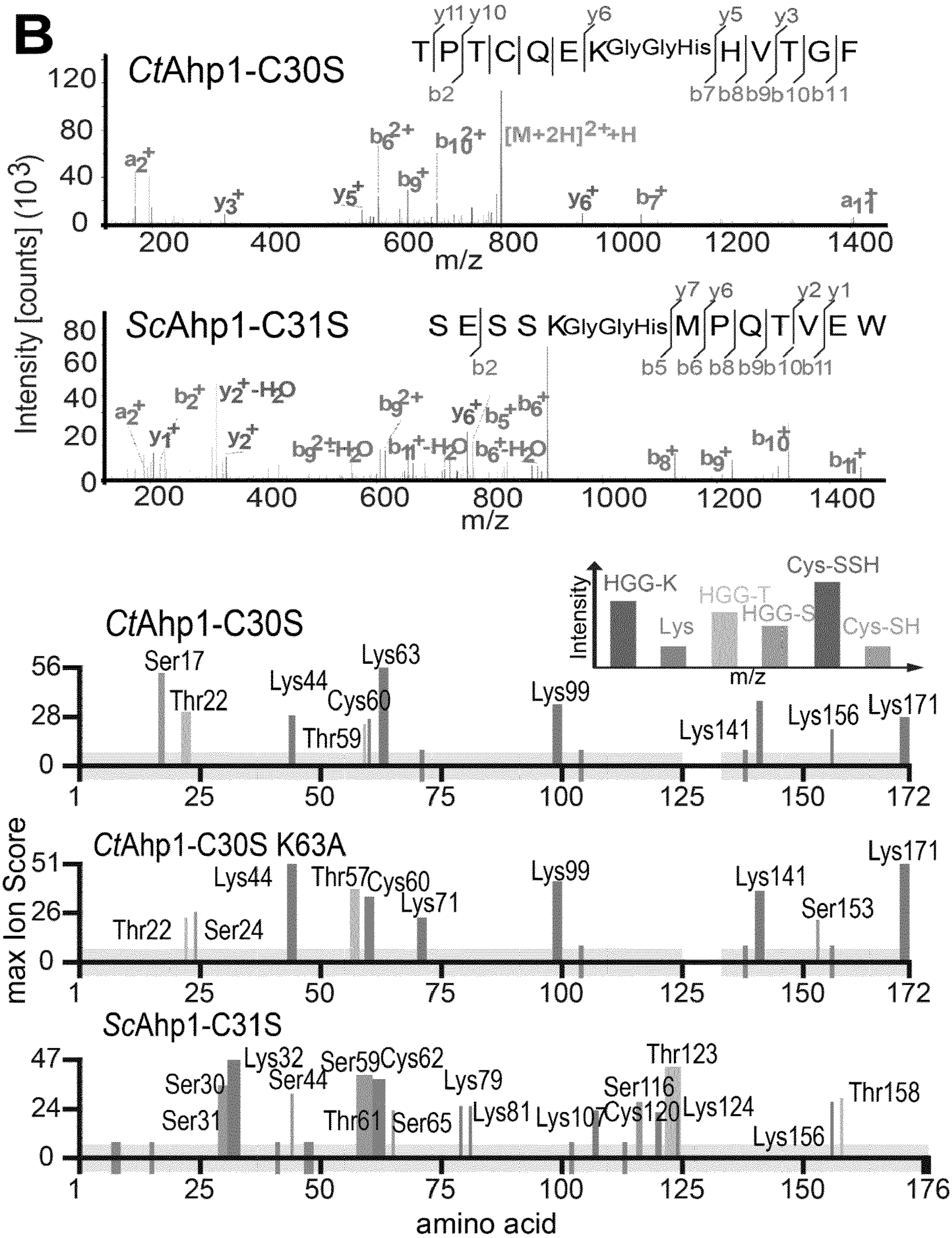
B
CtAhp1-C30S
T P T C Q E K GlyGlyHis H V T G F
Intensity [counts] (10^3)
m/z
ScAhp1-C31S
S E S S K GlyGlyHis M P Q T V E W
CtAhp1-C30S
CtAhp1-C30S K63A
ScAhp1-C31S
max Ion Score
amino acid
HGG-K
Lys
HGG-T
HGG-S
Cys-SSH
Cys-SH
Intensity

Fig. 3 B

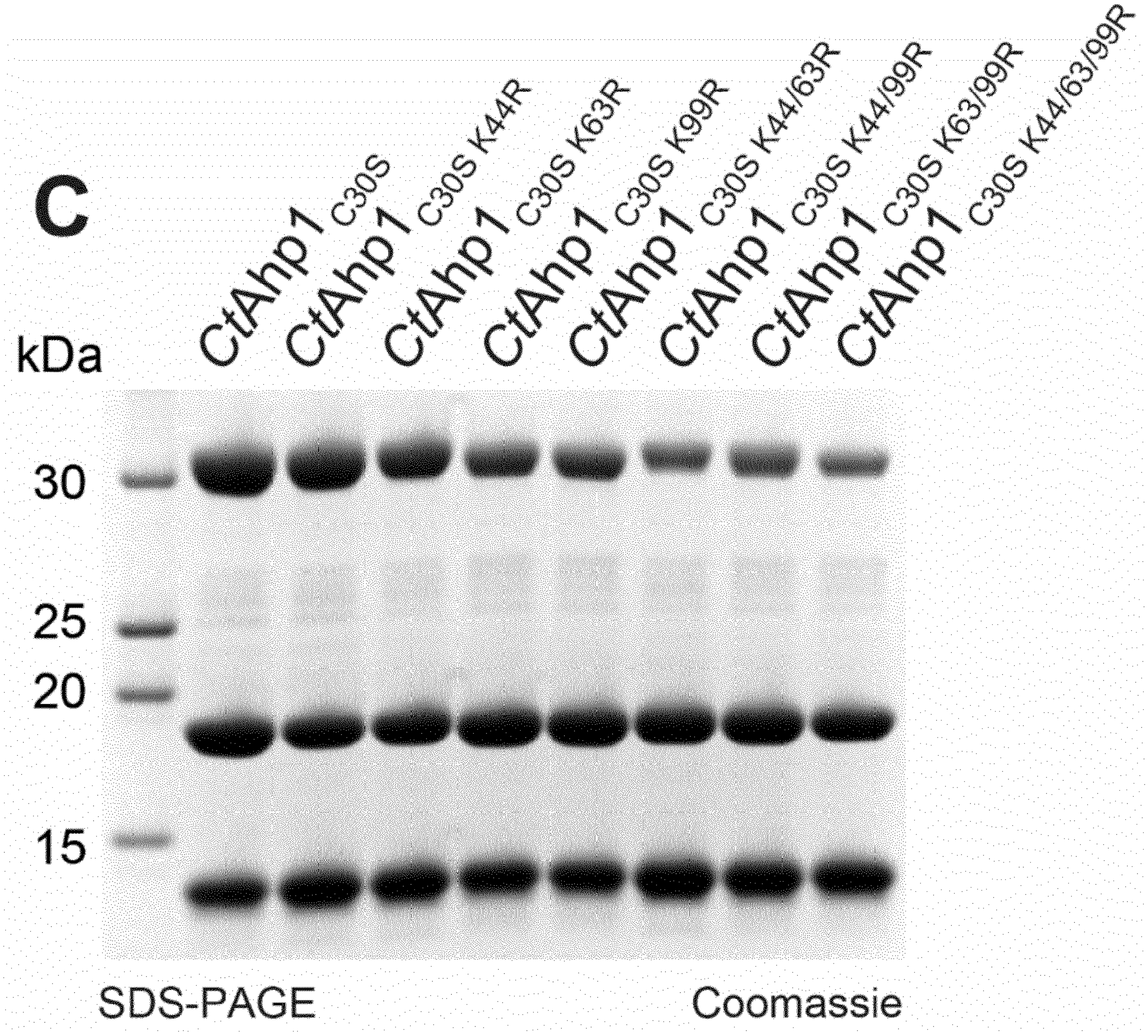
C
kDa
CtAhp1 C30S
CtAhp1 C30S K44R
CtAhp1 C30S K63R
CtAhp1 C30S K99R
CtAhp1 C30S K44/63R
CtAhp1 C30S K44/99R
CtAhp1 C30S K63/99R
CtAhp1 C30S K44/63/99R
30
25
20
15
SDS-PAGE
Coomassie

Fig. 3 C

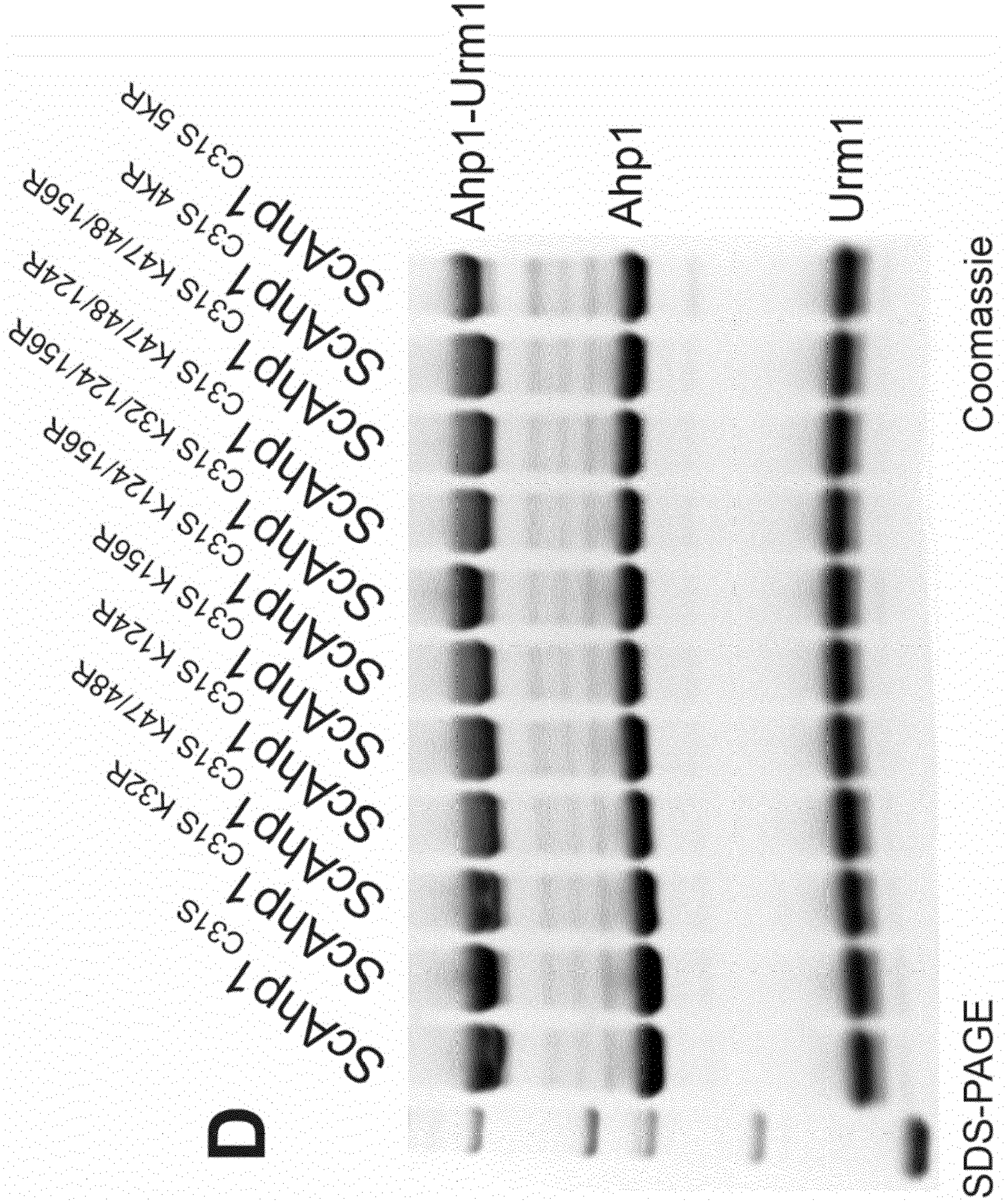
D
SCAhp1
SCAhp1 C31S
SCAhp1 C31S K32R
SCAhp1 C31S K47/48R
SCAhp1 C31S K124R
SCAhp1 C31S K156R
SCAhp1 C31S K124/156R
SCAhp1 C31S K32/124/156R
SCAhp1 C31S K47/48/124R
SCAhp1 C31S K47/48/156R
C31S 4KR
C31S 5KR
Ahp1-Urm1
Ahp1
Urm1
SDS-PAGE
Coomassie

Fig. 3 D

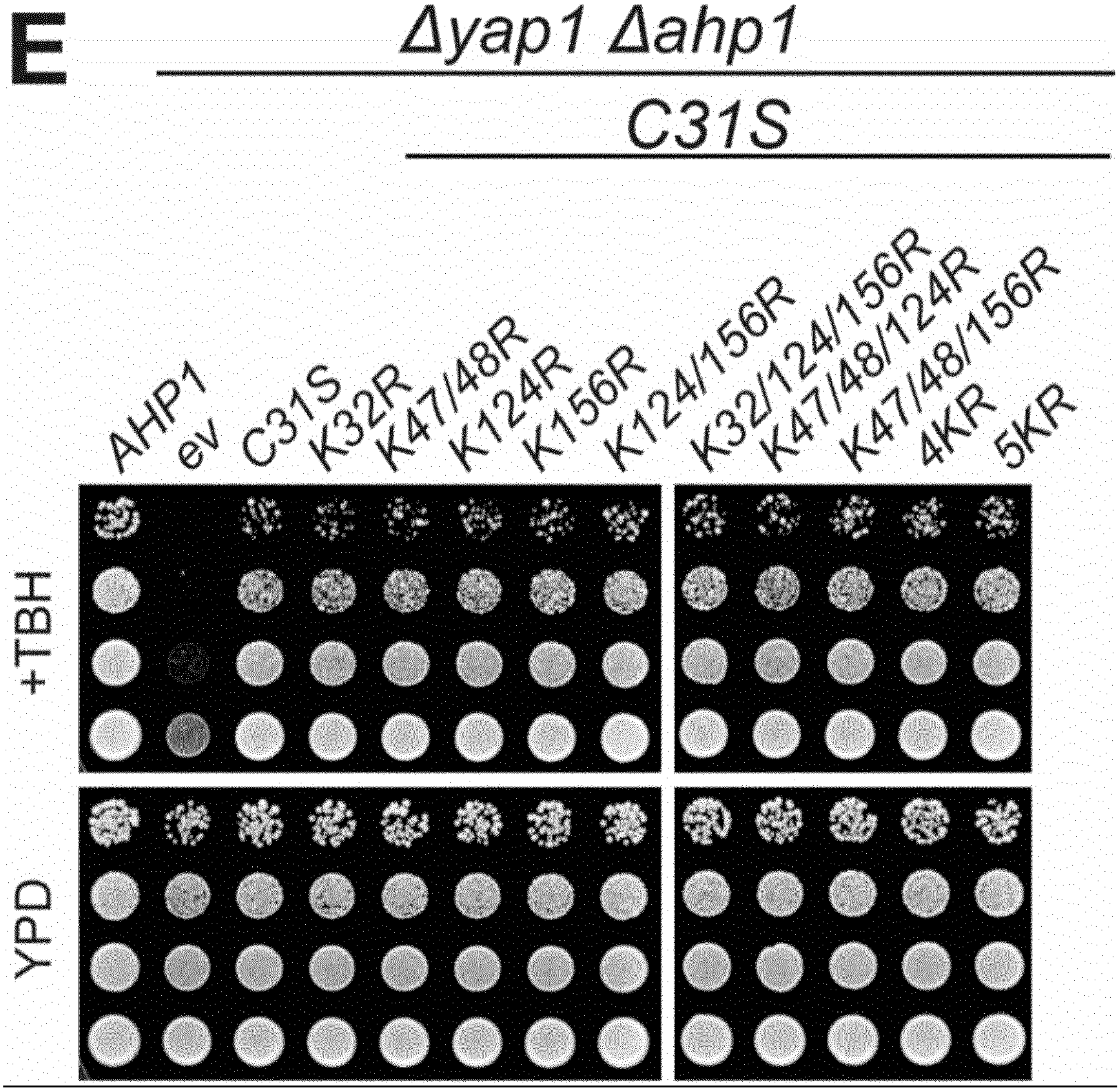
E
Δyap1 Δahp1
C31S
AHP1
ev
C31S
K32R
K47/48R
K124R
K156R
K124/156R
K32/124/156R
K47/48/124R
K47/48/156R
4KR
5KR
+TBH
YPD

Fig. 3 E

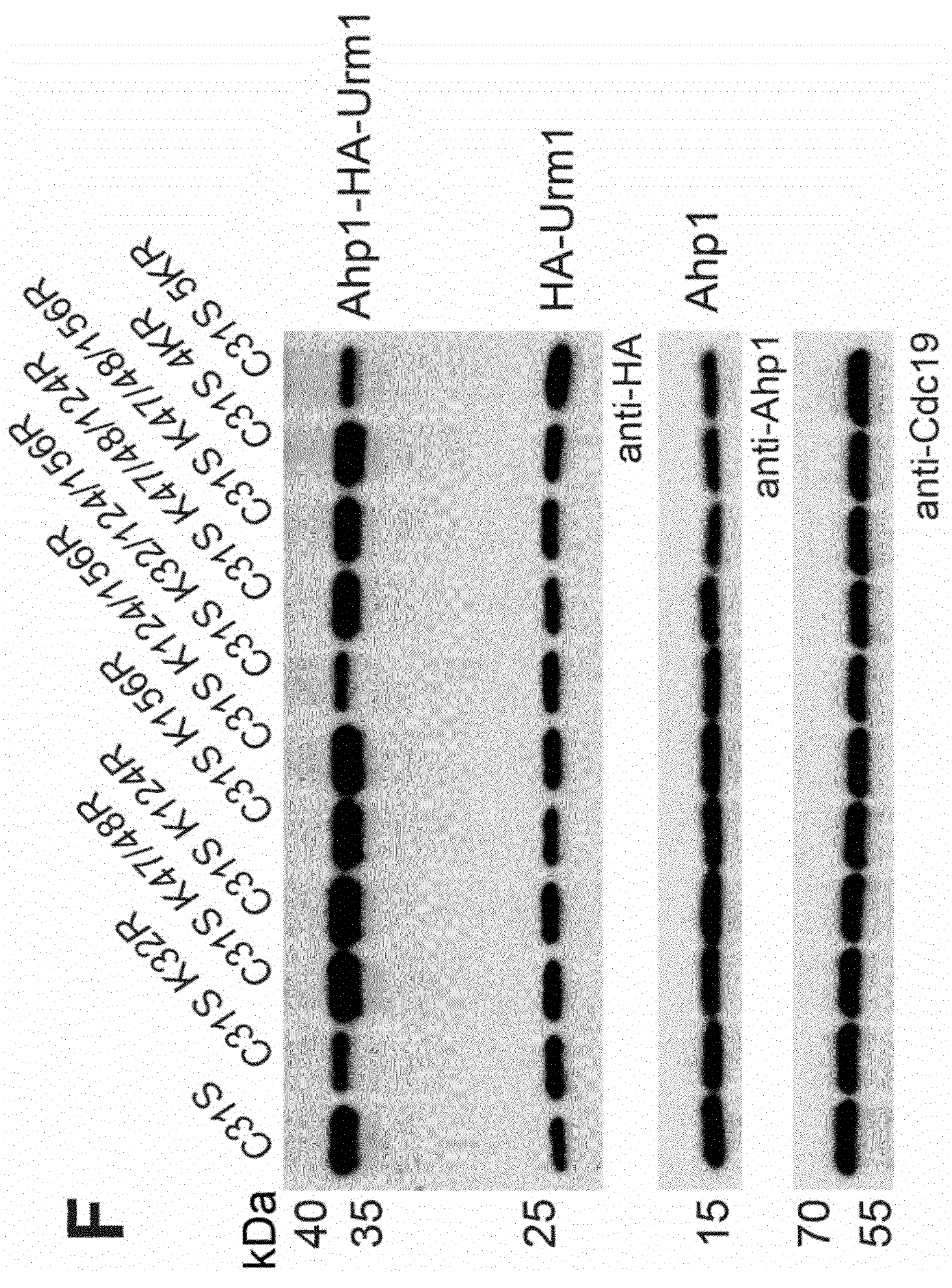
F
kDa
40
35
25
15
70
55
C31S
C31S K32R
C31S K47/48R
C31S K124R
C31S K156R
C31S K124/156R
C31S K32/124/156R
C31S K47/48/124R
C31S K47/48/156R
C31S 4KR
C31S 5KR
Ahp1-HA-Urm1
HA-Urm1
Ahp1
anti-HA
anti-Ahp1
anti-Cdc19

Fig. 3 F

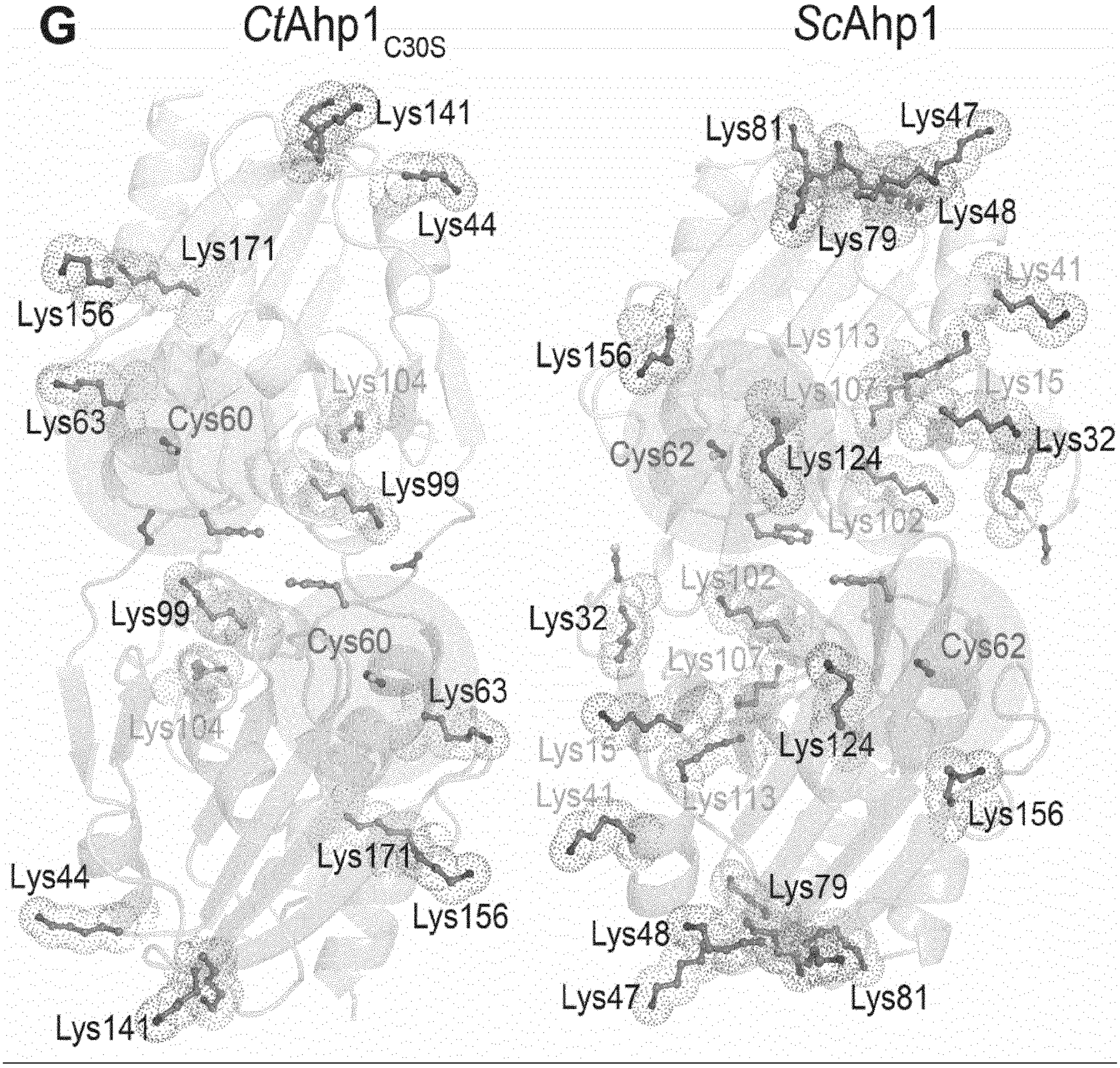
G
CtAhp1 C30S
ScAhp1
Lys141
Lys44
Lys171
Lys156
Lys104
Lys63
Cys60
Lys99
Lys99
Cys60
Lys63
Lys104
Lys171
Lys44
Lys156
Lys141
Lys81
Lys47
Lys48
Lys79
Lys41
Lys113
Lys156
Lys107
Lys15
Cys62
Lys124
Lys32
Lys102
Lys102
Lys32
Lys107
Cys62
Lys15
Lys124
Lys41
Lys113
Lys156
Lys79
Lys48
Lys47
Lys81

Fig. 3 G

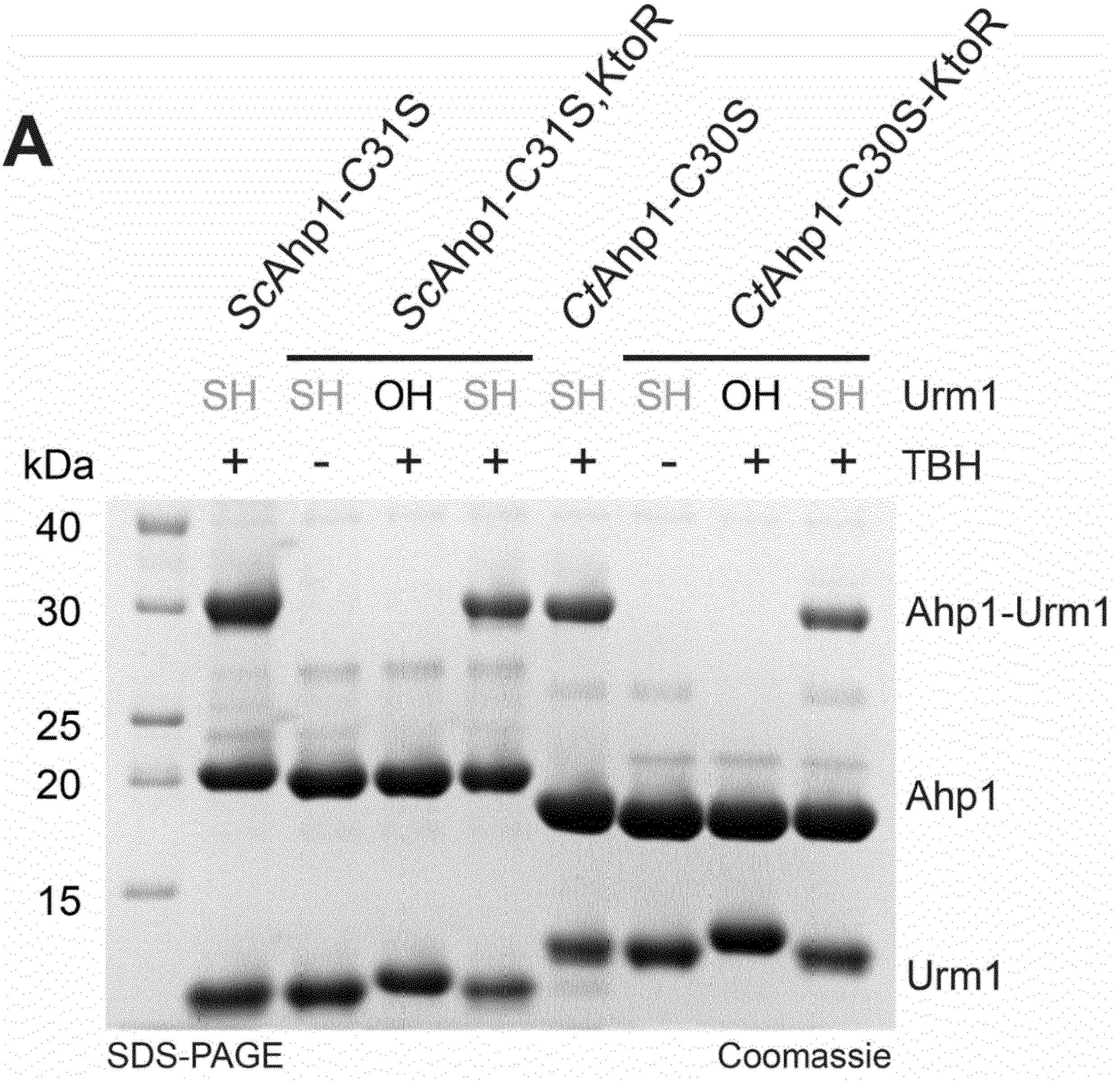
A
ScAhp1-C31S
ScAhp1-C31S,KtoR
CtAhp1-C30S
CtAhp1-C30S-KtoR
SH SH OH SH SH SH OH SH Urm1
kDa + - + + + - + + TBH
40
30
25
20
15
Ahp1-Urm1
Ahp1
Urm1
SDS-PAGE
Coomassie

Fig. 4 A

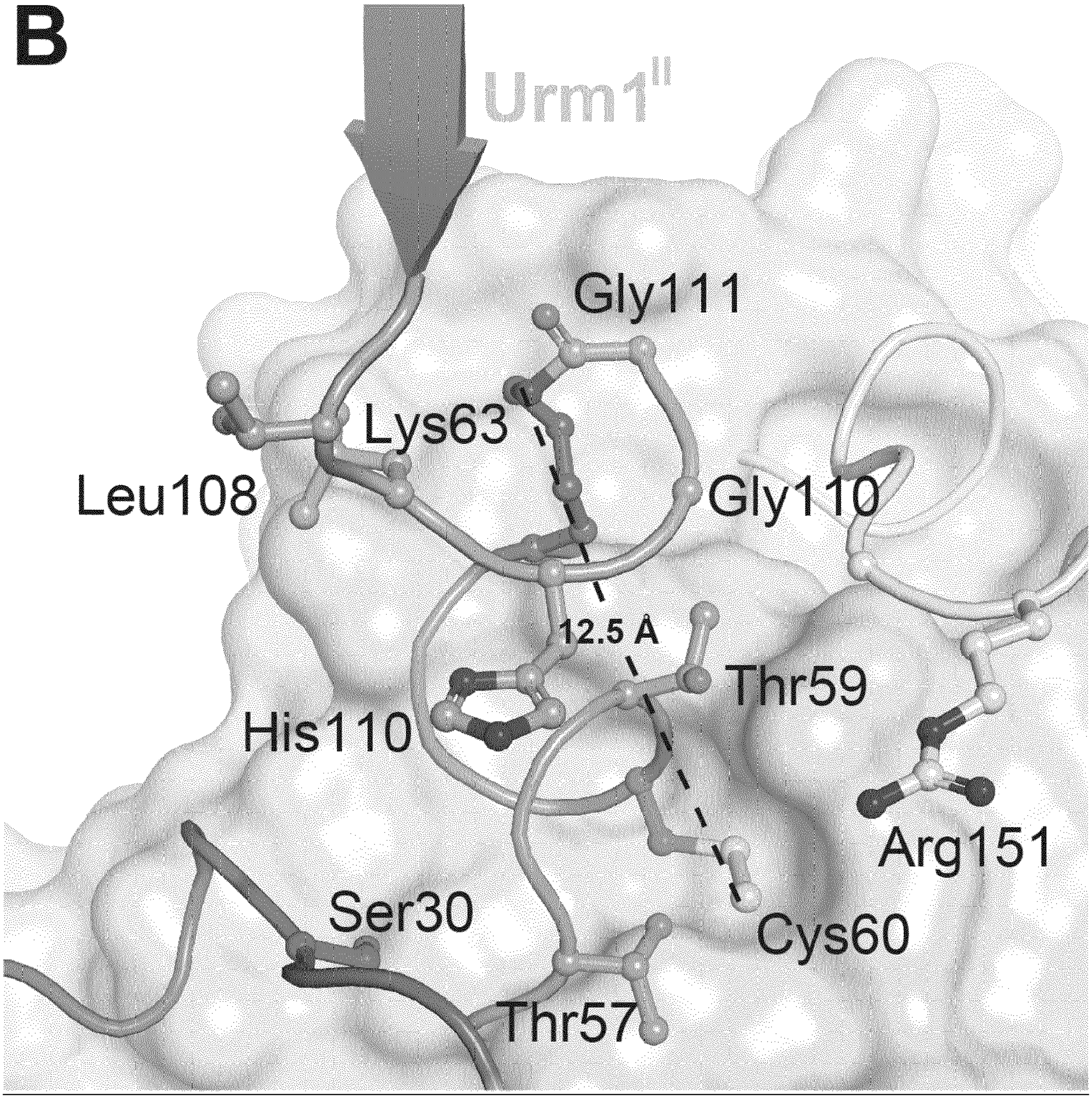
B
Urm1[II]
Gly111
Lys63
Leu108
Gly110
12.5 Å
Thr59
His110
Arg151
Ser30
Cys60
Thr57

Fig. 4 B

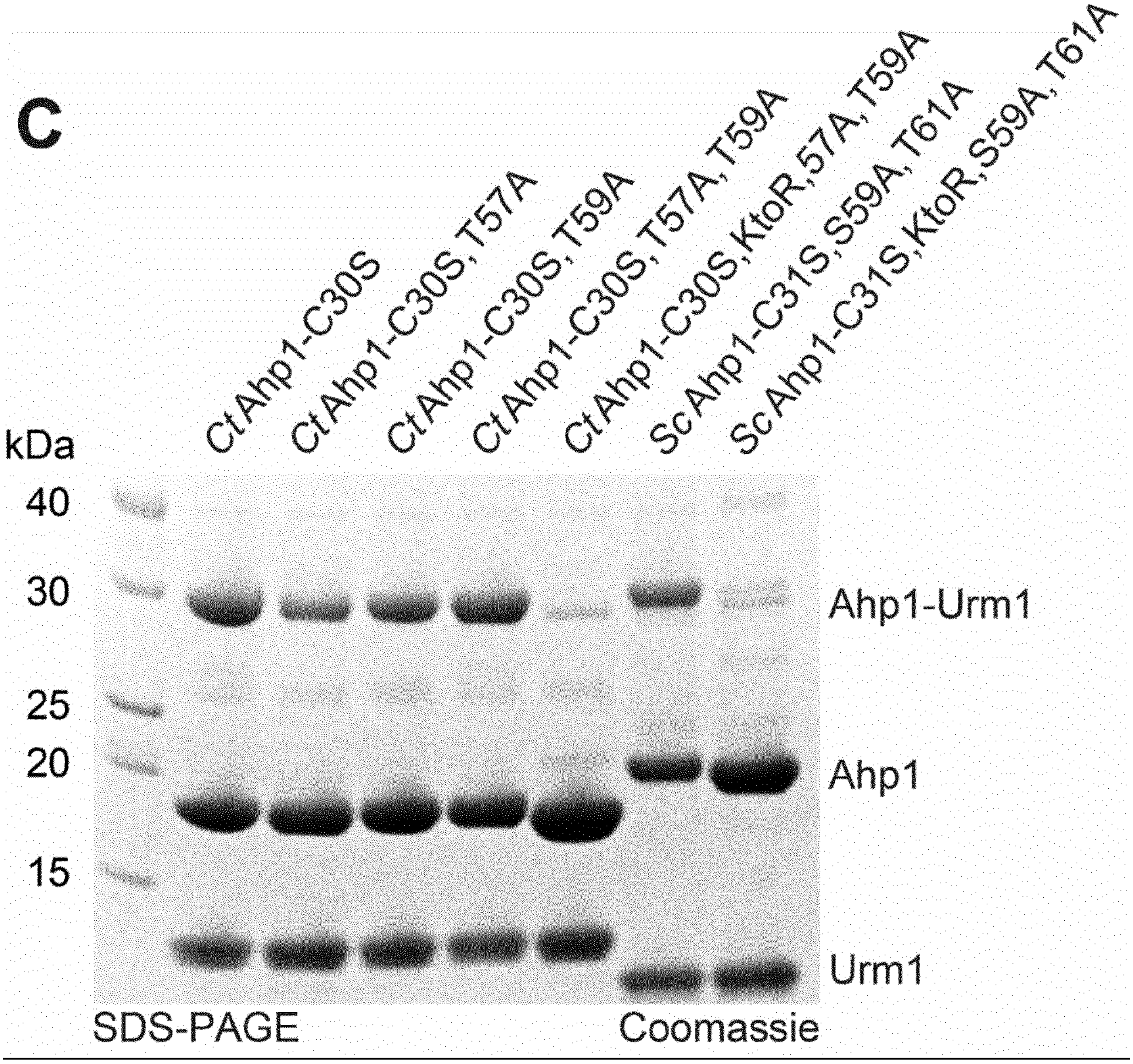
C
CtAhp1-C30S
CtAhp1-C30S,T57A
CtAhp1-C30S,T59A
CtAhp1-C30S,T57A,T59A
CtAhp1-C30S,KtoR,57A,T59A
ScAhp1-C31S,S59A,T61A
ScAhp1-C31S,KtoR,S59A,T61A
kDa
40
30
25
20
15
Ahp1-Urm1
Ahp1
Urm1
SDS-PAGE
Coomassie

Fig. 4 C

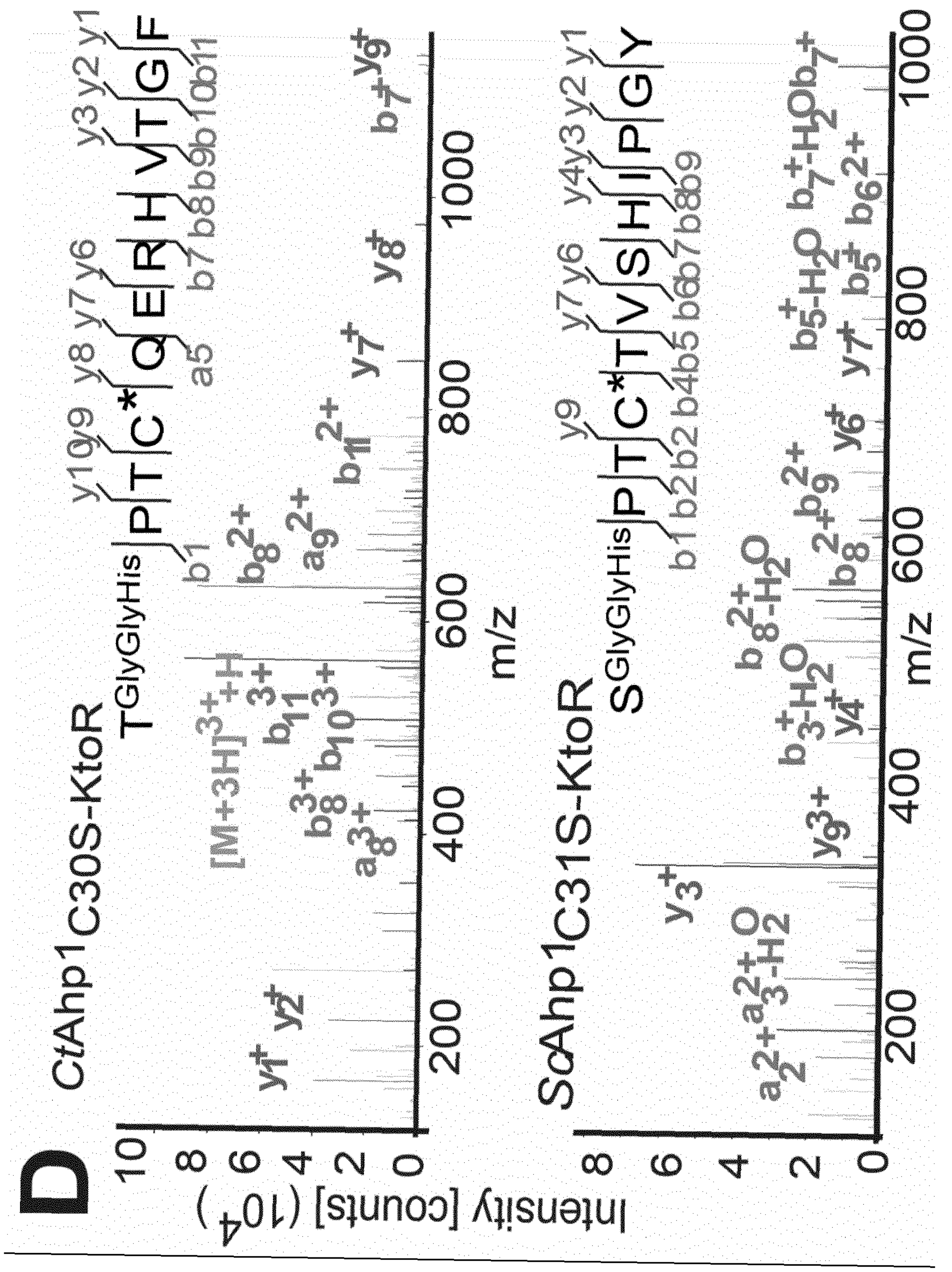


Fig. 4 D

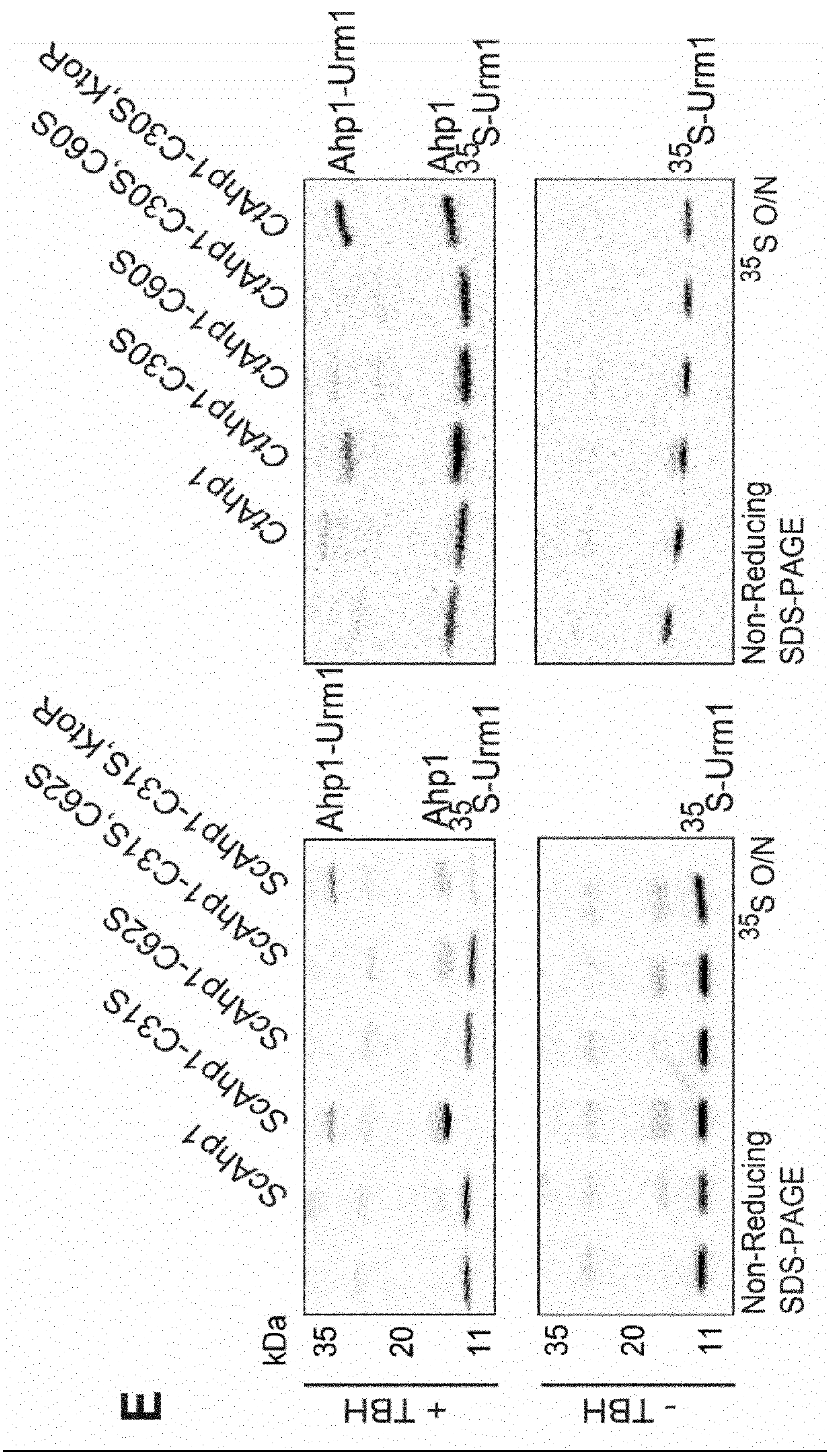
E
+ TBH
- TBH
kDa
35
20
11
ScAhp1
ScAhp1-C31S
ScAhp1-C62S
ScAhp1-C31S,C62S
ScAhp1-C31S,KtoR
CtAhp1
CtAhp1-C30S
CtAhp1-C60S
CtAhp1-C30S,C60S
CtAhp1-C30S,KtoR
Ahp1-Urm1
Ahp1
35S-Urm1
35S O/N
Non-Reducing
SDS-PAGE

Fig. 4 E

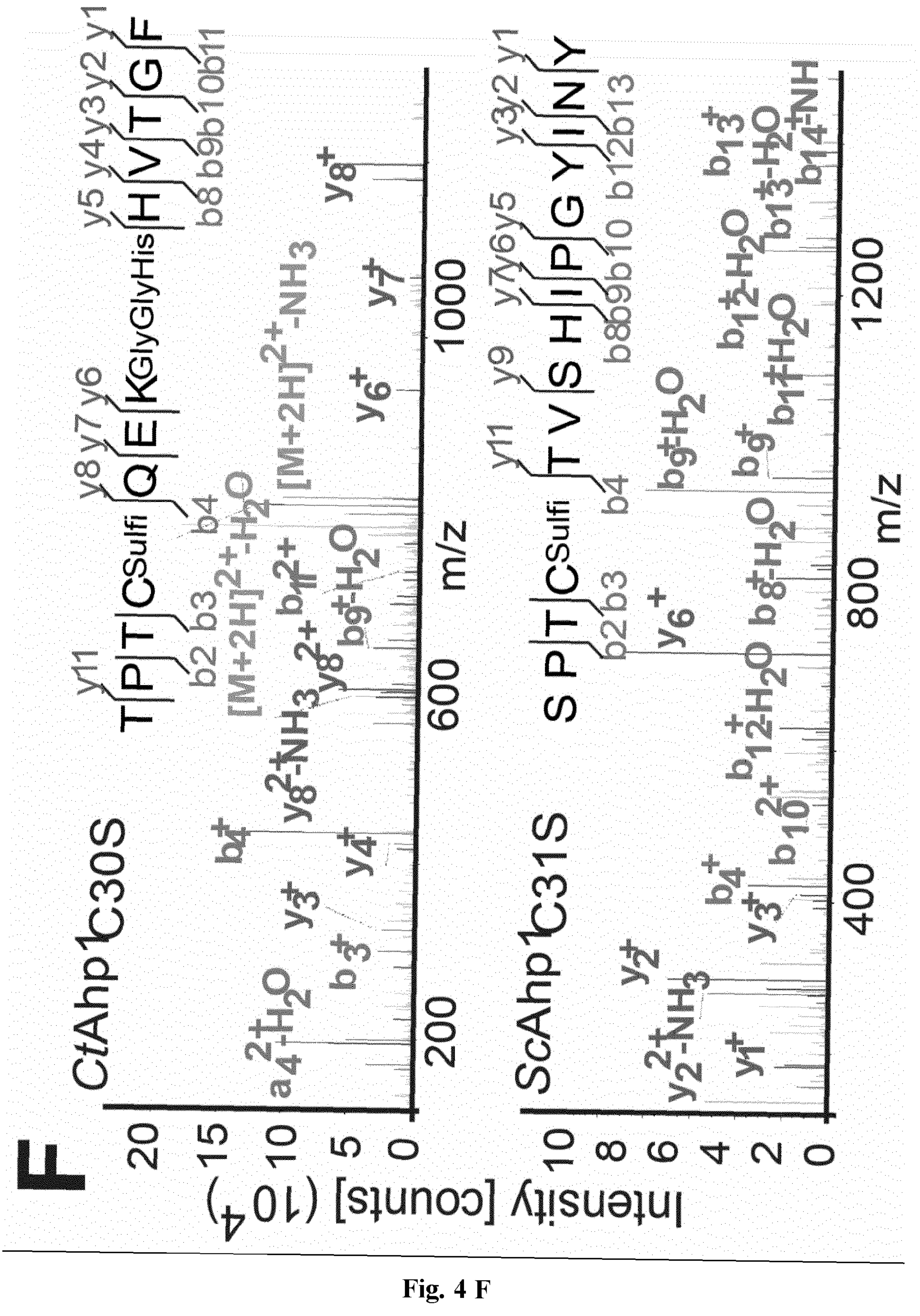


Fig. 4 F

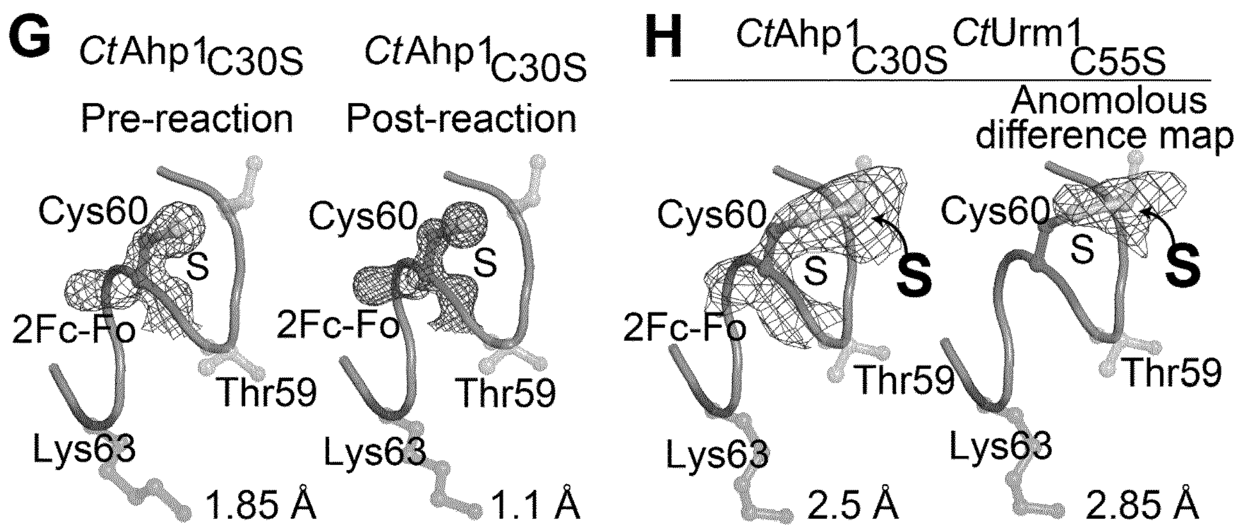


Fig. 4 G, H

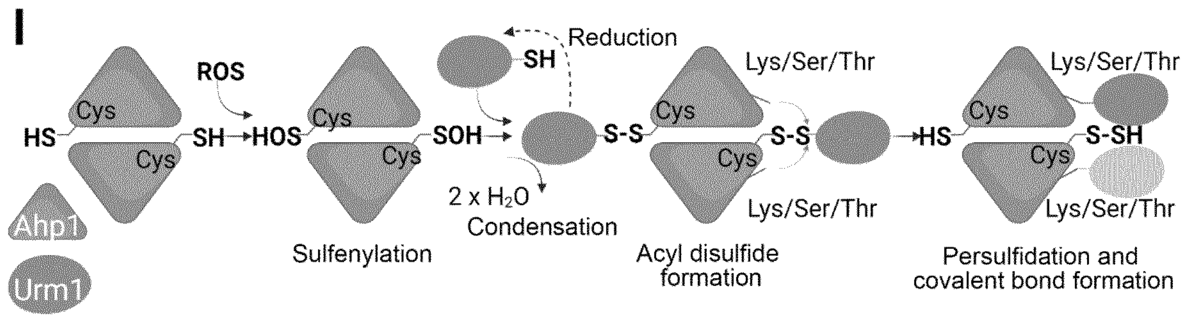


Fig. 4 I

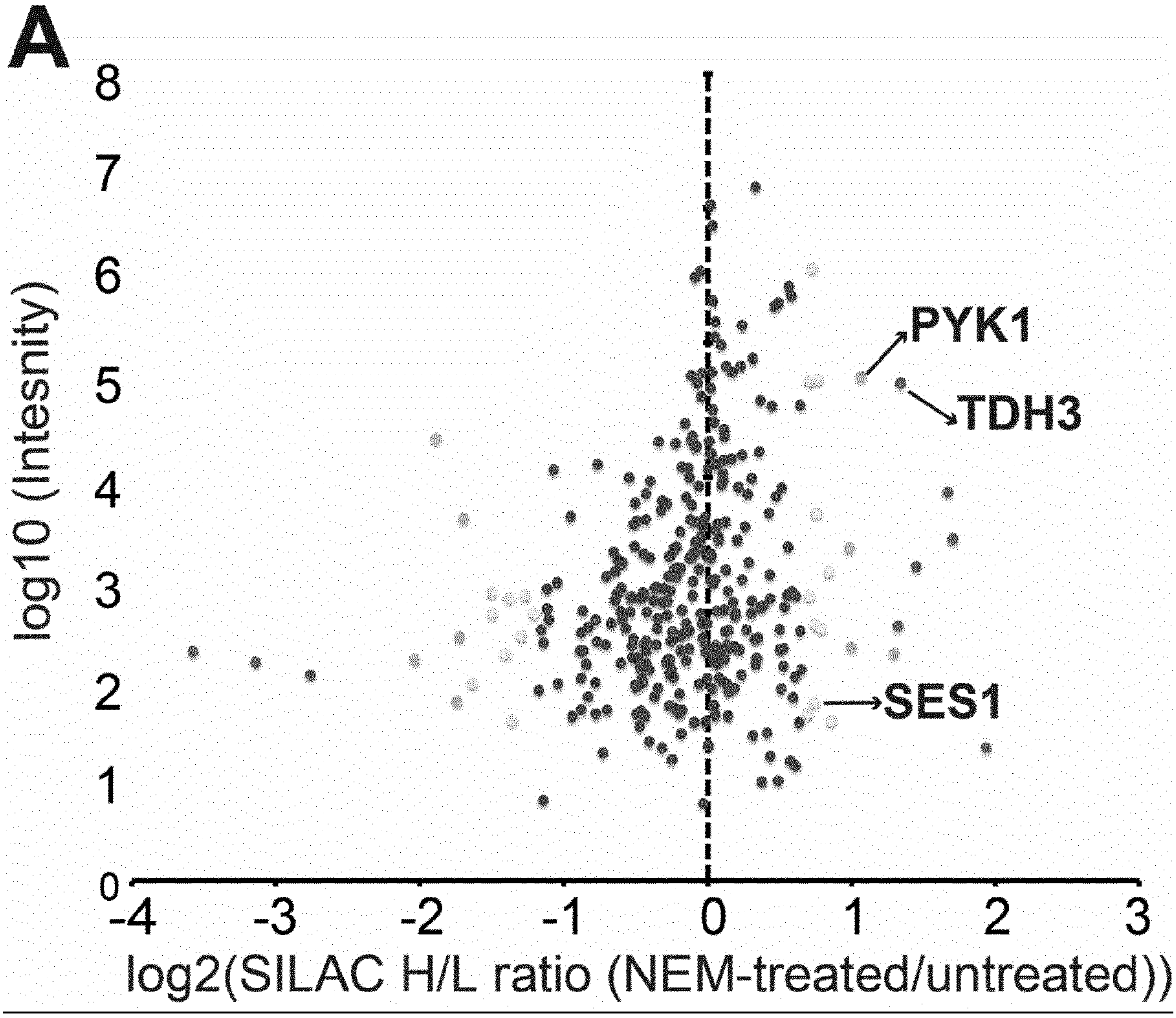
A
8
7
6
5
4
3
2
1
0
log10 (Intesnity)
-4
-3
-2
-1
0
1
2
3
log2(SILAC H/L ratio (NEM-treated/untreated))
PYK1
TDH3
SES1

Fig. 5 A

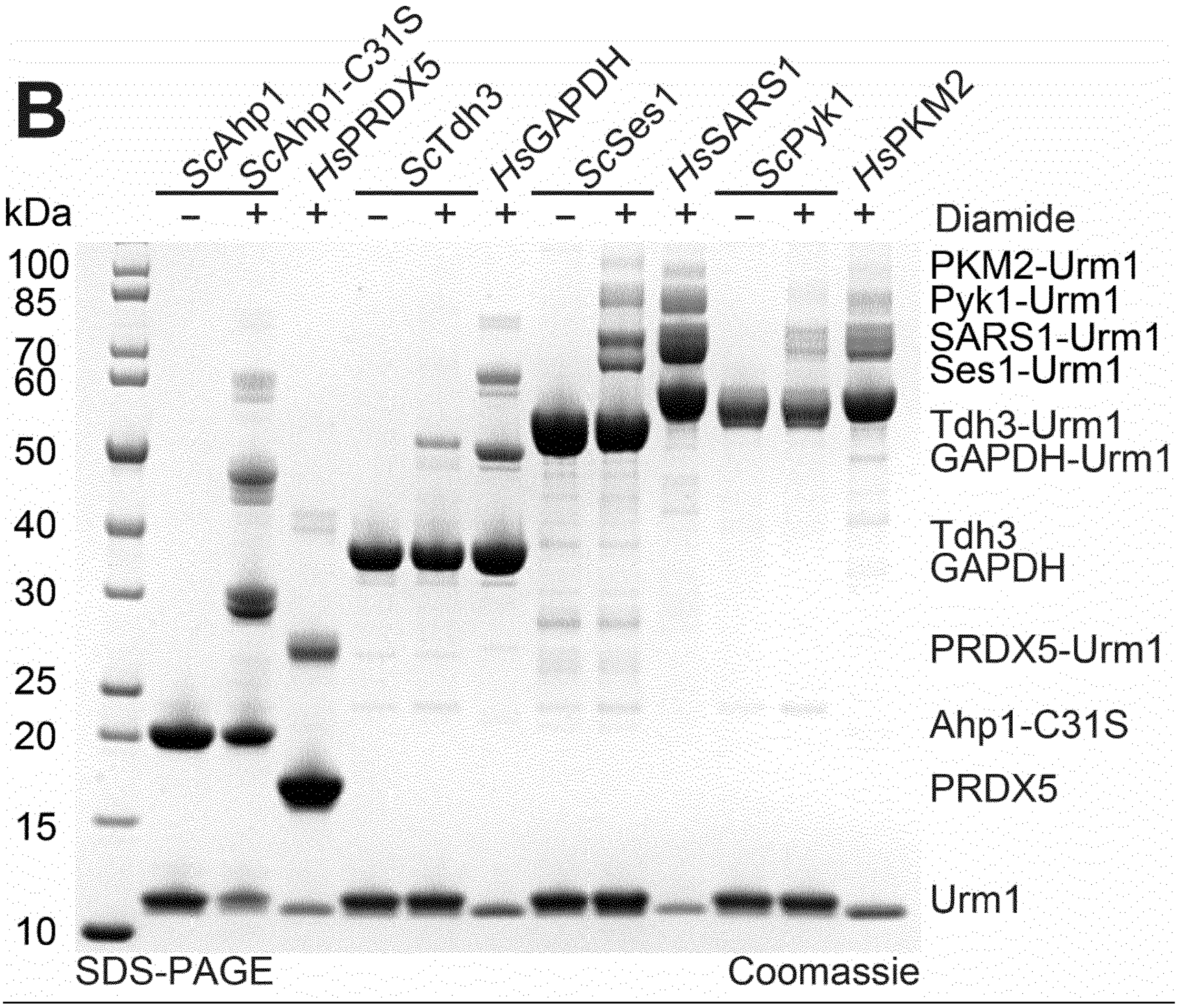
B
ScAhp1
ScAhp1-C31S
HsPRDX5
ScTdh3
HsGAPDH
ScSes1
HsSARS1
ScPyk1
HsPKM2
kDa
– + + – + + – + + – + +
Diamide
100
85
70
60
50
40
30
25
20
15
10
PKM2-Urm1
Pyk1-Urm1
SARS1-Urm1
Ses1-Urm1
Tdh3-Urm1
GAPDH-Urm1
Tdh3
GAPDH
PRDX5-Urm1
Ahp1-C31S
PRDX5
Urm1
SDS-PAGE
Coomassie

**Fig. 5 B**

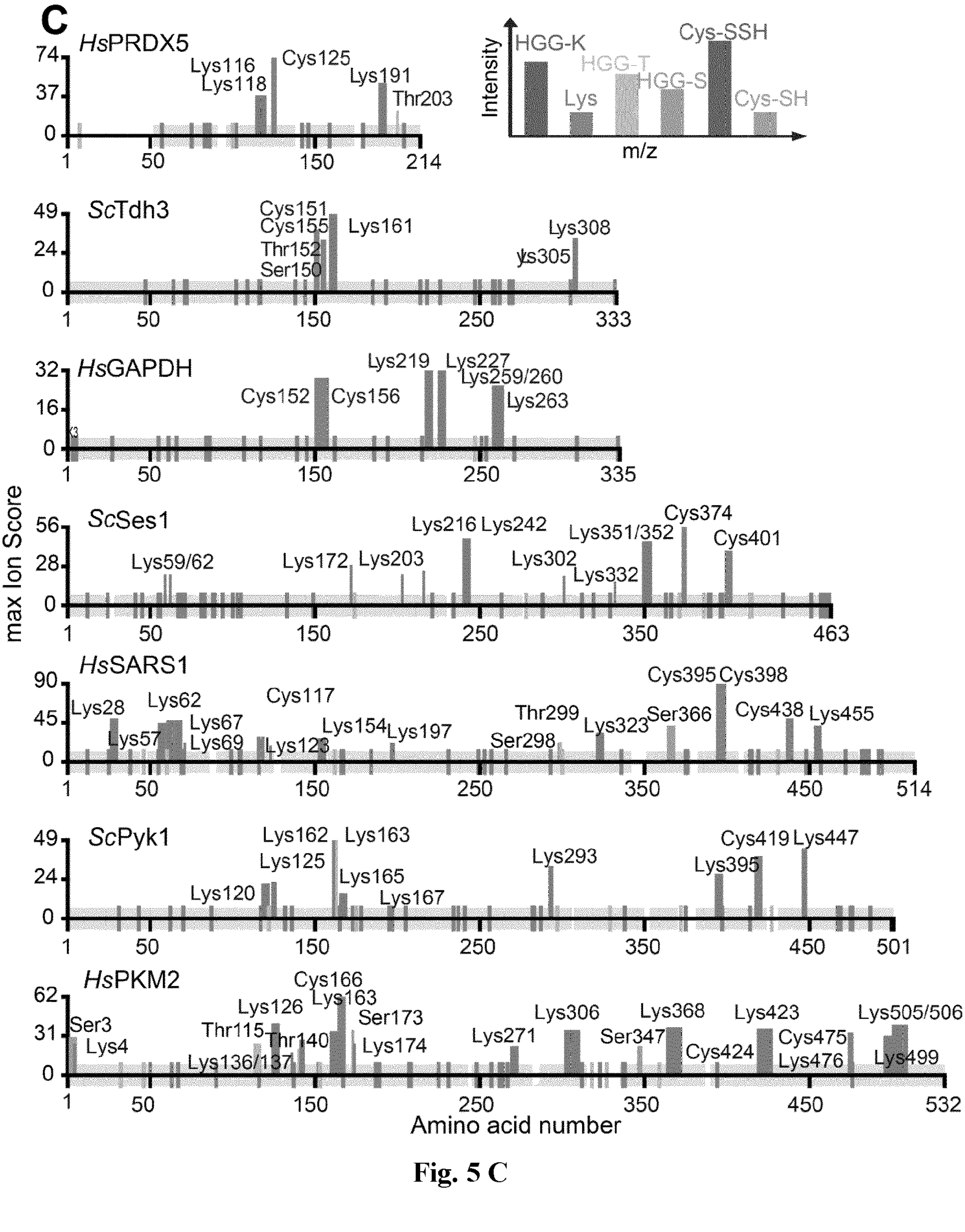
C
HsPRDX5
Lys116
Lys118
Cys125
Lys191
Thr203
Intensity
HGG-K
Lys
HGG-T
HGG-S
Cys-SSH
Cys-SH
m/z
ScTdh3
Cys151
Cys155
Thr152
Ser150
Lys161
Lys308
Lys305
HsGAPDH
Cys152
Cys156
Lys219
Lys227
Lys259/260
Lys263
max Ion Score
ScSes1
Lys59/62
Lys172
Lys203
Lys216
Lys242
Lys302
Lys332
Lys351/352
Cys374
Cys401
HsSARS1
Lys28
Lys57
Lys62
Lys67
Lys69
Cys117
Lys123
Lys154
Lys197
Thr299
Ser298
Lys323
Ser366
Cys395
Cys398
Cys438
Lys455
ScPyk1
Lys120
Lys125
Lys162
Lys163
Lys165
Lys167
Lys293
Lys395
Cys419
Lys447
HsPKM2
Ser3
Lys4
Thr115
Lys126
Lys136/137
Thr140
Cys166
Lys163
Ser173
Lys174
Lys271
Lys306
Ser347
Lys368
Cys424
Lys423
Cys475
Lys476
Lys505/506
Lys499
Amino acid number

Fig. 5 C

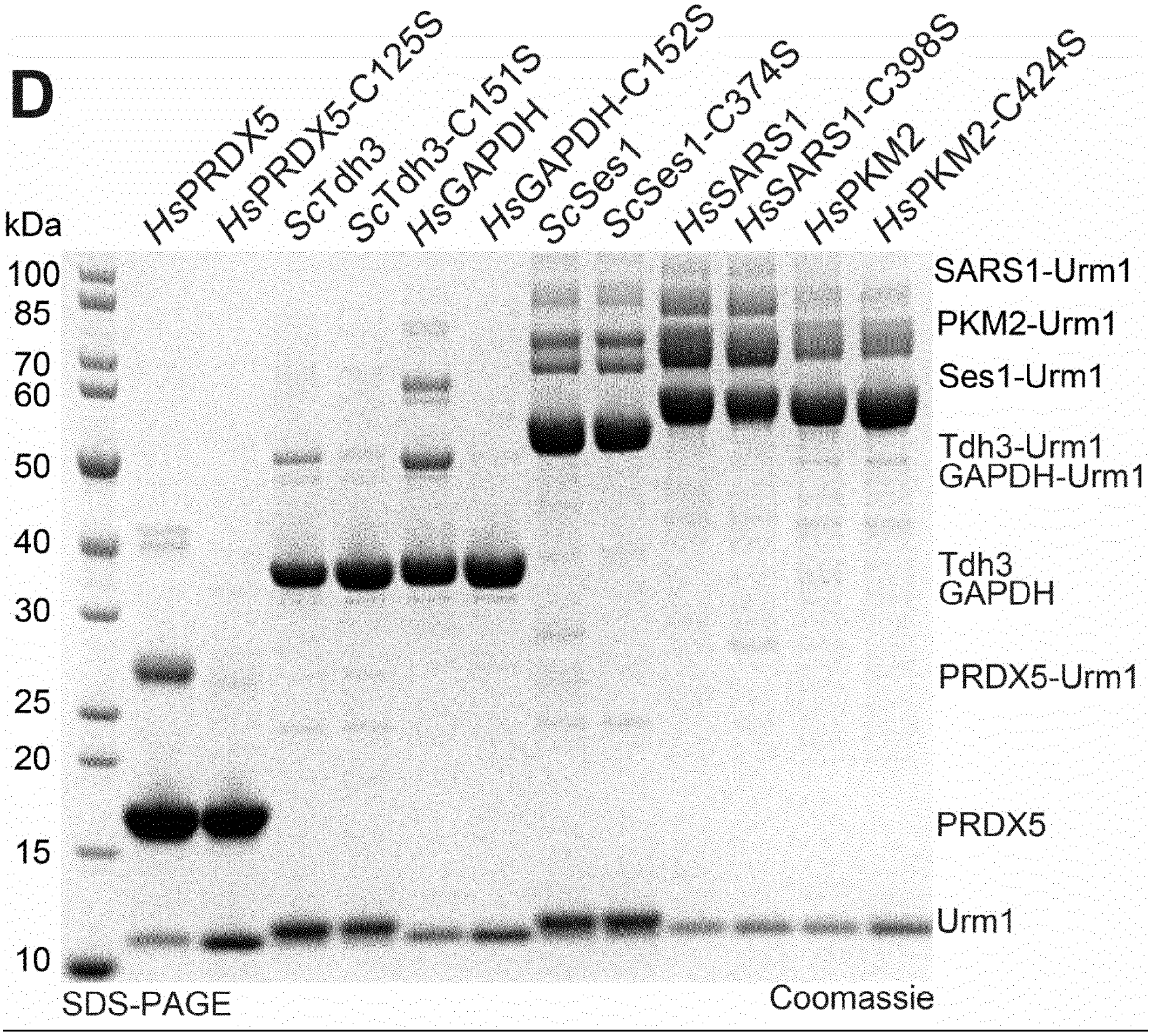
D
kDa
HsPRDX5
HsPRDX5-C125S
ScTdh3
ScTdh3-C151S
HsGAPDH
HsGAPDH-C152S
ScSes1
ScSes1-C374S
HsSARS1
HsSARS1-C398S
HsPKM2
HsPKM2-C424S
100
85
70
60
50
40
30
25
20
15
10
SARS1-Urm1
PKM2-Urm1
Ses1-Urm1
Tdh3-Urm1
GAPDH-Urm1
Tdh3
GAPDH
PRDX5-Urm1
PRDX5
Urm1
SDS-PAGE
Coomassie

Fig. 5 D

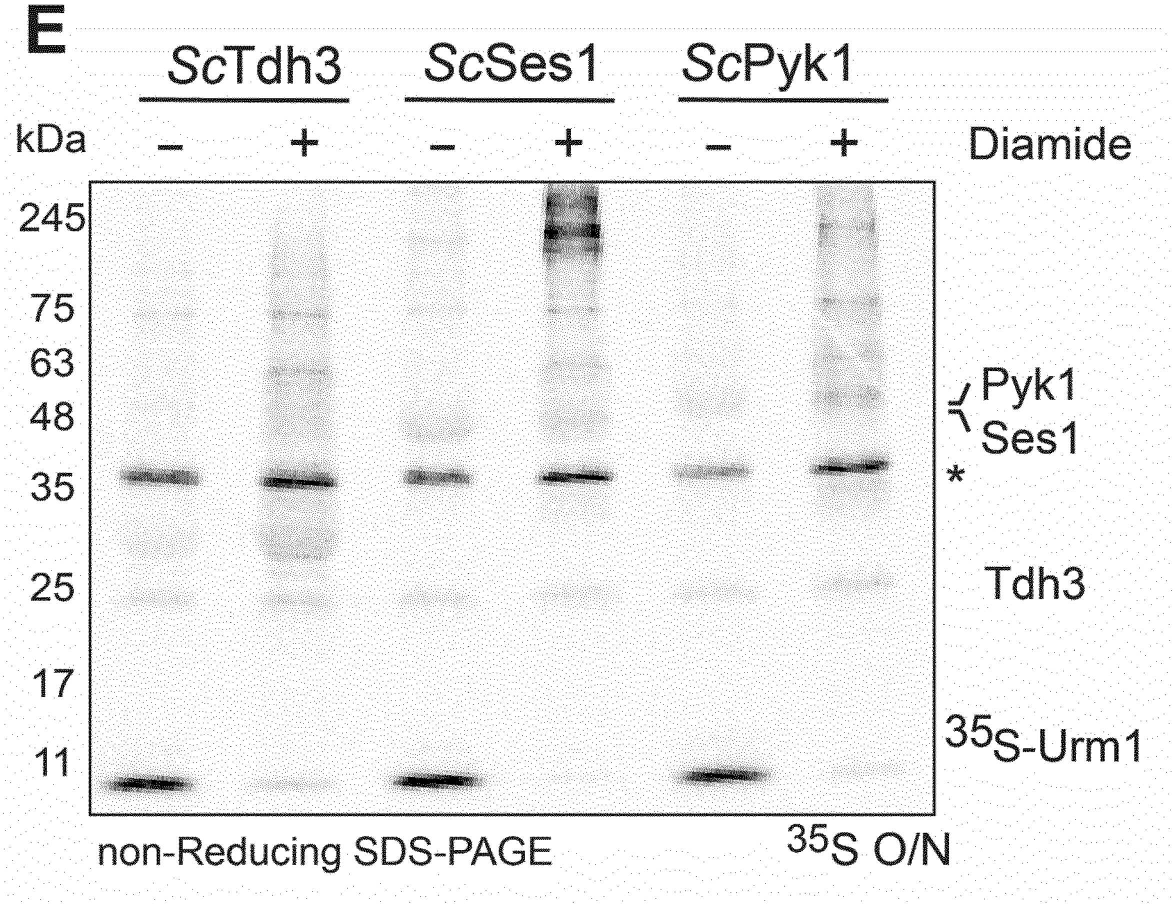
E
ScTdh3
ScSes1
ScPyk1
kDa
–
+
–
+
–
+
Diamide
245
75
63
48
35
25
17
11
Pyk1
Ses1
*
Tdh3
35S-Urm1
non-Reducing SDS-PAGE
35S O/N

Fig. 5 E

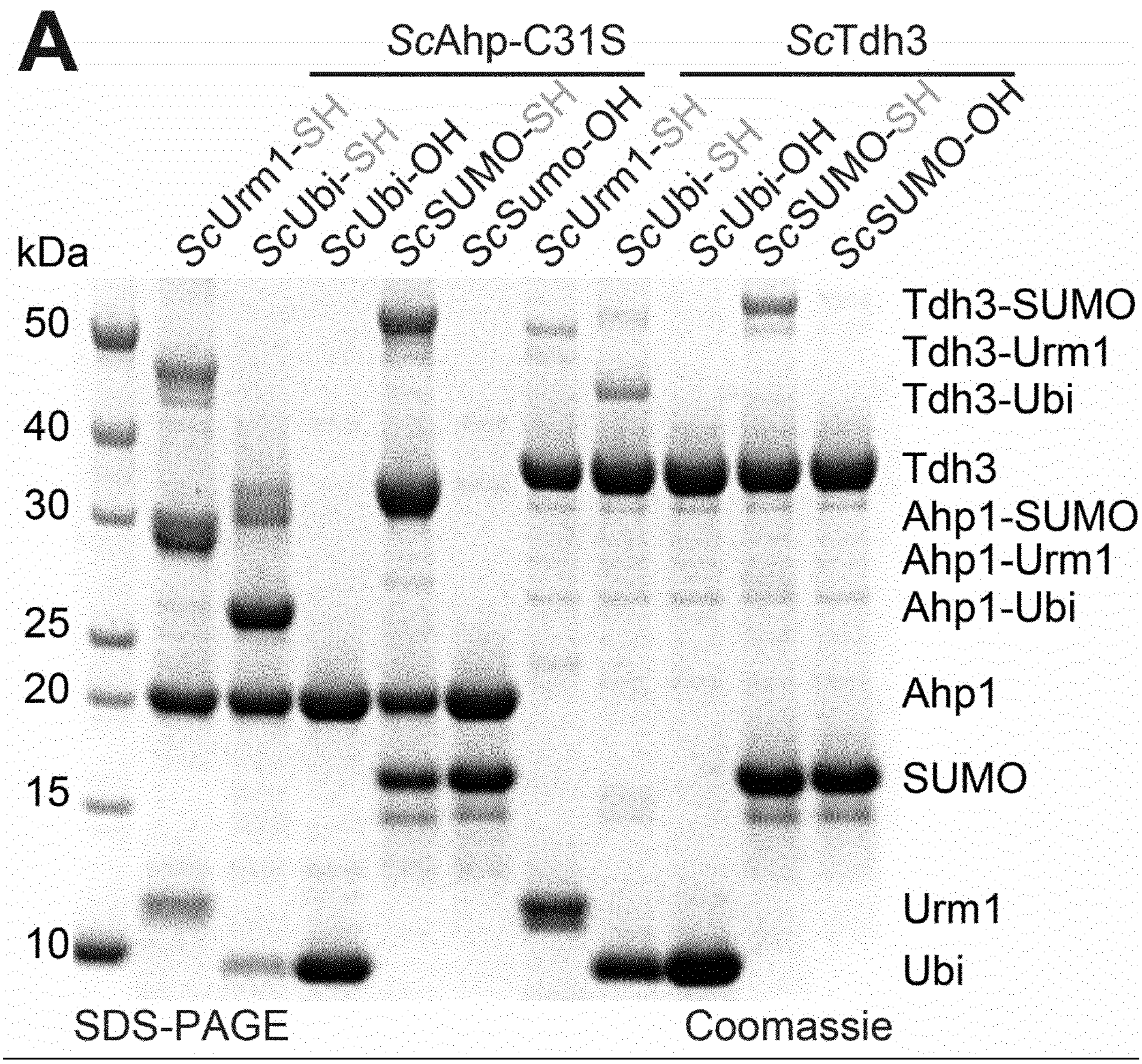
A
ScAhp-C31S
ScTdh3
ScUrm1-SH
ScUbi-SH
ScUbi-OH
ScSUMO-SH
ScSumo-OH
ScUrm1-SH
ScUbi-SH
ScUbi-OH
ScSUMO-SH
ScSUMO-OH
kDa
50
40
30
25
20
15
10
Tdh3-SUMO
Tdh3-Urm1
Tdh3-Ubi
Tdh3
Ahp1-SUMO
Ahp1-Urm1
Ahp1-Ubi
Ahp1
SUMO
Urm1
Ubi
SDS-PAGE
Coomassie

Fig. 6 A

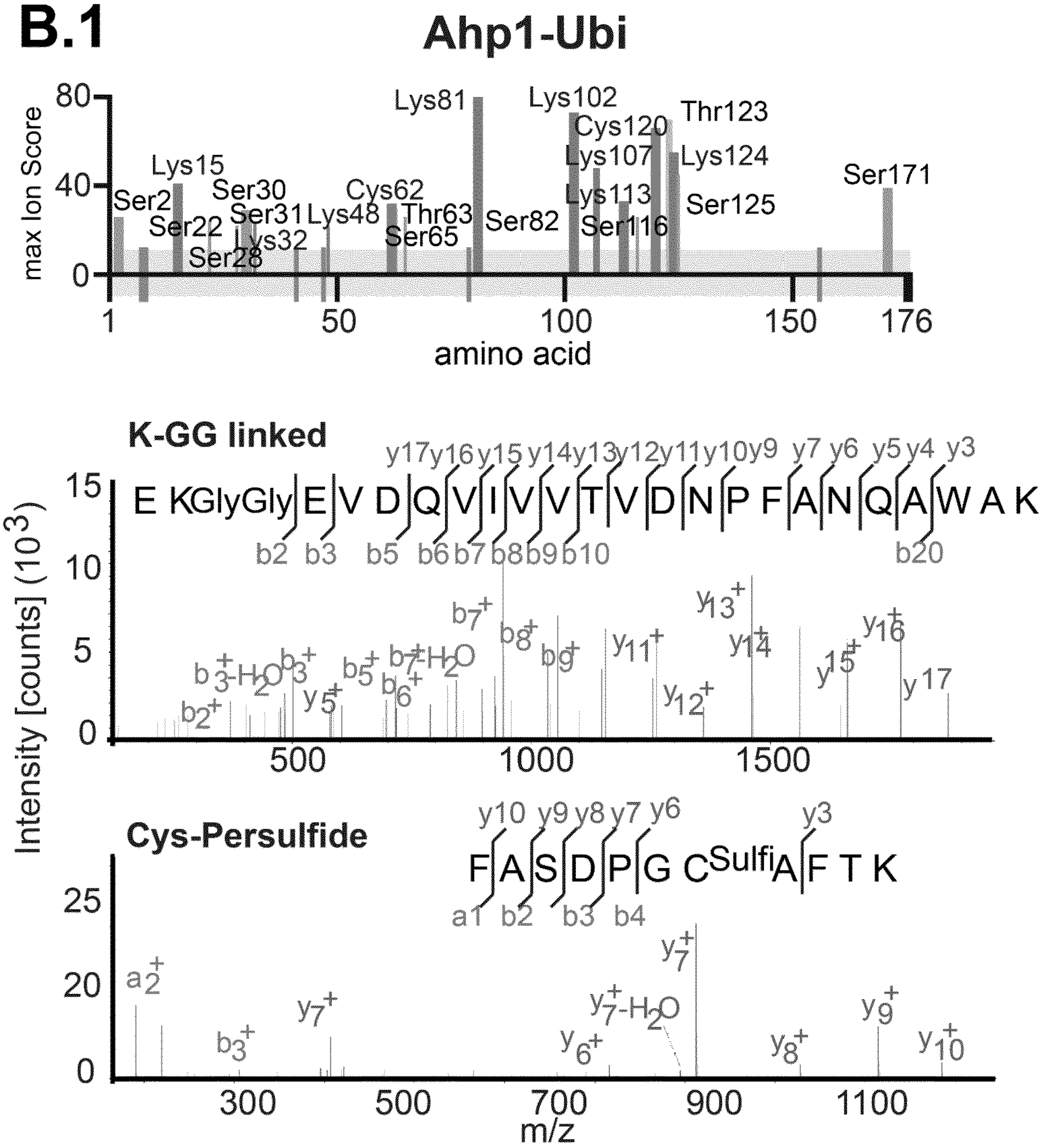
B.1
Ahp1-Ubi
max Ion Score
amino acid
Lys81
Lys102
Cys120
Thr123
Lys107
Lys124
Lys15
Ser171
Ser30
Cys62
Ser2
Lys113
Ser125
Ser31
Lys48
Thr63
Ser82
Ser22
Ser116
Lys32
Ser65
Ser28
K-GG linked
E KGlyGly E V D Q V I V V T V D N P F A N Q A W A K
Intensity [counts] (10^3)
Cys-Persulfide
F A S D P G C^Sulfi A F T K
m/z

Fig. 6 B.1

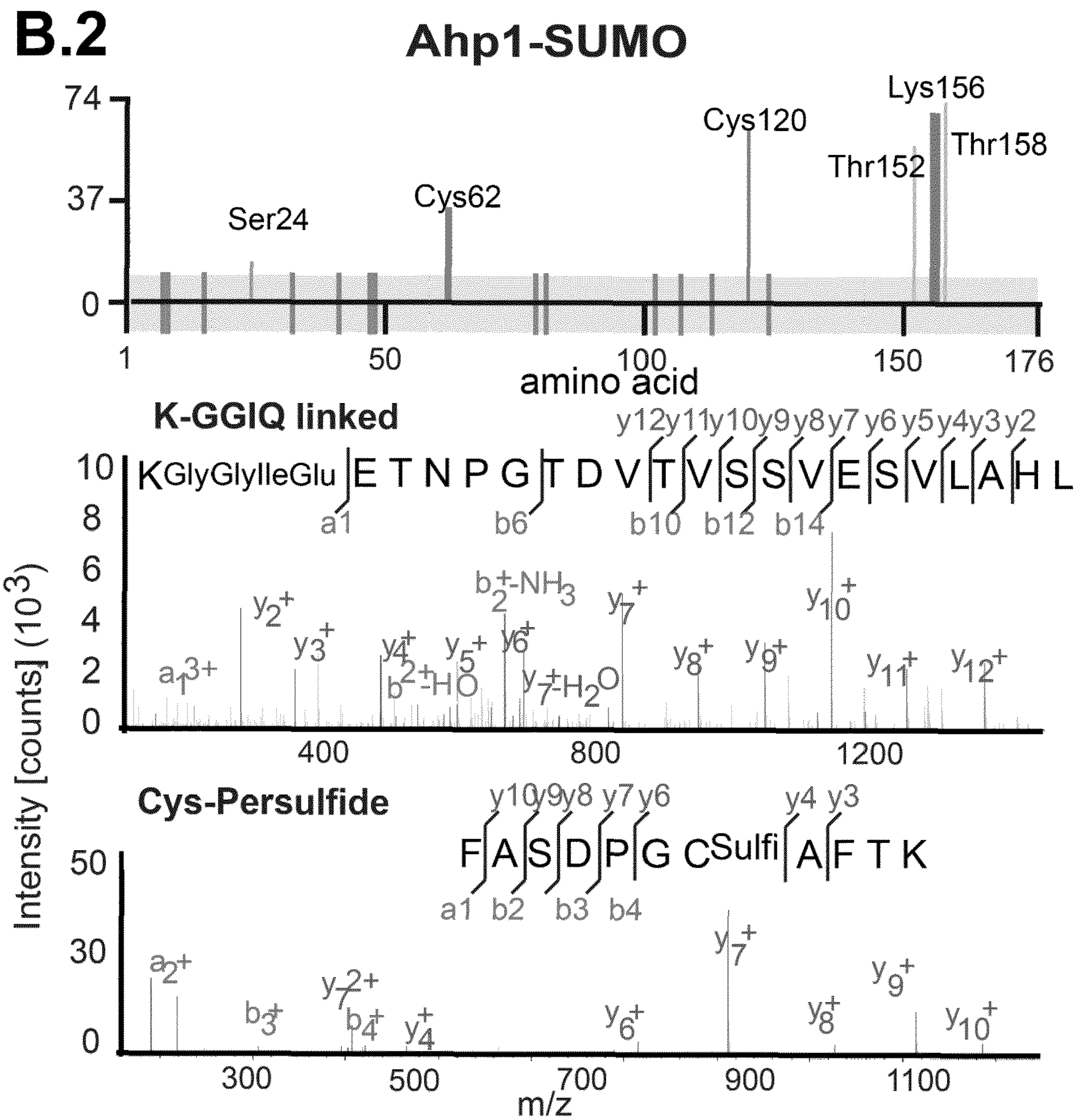
B.2
Ahp1-SUMO
74
37
0
Ser24
Cys62
Cys120
Thr152
Lys156
Thr158
1
50
100
150
176
amino acid
K-GGIQ linked
KGlyGlyIleGlu E T N P G T D V T V S S V E S V L A H L
Cys-Persulfide
F A S D P G CSulfi A F T K
Intensity [counts] (10³)
m/z

Fig. 6 B.2

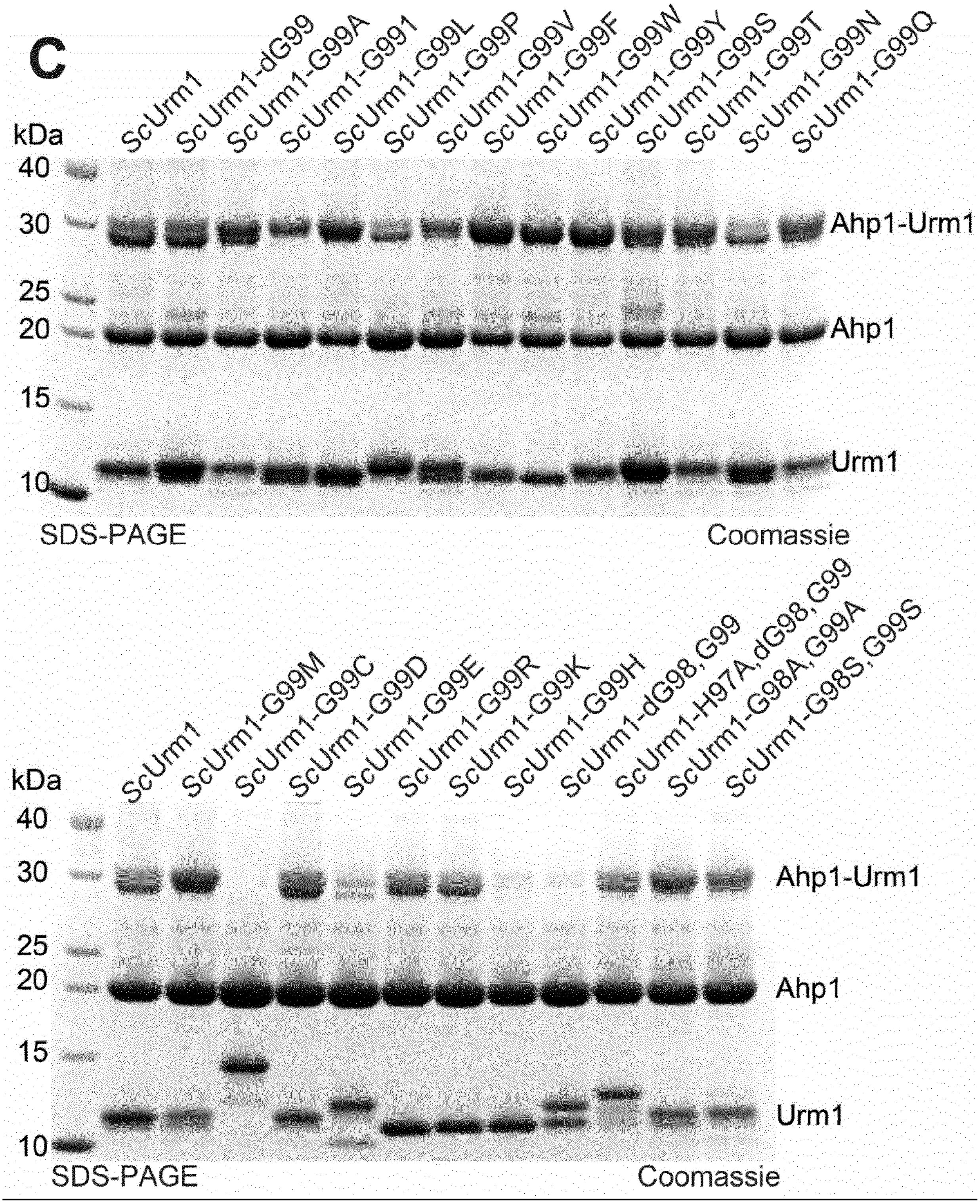
C
kDa
40
30
25
20
15
10
ScUrm1
ScUrm1-dG99
ScUrm1-G99A
ScUrm1-G99I
ScUrm1-G99L
ScUrm1-G99P
ScUrm1-G99V
ScUrm1-G99F
ScUrm1-G99W
ScUrm1-G99Y
ScUrm1-G99S
ScUrm1-G99T
ScUrm1-G99N
ScUrm1-G99Q
Ahp1-Urm1
Ahp1
Urm1
SDS-PAGE
Coomassie
kDa
40
30
25
20
15
10
ScUrm1
ScUrm1-G99M
ScUrm1-G99C
ScUrm1-G99D
ScUrm1-G99E
ScUrm1-G99R
ScUrm1-G99K
ScUrm1-G99H
ScUrm1-dG98,G99
ScUrm1-H97A,dG98,G99
ScUrm1-G98A,G99A
ScUrm1-G98S,G99S
Ahp1-Urm1
Ahp1
Urm1
SDS-PAGE
Coomassie

Fig. 6 C

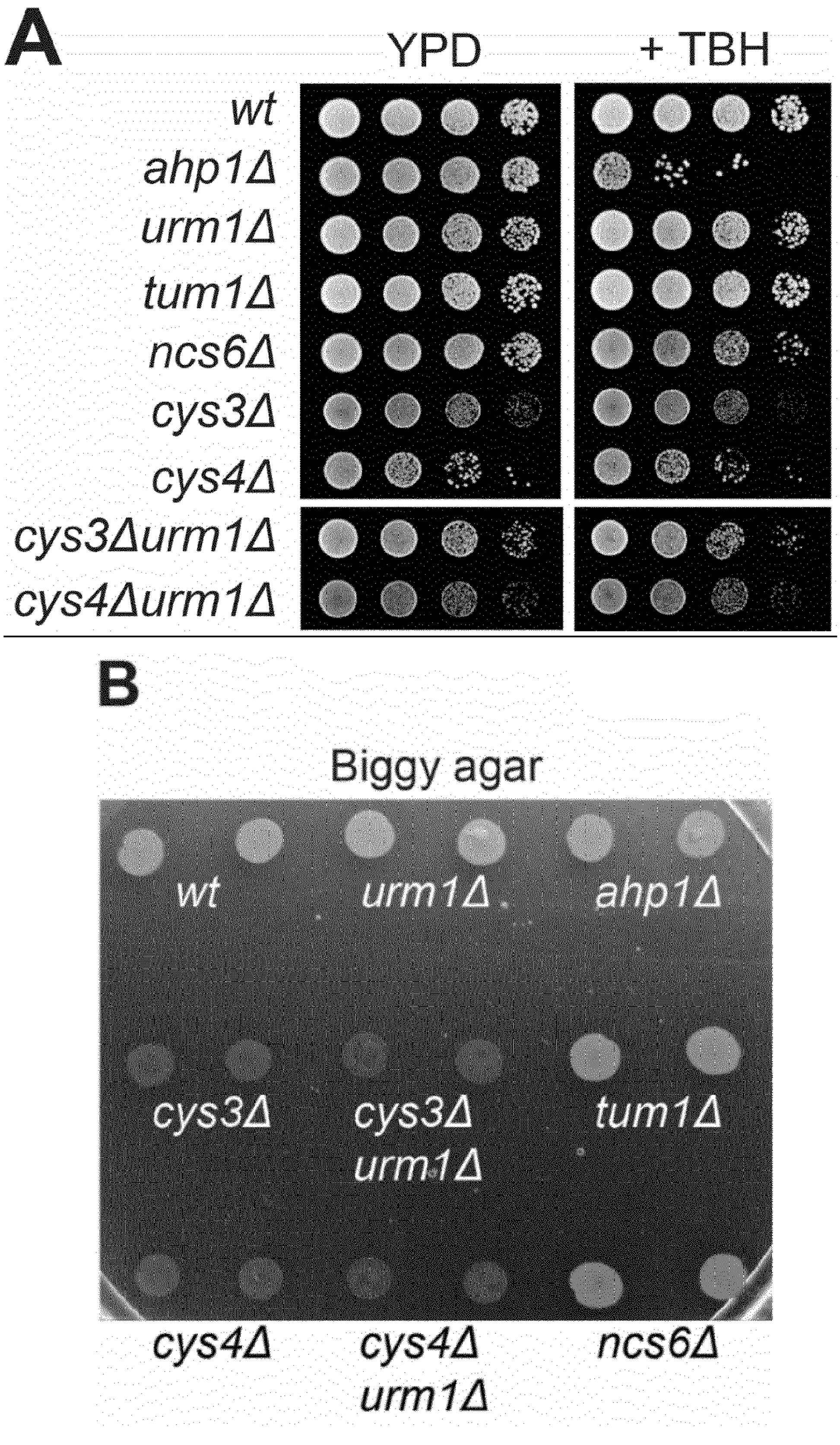
A
YPD
+ TBH
wt
ahp1Δ
urm1Δ
tum1Δ
ncs6Δ
cys3Δ
cys4Δ
cys3Δurm1Δ
cys4Δurm1Δ
B
Biggy agar
wt
urm1Δ
ahp1Δ
cys3Δ
cys3Δ urm1Δ
tum1Δ
cys4Δ
cys4Δ urm1Δ
ncs6Δ

Fig. 7 A, B

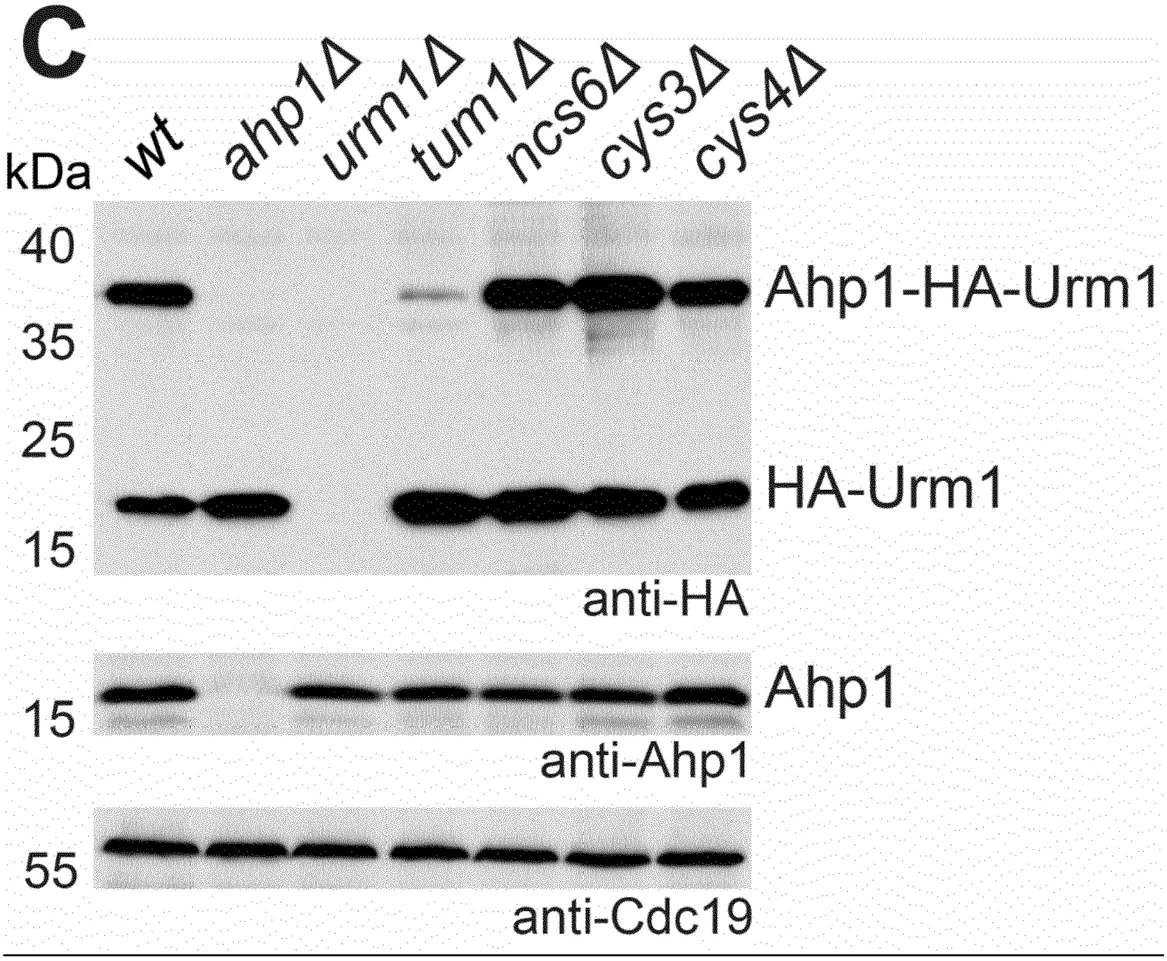
C
wt
ahp1Δ
urm1Δ
tum1Δ
ncs6Δ
cys3Δ
cys4Δ
kDa
40
35
25
15
Ahp1-HA-Urm1
HA-Urm1
anti-HA
15
Ahp1
anti-Ahp1
55
anti-Cdc19

Fig. 7 C

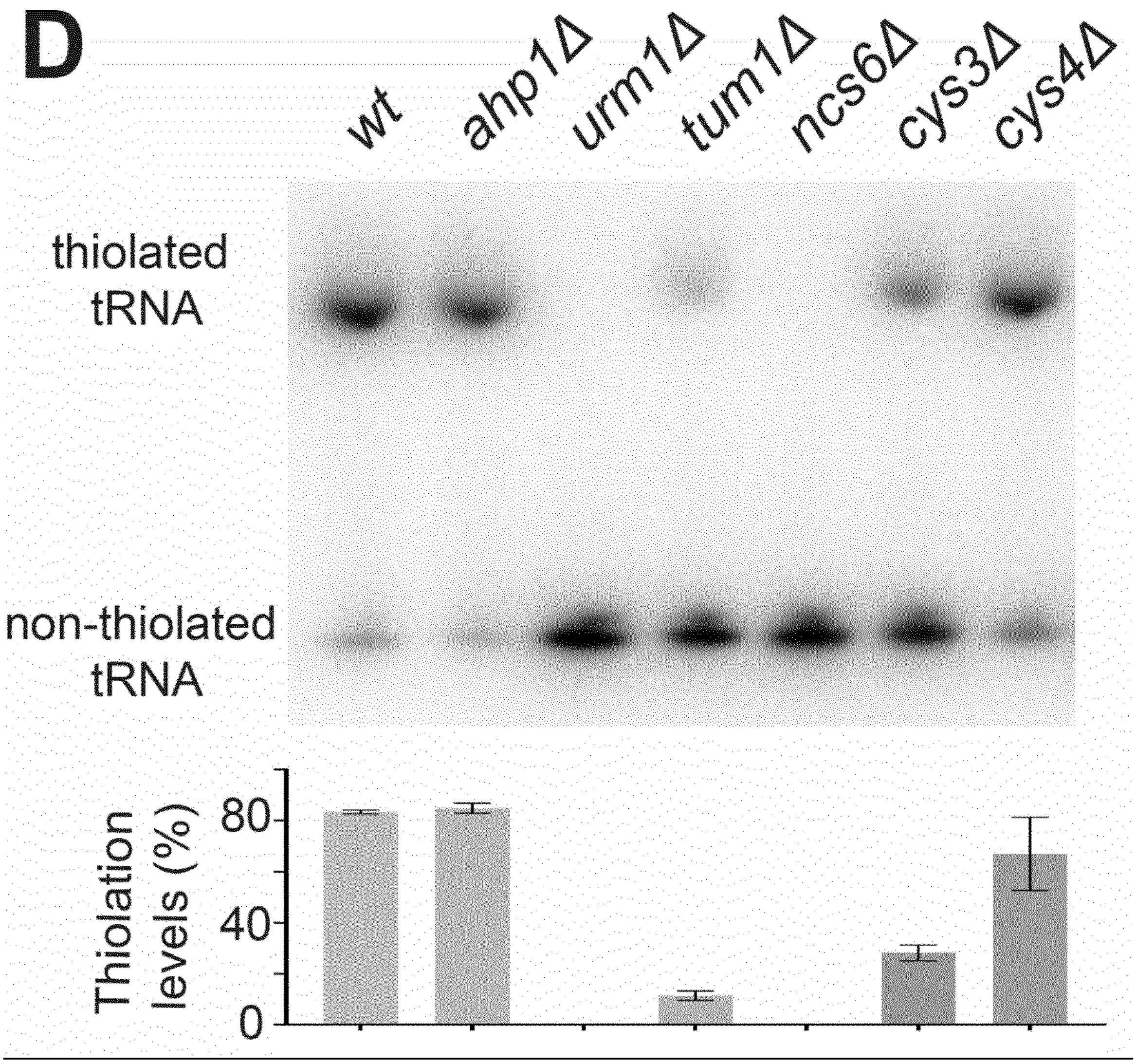
D
wt
ahp1Δ
urm1Δ
tum1Δ
ncs6Δ
cys3Δ
cys4Δ
thiolated tRNA
non-thiolated tRNA
Thiolation levels (%)
80
40
0

Fig. 7 D

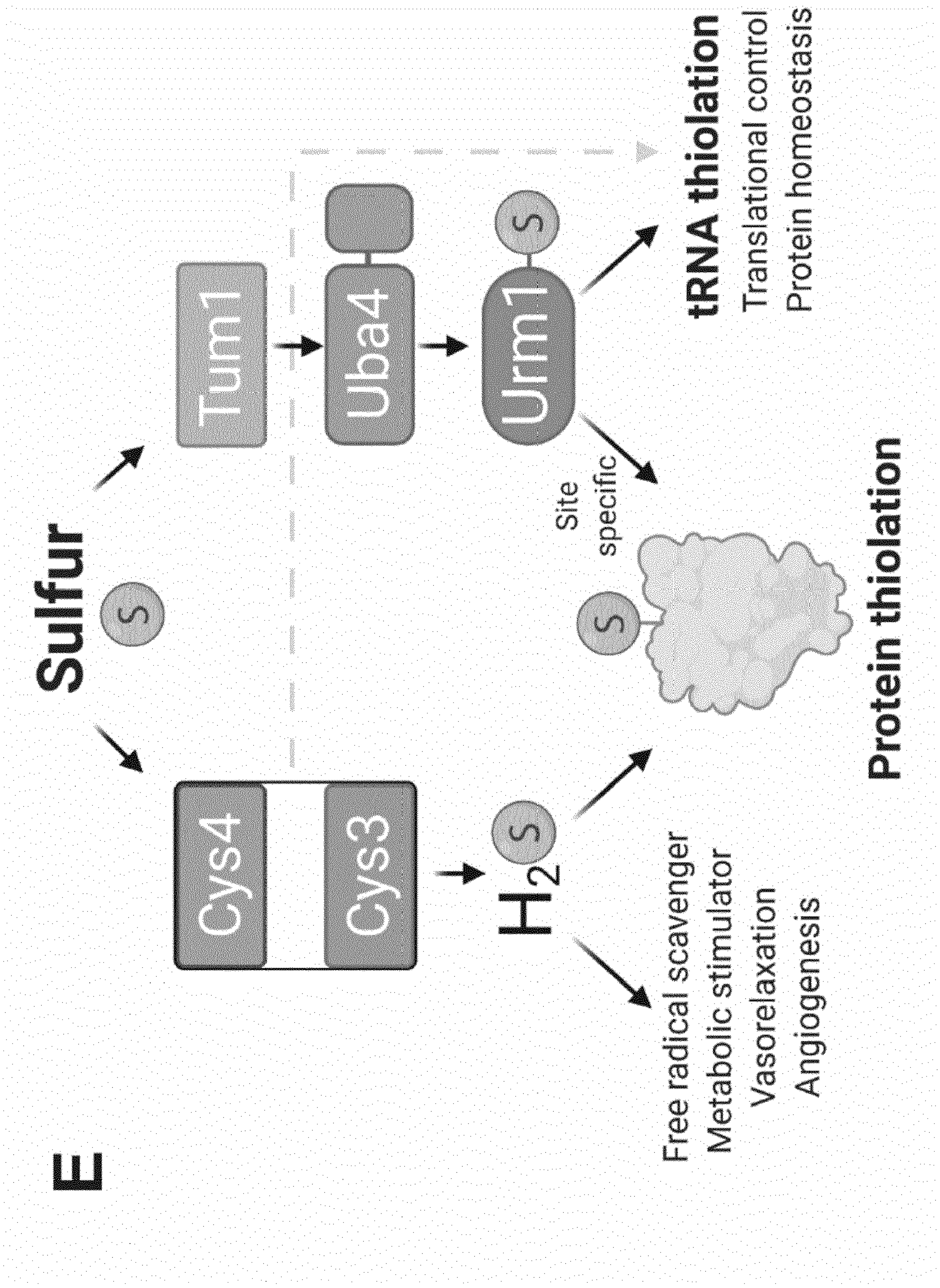
E
Sulfur
S
Cys4
Cys3
Tum1
Uba4
Urm1
S
$H_2S$
Site specific
S
tRNA thiolation
Translational control
Protein homeostasis
Protein thiolation
Free radical scavenger
Metabolic stimulator
Vasorelaxation
Angiogenesis

Fig. 7 E

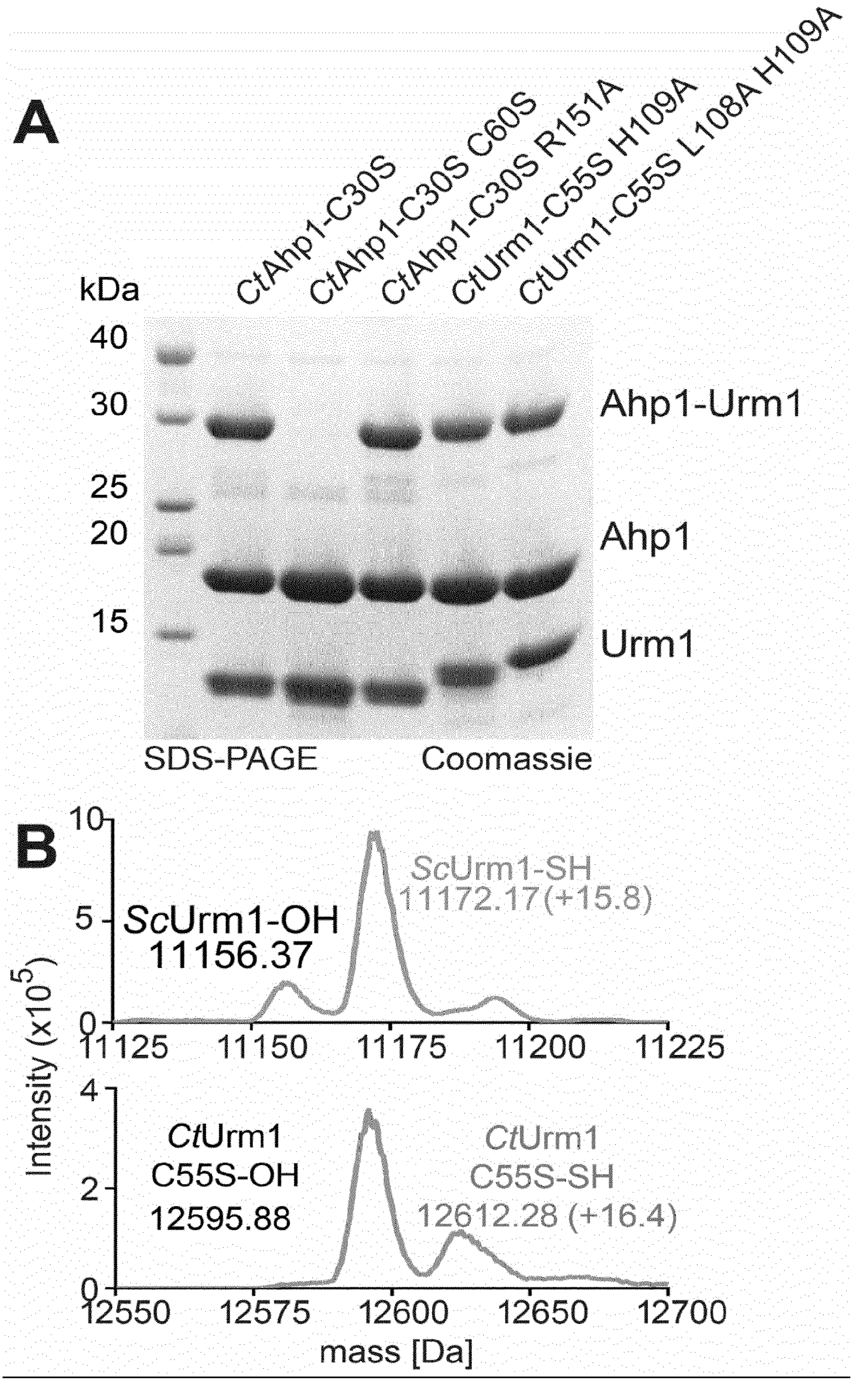
A
kDa
CtAhp1-C30S
CtAhp1-C30S C60S
CtAhp1-C30S R151A
CtUrm1-C55S H109A
CtUrm1-C55S L108A H109A
40
30
25
20
15
Ahp1-Urm1
Ahp1
Urm1
SDS-PAGE
Coomassie
B
ScUrm1-OH
11156.37
ScUrm1-SH
11172.17(+15.8)
Intensity (x10^5)
10
5
0
11125
11150
11175
11200
11225
CtUrm1
C55S-OH
12595.88
CtUrm1
C55S-SH
12612.28 (+16.4)
4
2
0
12550
12575
12600
12650
12700
mass [Da]

Fig. 8 A, B

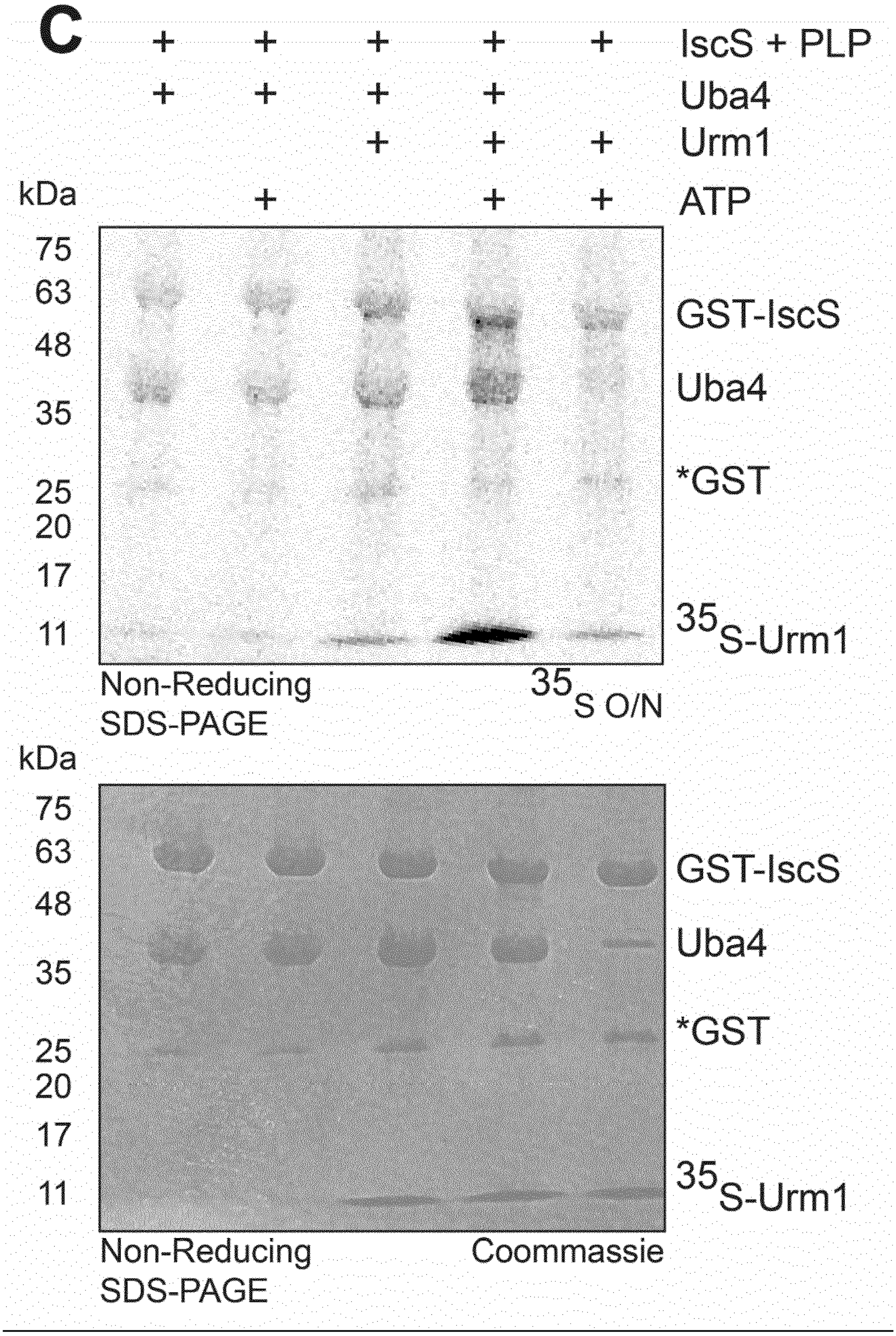
C
IscS + PLP
Uba4
Urm1
ATP
kDa
75
63
48
35
25
20
17
11
GST-IscS
Uba4
*GST
35S-Urm1
Non-Reducing
SDS-PAGE
35S O/N
kDa
75
63
48
35
25
20
17
11
GST-IscS
Uba4
*GST
35S-Urm1
Non-Reducing
SDS-PAGE
Coommassie

**Fig. 8 C**

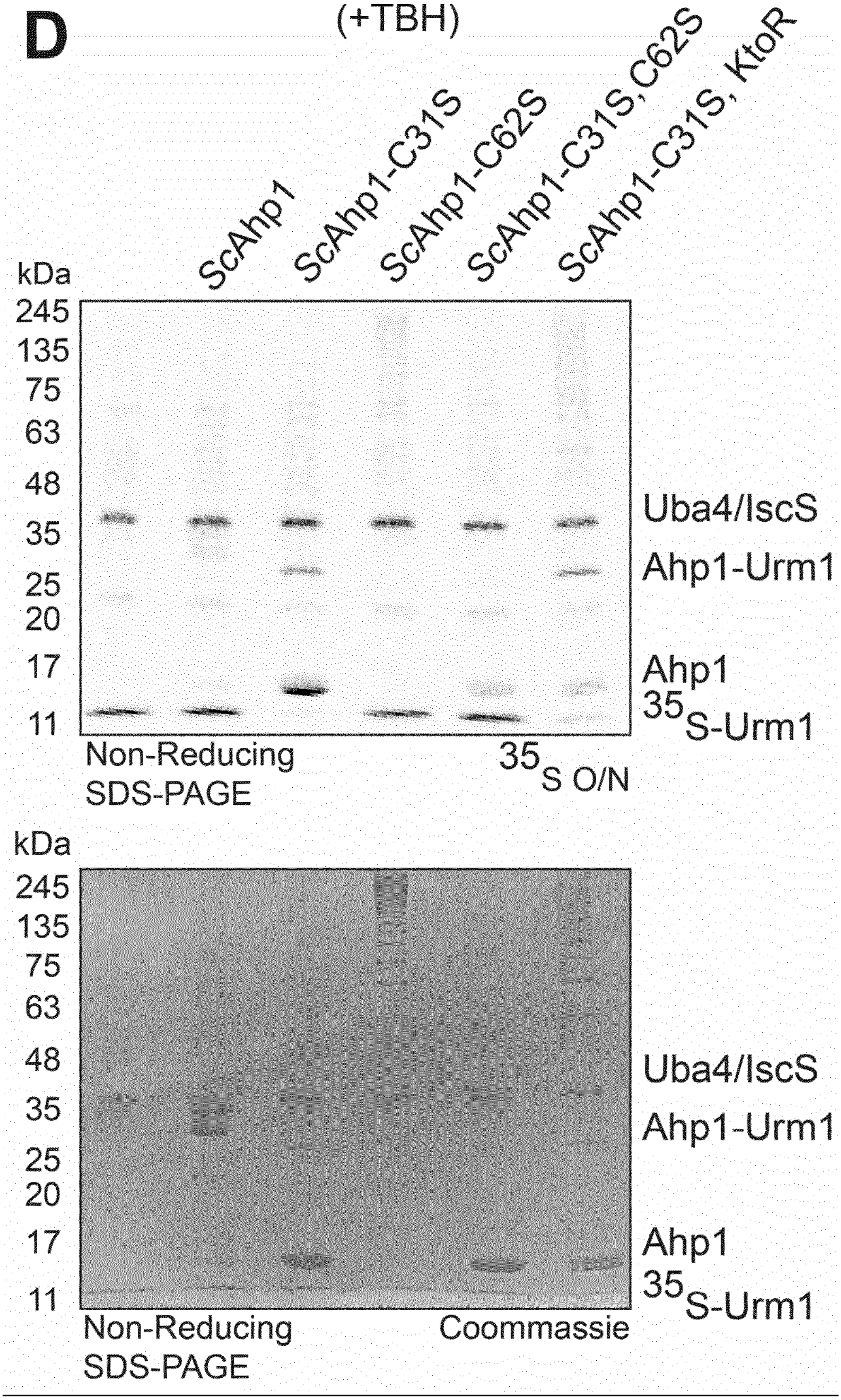
D
(+TBH)
ScAhp1
ScAhp1-C31S
ScAhp1-C62S
ScAhp1-C31S,C62S
ScAhp1-C31S, KtoR
kDa
245
135
75
63
48
35
25
20
17
11
Uba4/IscS
Ahp1-Urm1
Ahp1
35S-Urm1
Non-Reducing
SDS-PAGE
35S O/N
kDa
245
135
75
63
48
35
25
20
17
11
Uba4/IscS
Ahp1-Urm1
Ahp1
35S-Urm1
Non-Reducing
SDS-PAGE
Coommassie

Fig. 8 D

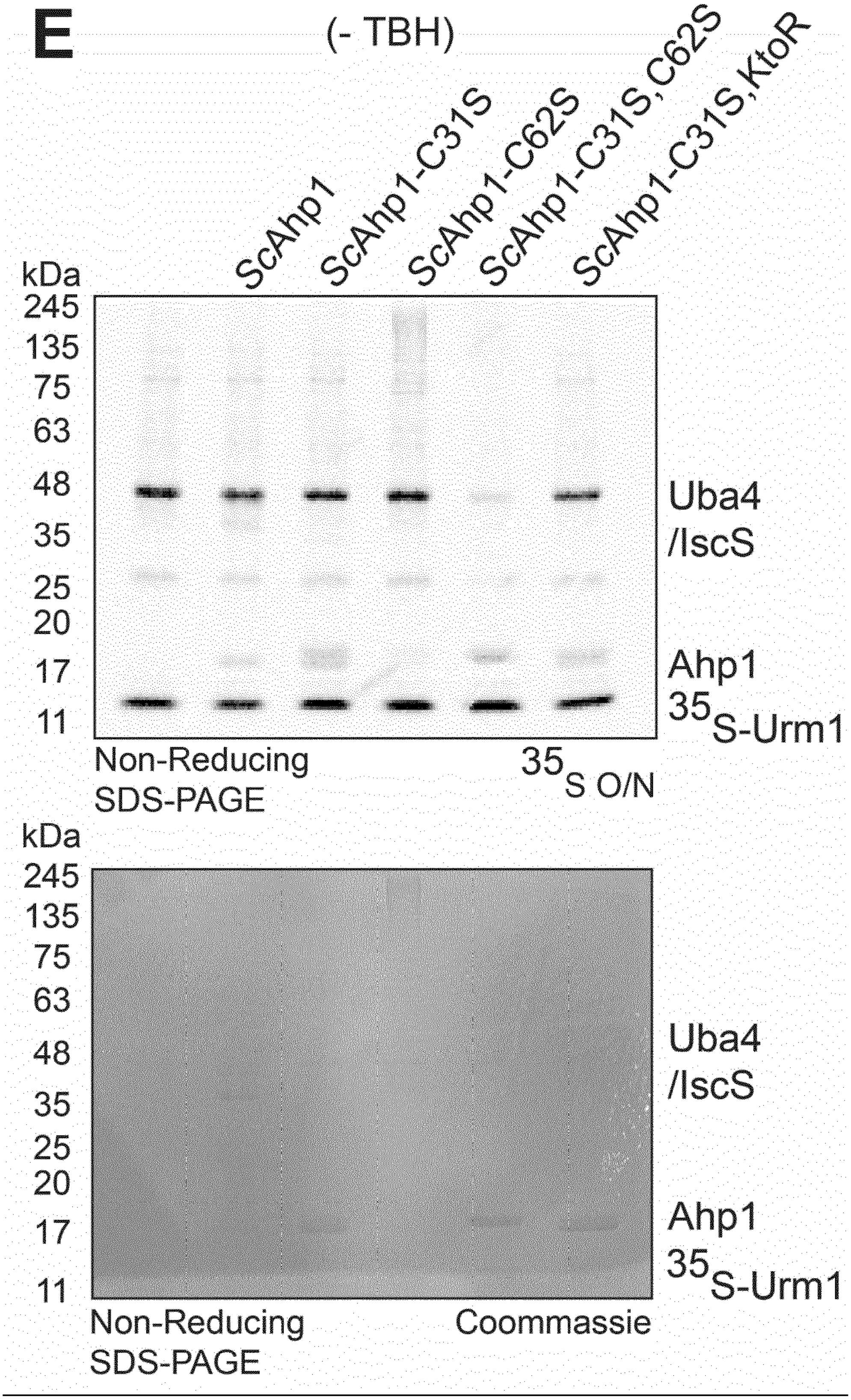
E
(- TBH)
ScAhp1
ScAhp1-C31S
ScAhp1-C62S
ScAhp1-C31S,C62S
ScAhp1-C31S,KtoR
kDa
245
135
75
63
48
35
25
20
17
11
Uba4
/IscS
Ahp1
35S-Urm1
Non-Reducing
SDS-PAGE
35S O/N
kDa
245
135
75
63
48
35
25
20
17
11
Uba4
/IscS
Ahp1
35S-Urm1
Non-Reducing
SDS-PAGE
Coommassie

Fig. 8 E

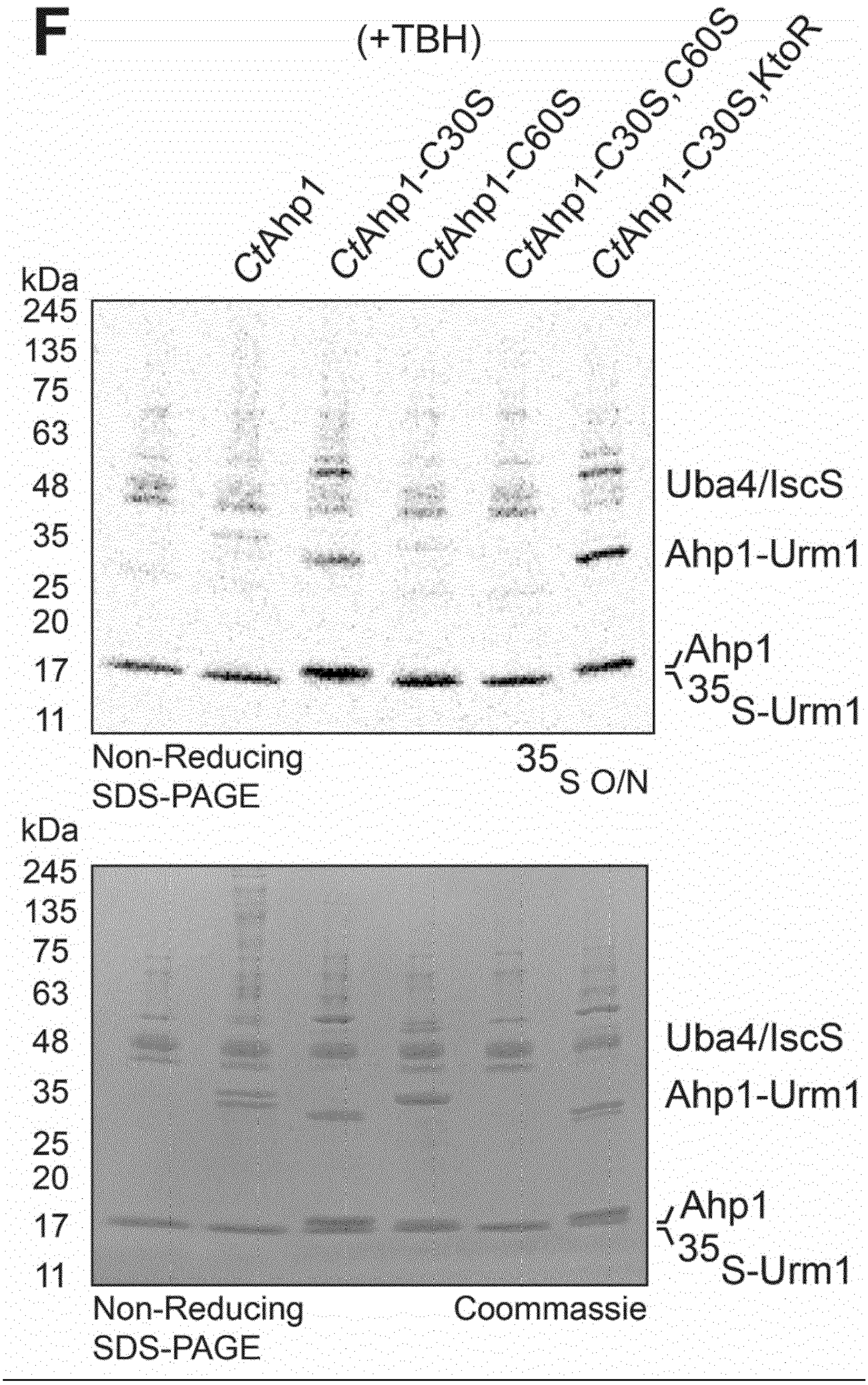
F
(+TBH)
CtAhp1
CtAhp1-C30S
CtAhp1-C60S
CtAhp1-C30S,C60S
CtAhp1-C30S,KtoR
kDa
245
135
75
63
48
35
25
20
17
11
Uba4/IscS
Ahp1-Urm1
Ahp1
35S-Urm1
Non-Reducing
SDS-PAGE
35S O/N
kDa
245
135
75
63
48
35
25
20
17
11
Uba4/IscS
Ahp1-Urm1
Ahp1
35S-Urm1
Non-Reducing
SDS-PAGE
Coommassie

Fig. 8 F

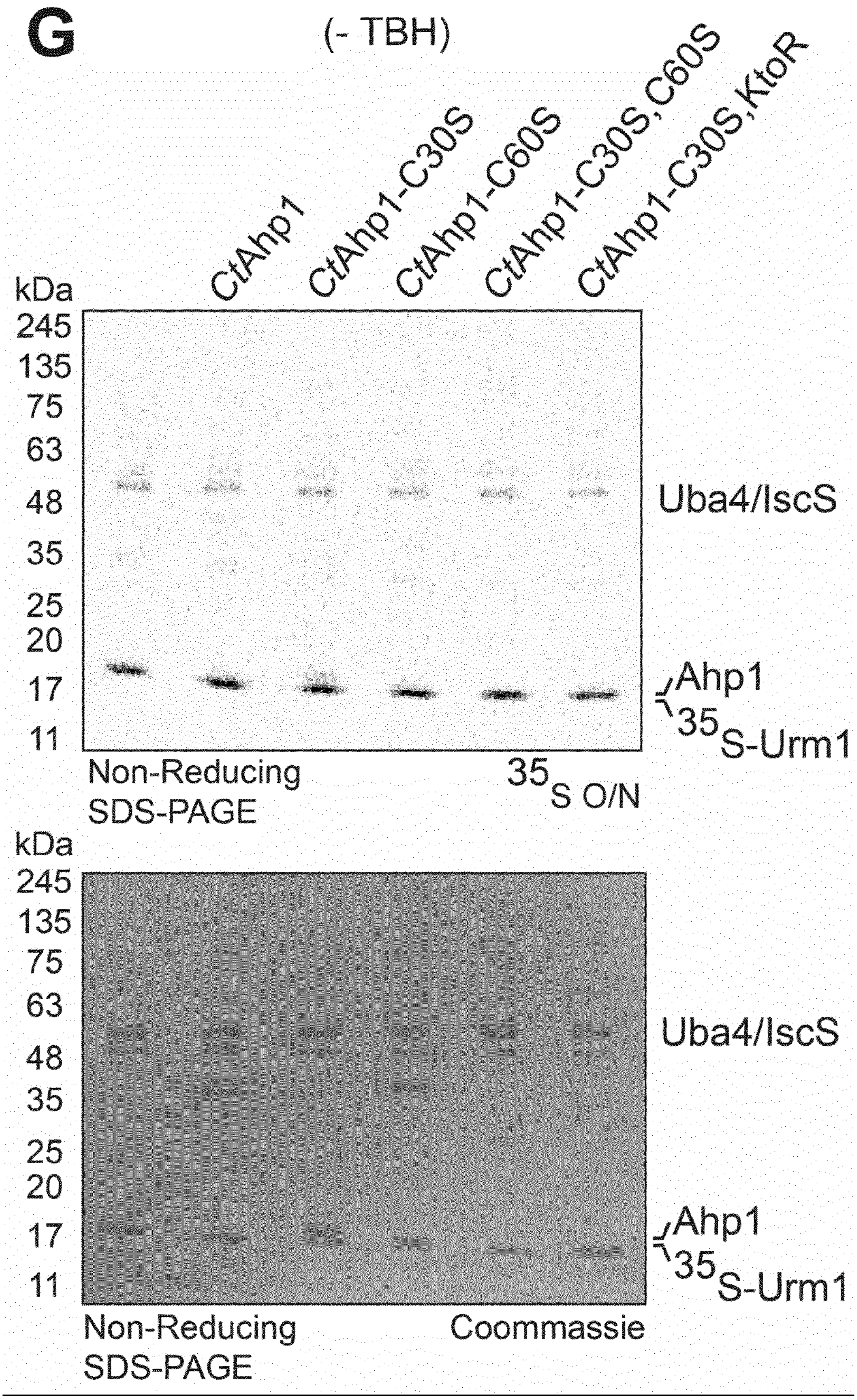
G
(- TBH)
CtAhp1
CtAhp1-C30S
CtAhp1-C60S
CtAhp1-C30S,C60S
CtAhp1-C30S,KtoR
kDa
245
135
75
63
48
35
25
20
17
11
Uba4/IscS
Ahp1
35S-Urm1
Non-Reducing SDS-PAGE
35S O/N
kDa
245
135
75
63
48
35
25
20
17
11
Uba4/IscS
Ahp1
35S-Urm1
Non-Reducing SDS-PAGE
Coommassie

Fig. 8 G

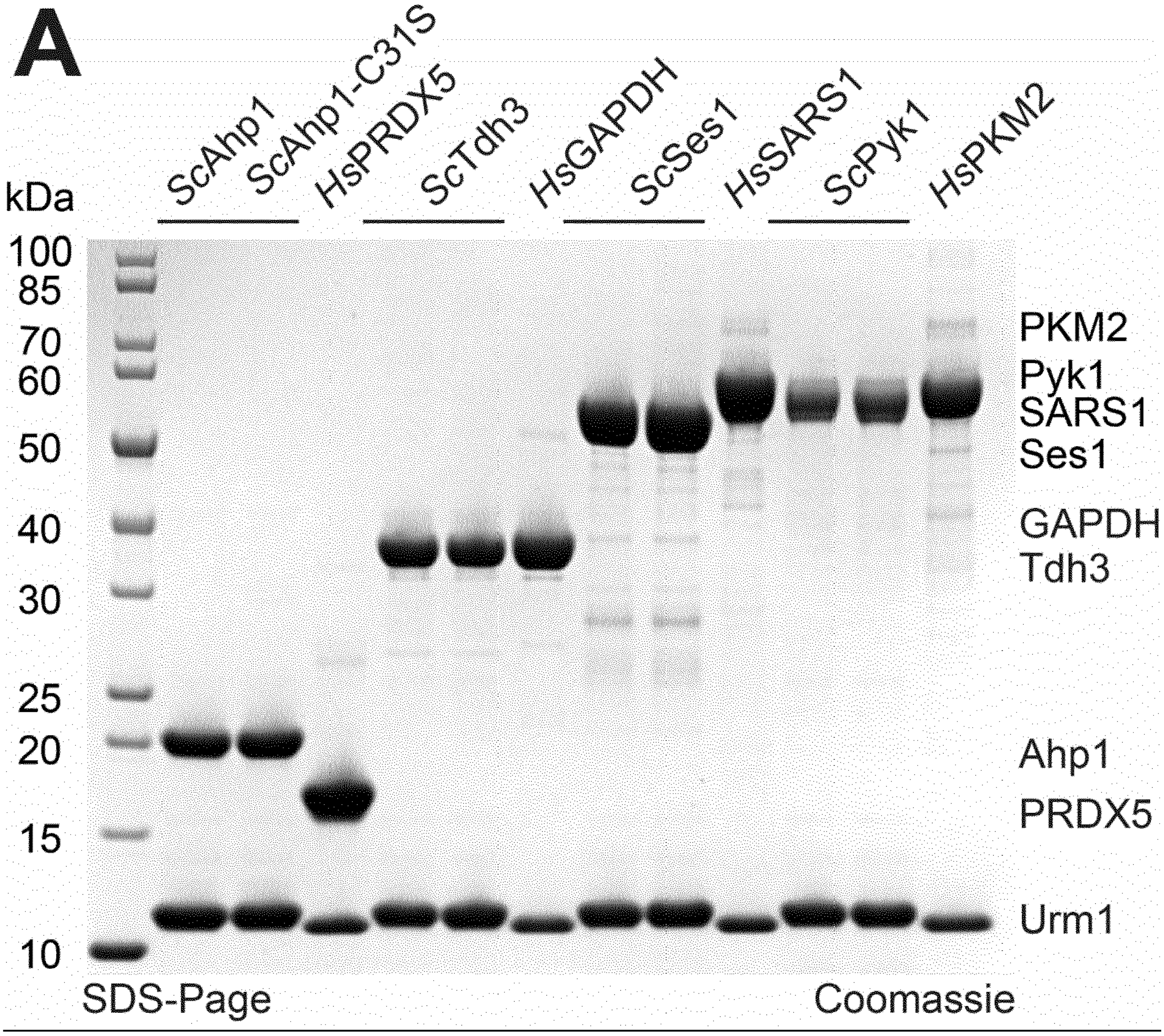
A
kDa
ScAhp1
ScAhp1-C31S
HsPRDX5
ScTdh3
HsGAPDH
ScSes1
HsSARS1
ScPyk1
HsPKM2
100
85
70
60
50
40
30
25
20
15
10
PKM2
Pyk1
SARS1
Ses1
GAPDH
Tdh3
Ahp1
PRDX5
Urm1
SDS-Page
Coomassie

Fig. 9 A

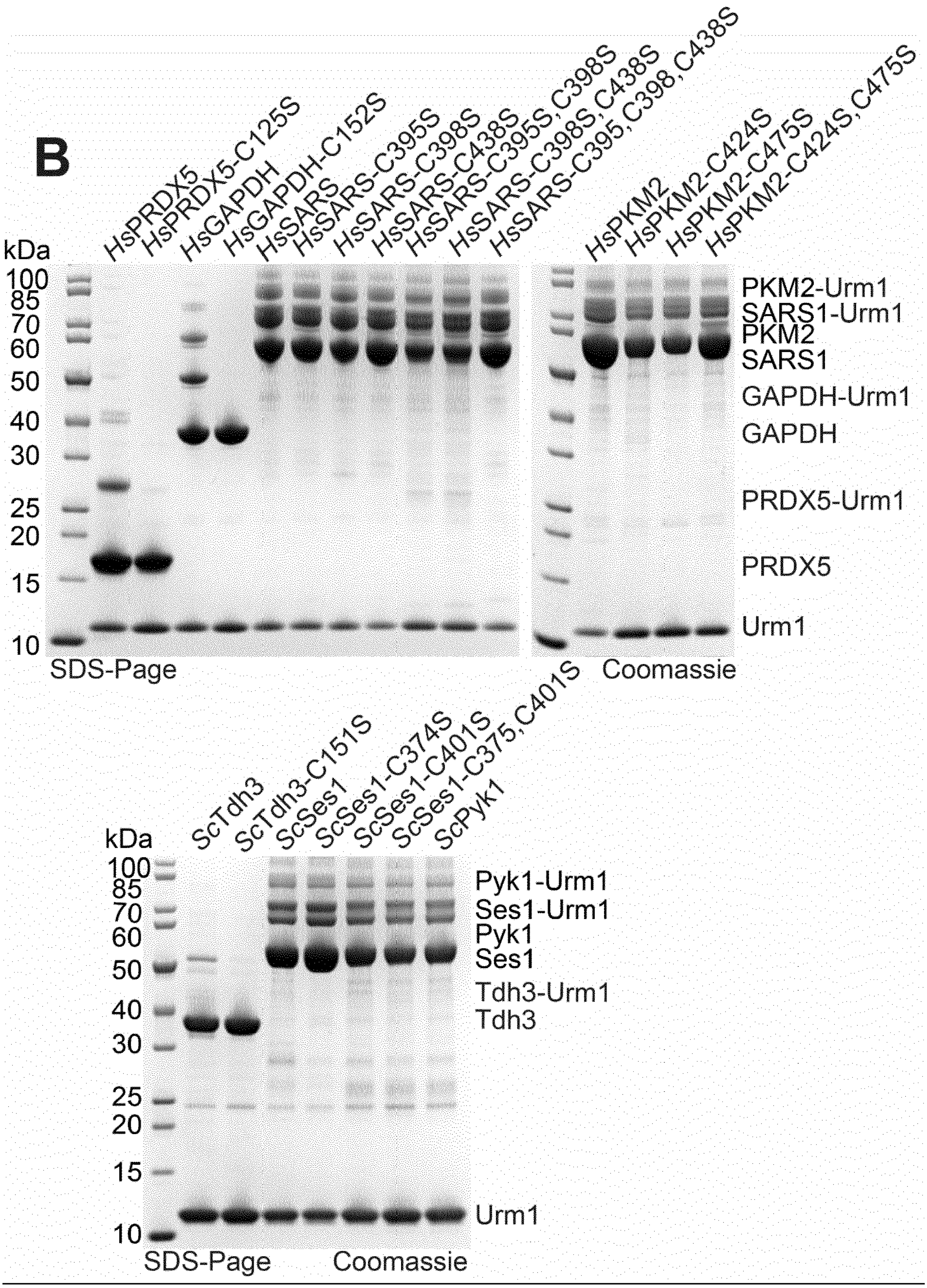
B
HsPRDX5
HsPRDX5-C125S
HsGAPDH
HsGAPDH-C152S
HsSARS
HsSARS-C395S
HsSARS-C398S
HsSARS-C438S
HsSARS-C395S,C398S
HsSARS-C398S,C438S
HsSARS-C395,C398,C438S
HsPKM2
HsPKM2-C424S
HsPKM2-C475S
HsPKM2-C424S,C475S
kDa
100
85
70
60
50
40
30
25
20
15
10
PKM2-Urm1
SARS1-Urm1
PKM2
SARS1
GAPDH-Urm1
GAPDH
PRDX5-Urm1
PRDX5
Urm1
SDS-Page
Coomassie
ScTdh3
ScTdh3-C151S
ScSes1
ScSes1-C374S
ScSes1-C401S
ScSes1-C375,C401S
ScPyk1
kDa
100
85
70
60
50
40
30
25
20
15
10
Pyk1-Urm1
Ses1-Urm1
Pyk1
Ses1
Tdh3-Urm1
Tdh3
Urm1
SDS-Page
Coomassie

**Fig. 9 B**

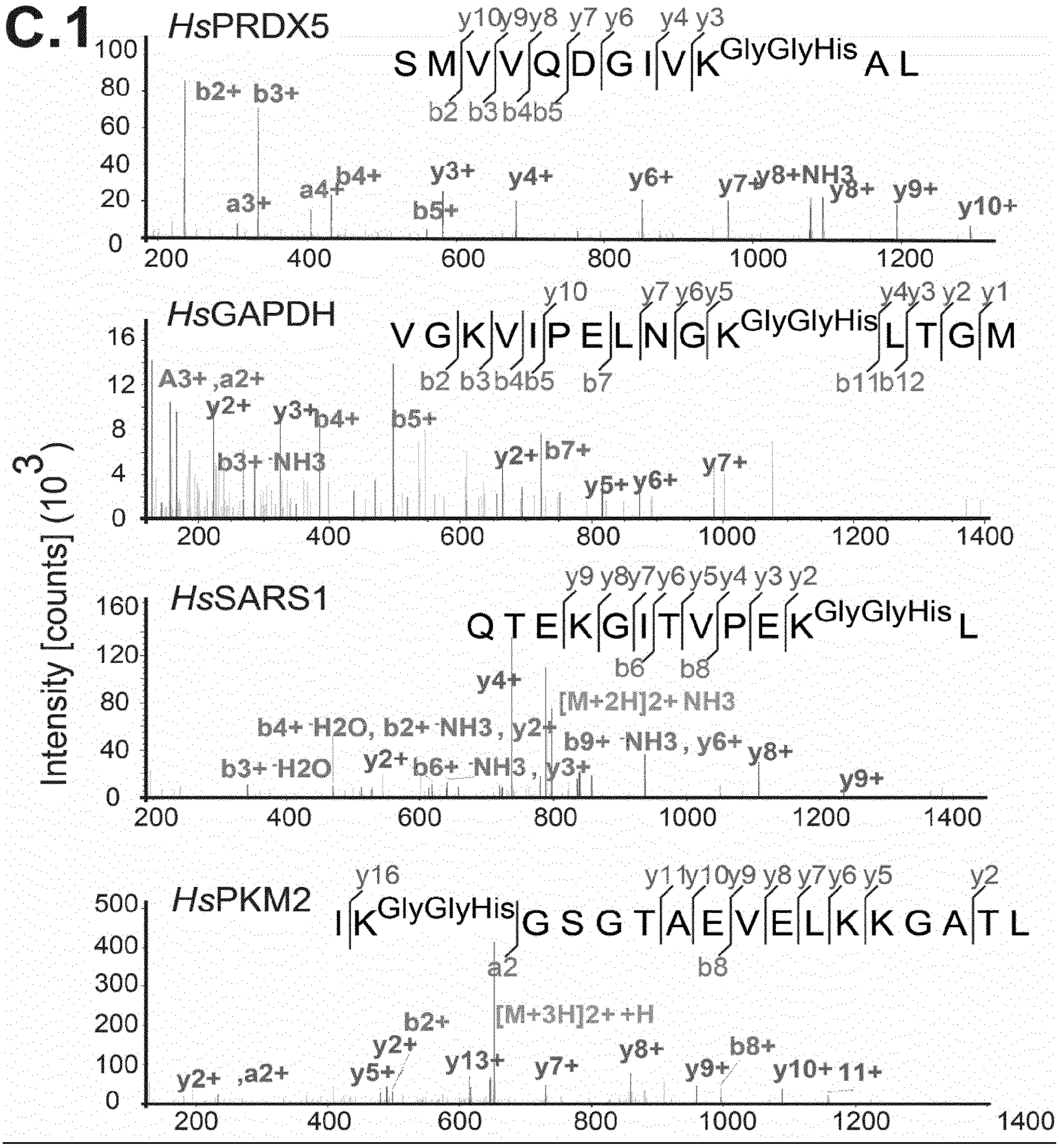
C.1
HsPRDX5
S M V V Q D G I V K GlyGlyHis A L
HsGAPDH
V G K V I P E L N G K GlyGlyHis L T G M
HsSARS1
Q T E K G I T V P E K GlyGlyHis L
HsPKM2
I K GlyGlyHis G S G T A E V E L K K G A T L
Intensity [counts] (10^3)

Fig. 9 C.1

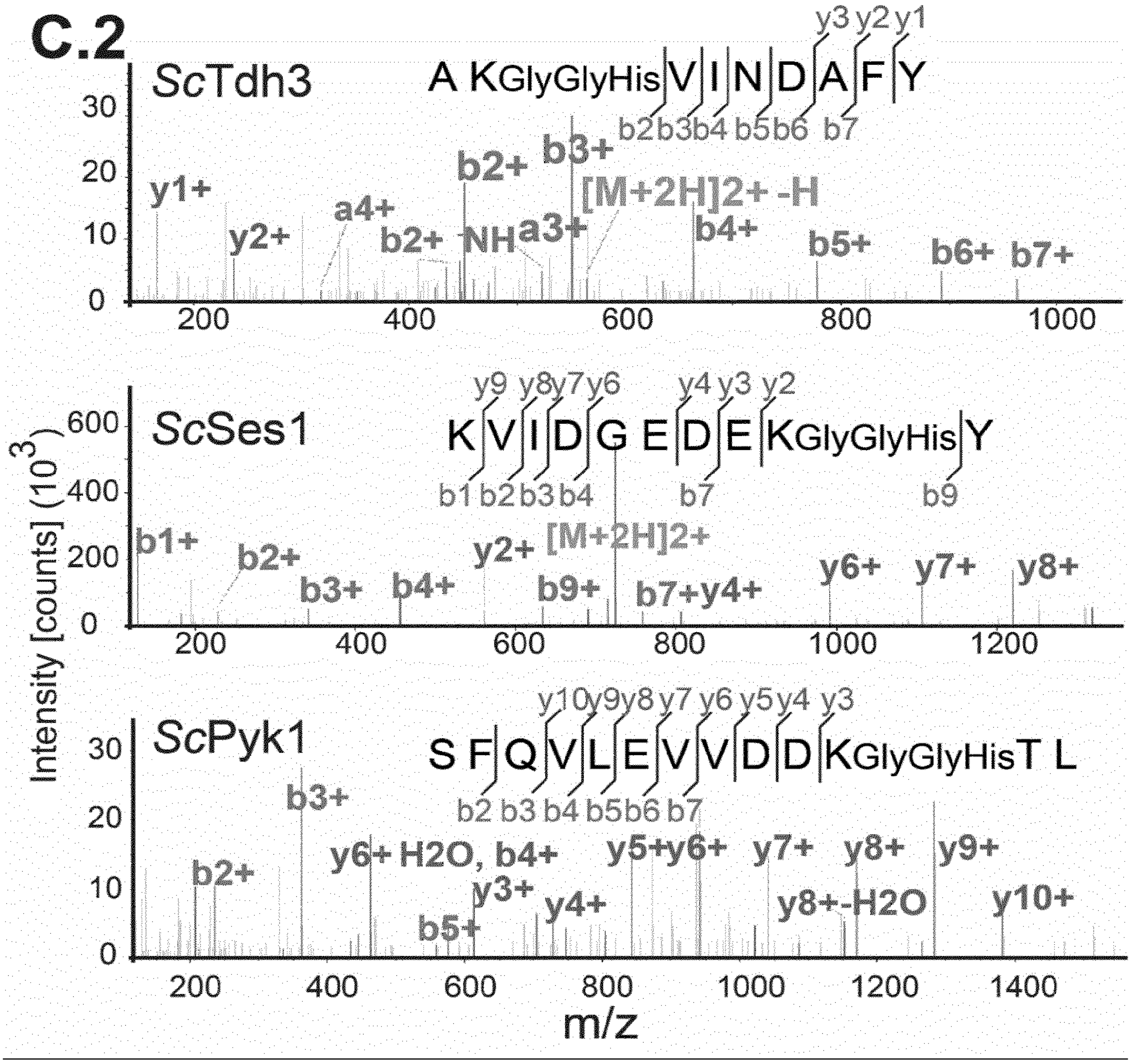
C.2
ScTdh3
A KGlyGlyHis V I N D A F Y
ScSes1
K V I D G E D E KGlyGlyHis Y
ScPyk1
S F Q V L E V V D D KGlyGlyHis T L
Intensity [counts] (10^3)
m/z

Fig. 9 C.2

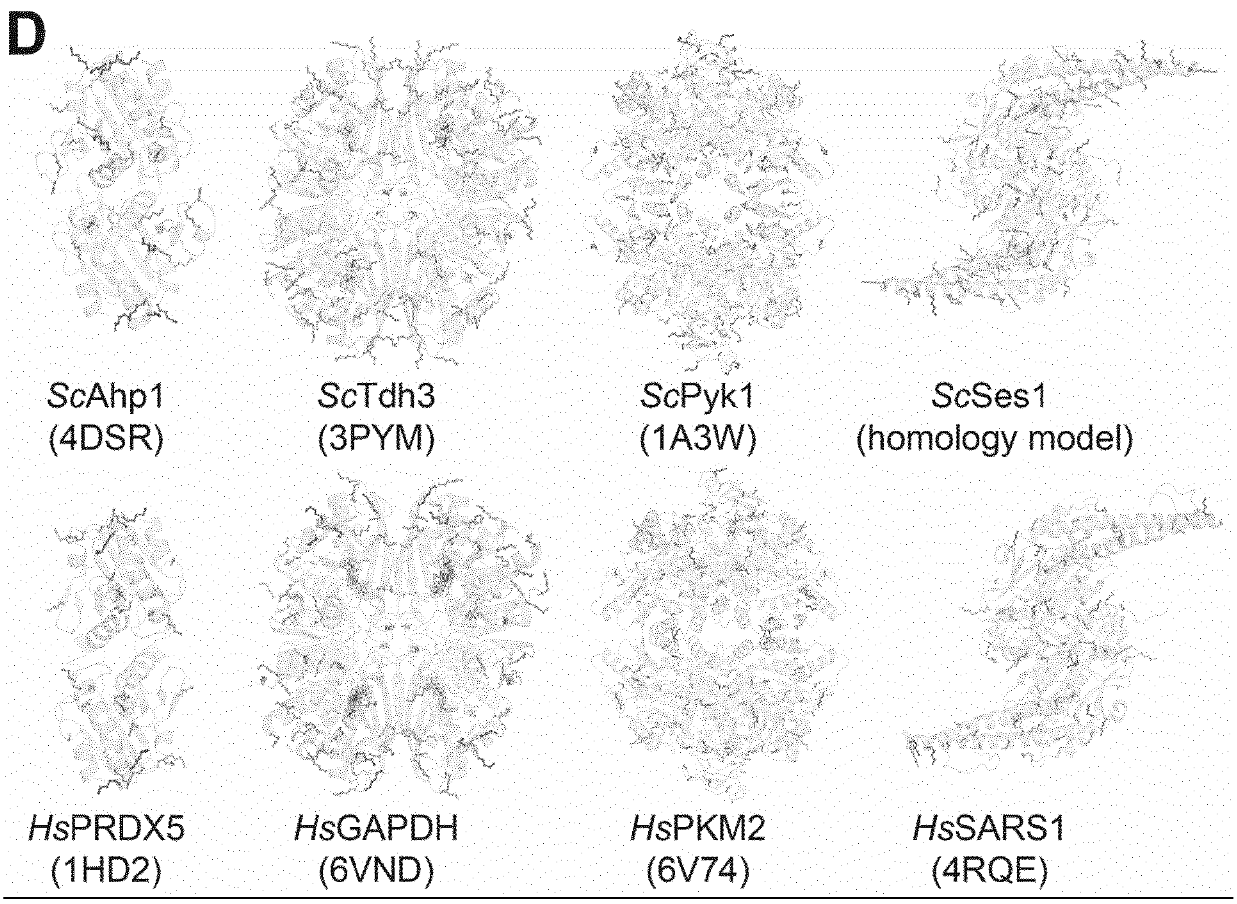
D
*Sc*Ahp1
(4DSR)
*Sc*Tdh3
(3PYM)
*Sc*Pyk1
(1A3W)
*Sc*Ses1
(homology model)
*Hs*PRDX5
(1HD2)
*Hs*GAPDH
(6VND)
*Hs*PKM2
(6V74)
*Hs*SARS1
(4RQE)

Fig. 9 D

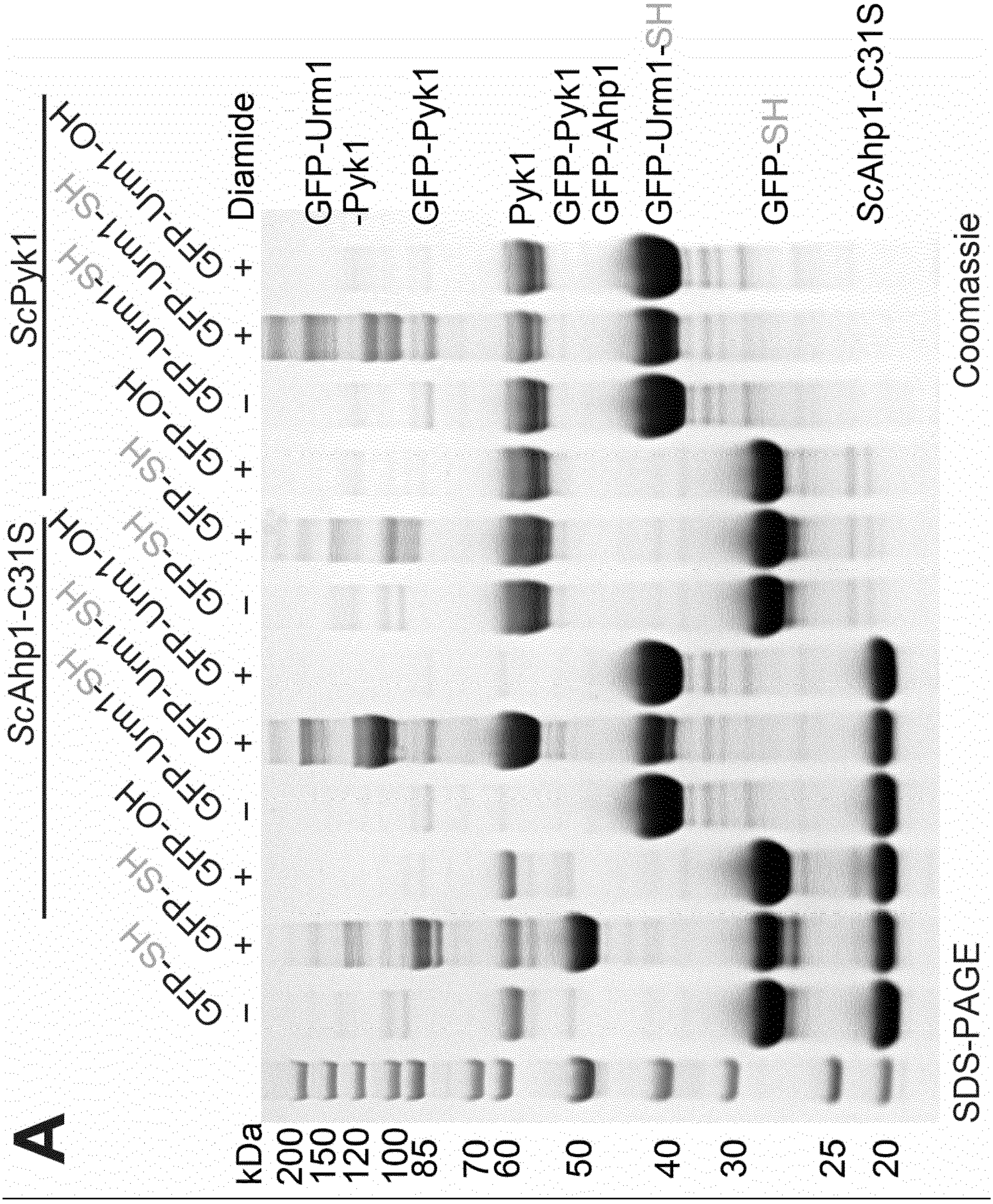
A
ScAhp1-C31S
ScPyk1
GFP-SH
GFP-OH
GFP-Urm1-SH
GFP-Urm1-OH
Diamide
- + + - + + - + + - + +
kDa
200
150
120
100
85
70
60
50
40
30
25
20
GFP-Urm1
-Pyk1
GFP-Pyk1
Pyk1
GFP-Pyk1
GFP-Ahp1
GFP-Urm1-SH
GFP-SH
ScAhp1-C31S
SDS-PAGE
Coomassie

Fig. 10 A

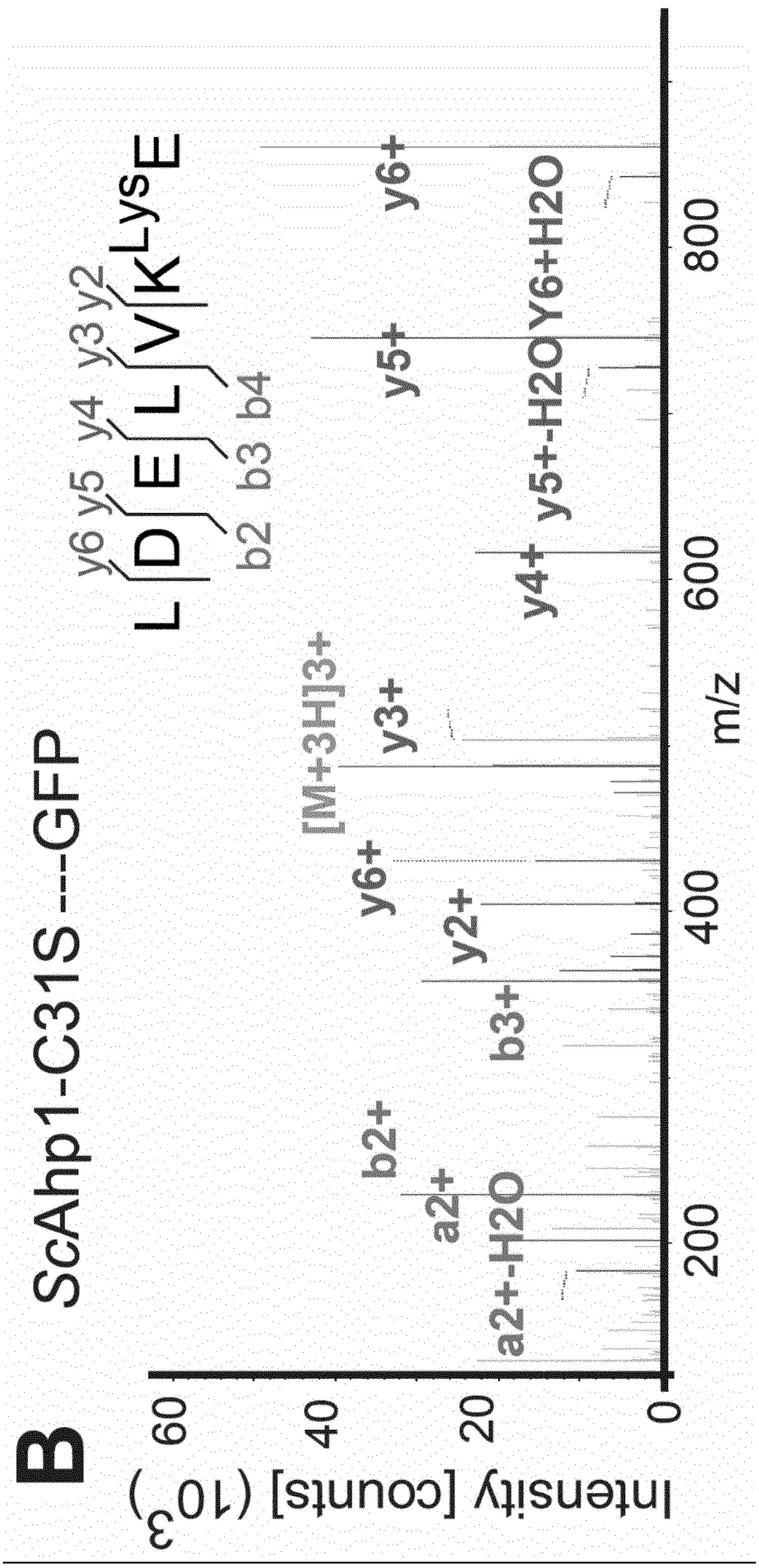
B
ScAhp1-C31S ---GFP
L D E L V K Lys E
y6 y5 y4 y3 y2
b2 b3 b4
Intensity [counts] (10^3)
60
40
20
0
200
400
600
800
m/z
a2+-H2O
a2+
b2+
b3+
y2+
y6+
[M+3H]3+
y3+
y4+
y5+-H2O
y5+
Y6+H2O
y6+

Fig. 10 B

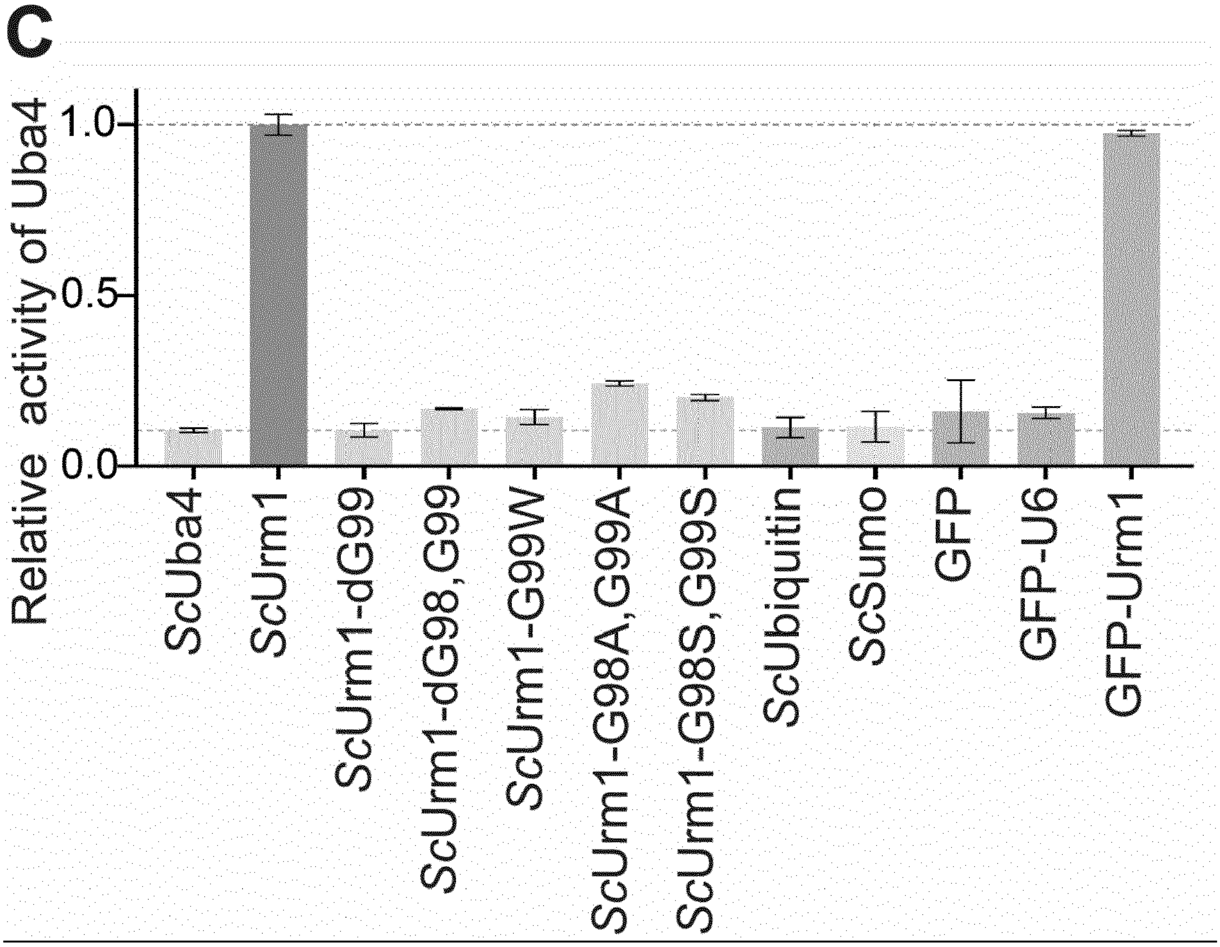
C
Relative activity of Uba4
1.0
0.5
0.0
ScUba4
ScUrm1
ScUrm1-dG99
ScUrm1-dG98,G99
ScUrm1-G99W
ScUrm1-G98A,G99A
ScUrm1-G98S,G99S
ScUbiquitin
ScSumo
GFP
GFP-U6
GFP-Urm1

Fig. 10 C

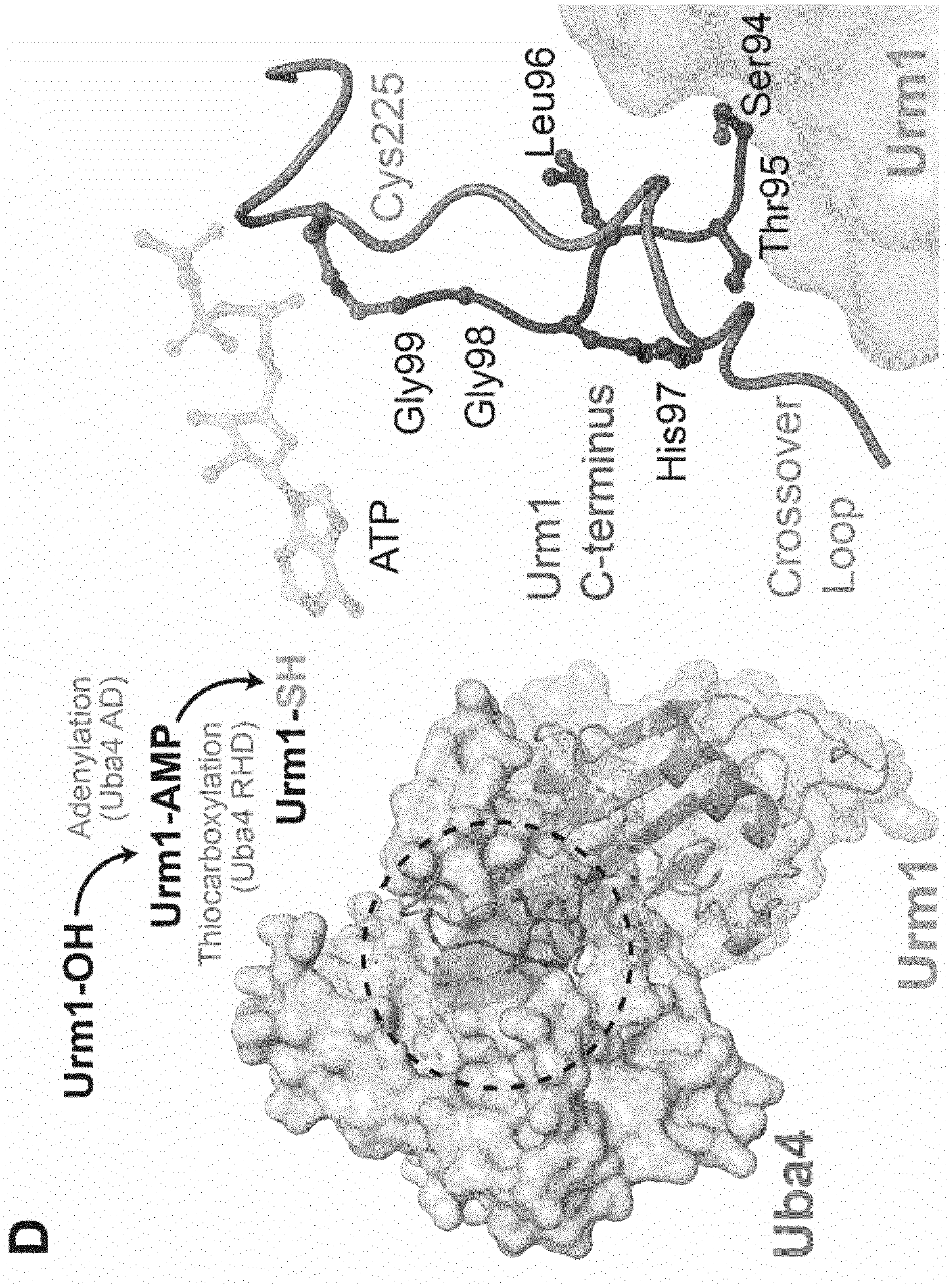
D
Urm1-OH
Adenylation
(Uba4 AD)
Urm1-AMP
Thiocarboxylation
(Uba4 RHD)
Urm1-SH
Uba4
Urm1
ATP
Urm1
C-terminus
Crossover
Loop
Gly99
Gly98
His97
Cys225
Leu96
Thr95
Ser94
Urm1

Fig. 10 D

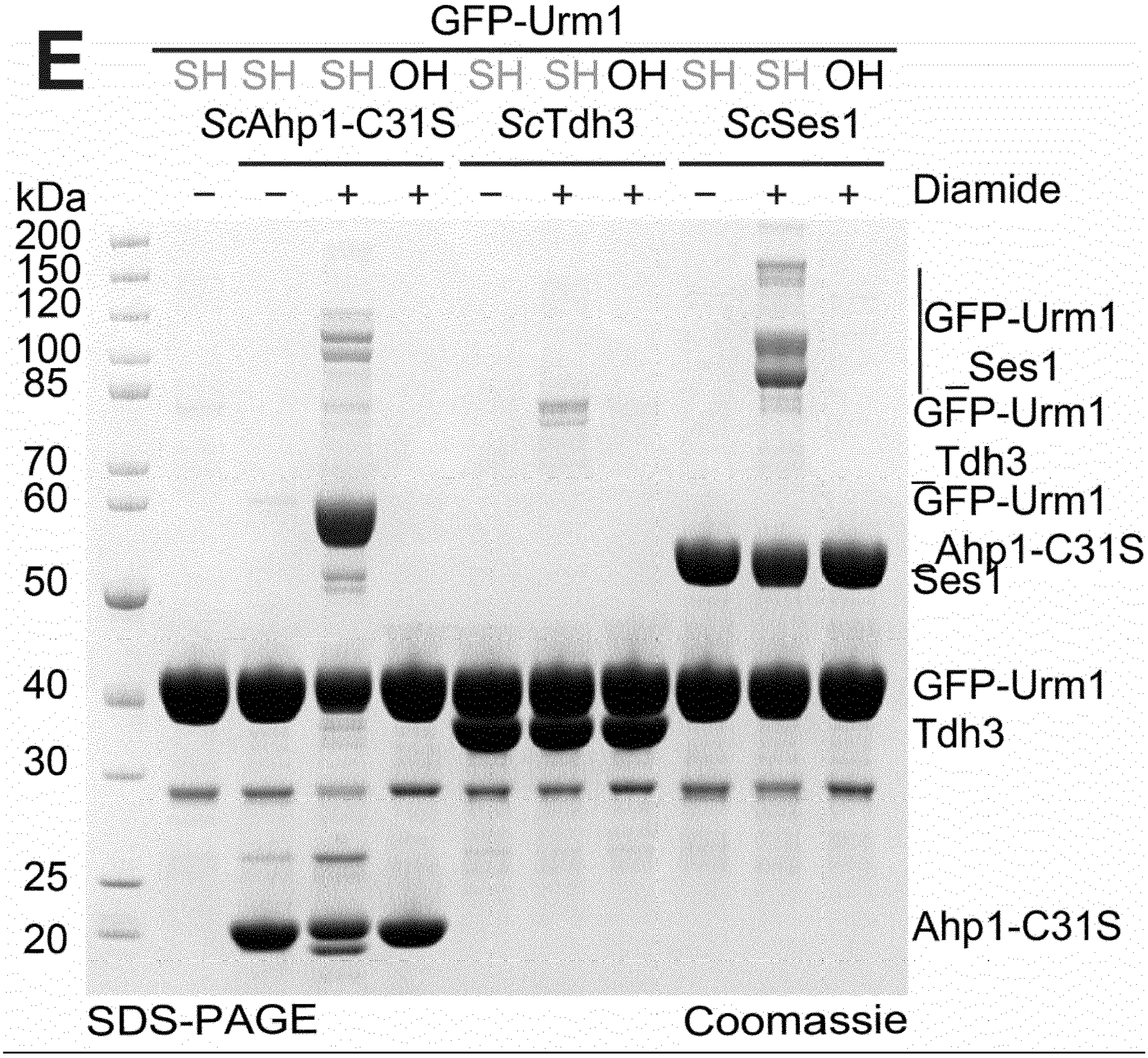
E
GFP-Urm1
SH SH SH OH SH SH OH SH SH OH
ScAhp1-C31S ScTdh3 ScSes1
kDa
− − + + − + + − + +
Diamide
200
150
120
100
85
70
60
50
40
30
25
20
GFP-Urm1
_Ses1
GFP-Urm1
_Tdh3
GFP-Urm1
_Ahp1-C31S
Ses1
GFP-Urm1
Tdh3
Ahp1-C31S
SDS-PAGE
Coomassie

Fig. 10 E

Europäisches Patentamt
European Patent Office
Office européen des brevets

# EUROPEAN SEARCH REPORT

Application Number
EP 22 46 1521

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KADUHR L ET AL: "Urm1, not quite a ubiquitin-like modifier?", MICROBIAL CELL, vol. 8, no. 11, 21 September 2021 (2021-09-21), pages 256-261, XP055963124, Retrieved from the Internet: URL:https://www.ncbi.nlm.nih.gov/pmc/articles/PMC8561144/pdf/mic-08-256.pdf> * the whole document, in particular abstract; page 257, paragraph spanning columns; figures 1, 3 * | 1-4, 7-17,23, 26-29 | INV. C07K14/00 |
| | ----- | | |
| X | VAN DER VEEN A G ET AL: "Role of the ubiquitin-like protein Urm1 as a noncanonical lysine-directed protein modifier", PNAS USA, vol. 108, no. 5, 5 January 2011 (2011-01-05), pages 1763-1770, XP055963292, DOI: 10.1073/pnas.1014402108 | 1-13, 15-19, 23-30 | |
| A | * the whole document, in particular abstract; page 1763, column 1, lines 19-23; page 1766, paragraph spanning columns; page 1767, sections "Urm1 is conjugated ..." and "Urm1 is Appended ..."; page 1769, section "In Vitro Urmylation Assay"; figure 4 * | 14,20-22 | TECHNICAL FIELDS SEARCHED (IPC): C07K G01N |
| | -/-- | | |

The present search report has been drawn up for all claims

1

| Place of search | Date of completion of the search | Examiner |
|---|---|---|
| Munich | 23 September 2022 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 22 46 1521

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | -& VAN DER VEEN A G ET AL: "Supplementary Information: Role of the ubiquitin-like protein Urm1 as a noncanonical lysine-directed protein modifier", PNAS USA, vol. 108, no. 5, 5 January 2011 (2011-01-05), pages 1-9, XP055964001, DOI: 10.1073/pnas.1014402108 * the whole document * ----- | | |
| X | TERMATHE M ET AL: "Urm1: A Non-Canonical UBL", BIOMOLECULES, vol. 11, no. 2, 139, 22 January 2021 (2021-01-22), XP055963132, DOI: 10.3390/biom11020139 * the whole document, in particular abstract; section 5 on pages 7 and 8; figure 3 * ----- | 1-4, 7-17,23, 26-29 | |
| | | | TECHNICAL FIELDS SEARCHED (IPC) |

The present search report has been drawn up for all claims

1

| Place of search | Date of completion of the search | Examiner |
|---|---|---|
| Munich | 23 September 2022 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **THOMPSON et al.** *Nucl Acids Res,* 1994, vol. 22, 4673-4680
- *Vector NTI AdvanceTM 10 DNA and protein sequence analysis software. User's Manual,* 2004, 389-662